# EUROPEAN PATENT SPECIFICATION

(11) **EP 4 626 414 B1**
(45) Date of publication and mention of the grant of the patent: **16.09.2026**
(21) Application number: 23817742.2
(22) Date of filing: 04.12.2023
(51) Int. Cl.: A61K 31/16, A61K 31/198, A61K 31/353, A61K 31/437, A61K 45/06, A61P 17/18

(54) **SYNERGISTIC COMBINATIONS OF REGULATED NECROSIS INHIBITORS WITH N-ACETYLCYSTEINE**
SYNERGISTISCHE KOMBINATIONEN VON REGULIERTEN NEKROSEINHIBITOREN MIT N-ACETYLCYSTEIN
COMBINAISONS SYNERGIQUES D'INHIBITEURS DE NÉCROSE RÉGULÉS AVEC DE LA N-ACÉTYLCYSTÉINE

(30) Priority: 02.12.2022 EP 22306783
(43) Date of publication of application: 08.10.2025
(73) Proprietor: Seabelife, 29680 Roscoff (FR); Université de Rennes, 35042 Rennes Cedex (FR); Institut National de la Santé et de la Recherche Médicale, 75013 Paris (FR); Ecole des Hautes Etudes en Santé Publique (EHESP), 35043 Rennes cedex (FR)
(72) Inventor: SIMOES EUGENIO, Mélanie, 35700 RENNES (FR); DIMANCHE-BOITREL, Marie-Thérèse, 35520 MELESSE (FR); BELAL, Sophie, 29440 PLOUGAR (FR); ROUSSELOT, Morgane, 29420 PLOUENAN (FR)
(74) Representative: Regimbeau
(86) International application number: PCT/EP2023/084156
(87) International publication number: WO 2024/115793

(56) References cited:
- WO-A1-2017/064217
- WO-A1-2018/073321
- WO-A1-2022/157392
- US-A1- 2017 333 393
- SARAVANAN S KARUPPAGOUNDER ET AL: "N-acetylcysteine targets 5 lipoxygenase-derived, toxic lipids and can synergize with prostaglandin E2 to inhibit ferroptosis and improve outcomes following hemorrhagic stroke in mice", ANNALS OF NEUROLOGY, JOHN WILEY AND SONS, BOSTON , US, vol. 84, no. 6, 29 November 2018 (2018-11-29), pages 854 - 872, XP071641839, ISSN: 0364-5134, DOI: 10.1002/ANA.25356
- ARAKAWA ET AL: "N-Acetylcysteine and ebselen but not nifedipine protected cerebellar granule neurons against 4-hydroxynonenal-induced neuronal death", NEUROSCIENCE RESEARCH, ELSEVIER, SHANNON, IR, vol. 57, no. 2, 1 February 2007 (2007-02-01), pages 220 - 229, XP005868278, ISSN: 0168-0102, DOI: 10.1016/J.NEURES.2006.10.011
- FATIH BULUCU ET AL: "Effects of N-Acetylcysteine, Deferoxamine and Selenium on Doxorubicin-Induced Hepatotoxicity", BIOLOGICAL TRACE ELEMENT RESEARCH., vol. 132, no. 1-3, 25 April 2009 (2009-04-25), US, pages 184 - 196, XP055706299, ISSN: 0163-4984, DOI: 10.1007/s12011-009-8377-y
- ADERBAL S AGUIAR ET AL: "The Effect of n-acetylcysteine and Deferoxamine on Exercise-induced Oxidative Damage in Striatum and Hippocampus of Mice", NEUROCHEMICAL RESEARCH, KLUWER ACADEMIC PUBLISHERS-PLENUM PUBLISHERS, NE, vol. 33, no. 5, 17 October 2007 (2007-10-17), pages 729 - 736, XP019579106, ISSN: 1573-6903
- WONGJAIKAM SUWAKON ET AL: "Combined Iron Chelator and Antioxidant Exerted Greater Efficacy on Cardioprotection Than Monotherapy in Iron-Overloaded Rats", PLOS ONE, vol. 11, no. 7, 18 July 2016 (2016-07-18), pages e0159414, XP093039662, DOI: 10.1371/journal.pone.0159414
- ORFANOS N: "The effects of antioxidants on a porcine model of liver hemo... : Journal of Trauma and Acute Care Surgery", 1 January 2016 (2016-01-01), pages 1 - 7, XP093039661, Retrieved from the Internet <URL:https://journals.lww.com/jtrauma/Abstract/2016/06000/The_effects_of_antioxidants_on_a_porcine_model_of.19.aspx> [retrieved on 20230417]
- MAYER M ET AL: "N-ACETYL-L-CYSTEINE IS A PLURIPOTENT PROTECTOR AGAINST CELL DEATH AND ENHANCER OF TROPHIC FACTOR-MEDIATED CELL SURVIVAL IN VITRO", PROCEEDINGS OF THE NATIONAL ACADEMY OF SCIENCES, NATIONAL ACADEMY OF SCIENCES, vol. 91, no. 16, 1 August 1994 (1994-08-01), pages 7496 - 7500, XP002037901, ISSN: 0027-8424, DOI: 10.1073/PNAS.91.16.7496

## Description

The present invention relates to a pharmaceutical composition comprising a combination of N-acetylcysteine with at least one inhibitor of regulated necrotic cell death, such as necroptosis and/or ferroptosis.

Necroptosis, a programmed cell death route, is clearly distinct from apoptosis as it does not involve key apoptosis regulators, such as caspases, Bcl-2 family members or cytochrome c release from mitochondria. "Necroptosis" is a specialized biochemical pathway of programmed necrosis that depends notably on the serine/threonine kinase activity of RIPK1 (Receptor-Interacting Protein Kinase 1). The ground-breaking finding that necroptosis is a genetically controlled process led to the hypothesis that this programmed cell-death is 'druggable', an emerging breakthrough that carries the potential to revolutionize every day clinical medicine [Linkermann and Green, N. Eng. J. Med. 2014, 370(5), 455-465]. Indeed, molecular targets, including RIPK1 (Receptor Interacting Protein 1), RIPK3 and MLKL (Mixed Lineage Kinase domain-Like), have convincingly been shown to contribute to multiple disorders where necroptosis is of central pathophysiological relevance.

Necroptosis plays important role in the pathogenesis of various diseases across the body, including conditions of the neurologic, cardiovascular, pulmonary, and gastrointestinal systems. Necroptosis also plays a role in infectious and autoimmune diseases. Necroptosis was also reported to mediate organ rejection in particular in cardiac and renal allografts (Khoury et al. Am j pathology, 2020; Jouan-Lanhouet et al. semin. Cell dev. Biol. 2014).

Ferroptosis is a new type of non-apoptotic regulated cell death that was first described in 2012, and usually involves high intracellular levels of free iron and lipid peroxidation. This death pathway is directly linked to the ability of the cell to regulate its internal oxidative stress, notably via the activity of the lipid repair enzyme glutathione peroxidase 4 (GPX4). The failure of the glutathione-dependent antioxidant defenses causes an accumulation of lipid-based reactive oxygen species (ROS), which result notably from lipid peroxidation by Fe²⁺, through the Fenton's reaction, leading to membrane damage and cell death.

Recent studies show that ferroptosis is involved in the pathophysiology of many human diseases [Li et al., Cell Death Dis., 2020, 11(88); Tang et al., Cell Research, 2021, 31:107-125; Sun et al., Biomed. Pharmacother., 2020, 127, 110108], affecting in particular the heart, the brain, the nervous system, the eyes, the gastrointestinal system, the liver, the skin, the kidneys, the lungs, the bowel the pancreas or the whole body. Ferroptosis involves three primary metabolisms including thiol, lipid and iron leading to an iron-dependent generation of lipid peroxidation and, ultimately to cell death.

Hallmarks of ferroptosis were used as key elements to define ferroptosis-associated disease biomarkers. Ferroptosis is an iron-dependent regulated tissue necrosis mainly caused by unrestricted lipid peroxidation and subsequent membrane damage. The modifications of the physiological levels of the following components were reported as associated with ferroptosis: iron, reactive oxygen species, ROS (including lipid ROS such as 4-Hydroxynonenal (4-HNE) and malondialdehyde (MDA), and oxidized phospatidylethanolamine (oxPE) species followed by oxidized phosphatidylserine (oxPS) and oxidized phosphatidylinositol (oxPI) [Wiernicki et al., Cell Death Dis., 2020, 11(922)]) and related peroxide detoxification molecules (including the thiol-containing compound gluthation, GSH, or Coenzyme Q10, also known as ubiquinone), and the long-chain-fatty-acid-CoA ligase 4 (ACSL4) [Chen X. et al., Front. in Cell and Dev. Biol., 2021, 9(637162)]. These key biochemical ferroptosis biomarkers can be measured and quantified by assays in bodily fluids (blood, plasma, serum, urine, cerebrospinal fluid) or highlighted by immunohistochemistry labeling on biopsies of damaged tissues.

Depending on both the pathology and the damaged organ, several among ferroptosis-associated biomarkers could vary (increase > or decrease <) in quantity and/or activity relative to normal physiological thresholds. Here we only described reference values for serum:
(1) Iron metabolism (by measuring iron and ferritin levels in serum) is over the physiological thresholds (serum iron, in male > 180 µg/dl, in female > 160 µg/dl; [Pagana et al., Mosby's Diagnostic and Laboratory Test Reference - Elsevier eBook on VitalSource, 14th Edition, Elsevier, 2019, ISBN: 9780323609678]), ferritin, in male >300 ng/ml, in female > 200 ng/ml, [Wang et al., Biochim Biophy. Acta, 2010, 1800(8): 760-769]);
(2) Glutathione redox status (by measuring reduced glutathione (GSH) and oxidized glutathione (GSSG) as well as glutathione peroxidase activity (GPx) in plasma using ELISA) (GSH < 717 µmol/L, GSSG > 5.32 µmol/L; ratio GSH/GSSG < 156; GPx, in male < 20 UI/gHb, in female < 26 UI/gHb), [Haleng J. et al., Rev. Med. Liege, 2007]);
(3) Oxidative stress (by measuring levels of total Q10 and reduced and active form of Q10 (Q10H2) in plasma (in male Q10 < 3.44 µmol/l and Q10H2 < 3.04 µmol/l; in female Q10 < 1.88 µmol/l and Q10H2 < 1.64 µmol/l, [Kaikkonen et al., Scand J Clin Lab Invest, 1999, 59: 457-466]);
(4) Lipid peroxidation (measured by detection of 4-Hydroxynonenal (4-HNE) and malondialdehyde (MDA) adducts) is over the physiological thresholds (> 10 µmol/L for 4-HNE [Chen and Niki, IUBMB Life, 2008, 58(372-373)] and > 3 µmol/L for MDA using thiobarbituric acid method [Banjare et al., J. Sci. Soc., 2017; 44(137-9)]).

Note here that the upregulation of ACSL4 enzyme level in damaged organ tissues was also reported as putative biomarker of ferroptosis (ACSL4 expression can be monitored by transcriptomic and proteomic approaches).

Pathologies associated with ferroptosis affecting the heart include myocardial ischemia-reperfusion injury, notably occurring after artery ligation, cardiomyopathy, notably doxorubicin-induced cardiomyopathy [Li *et al.,* 2020], and cardiovascular disease, notably aortic dissection [Chen et al., Pharmacol. Res., 2022, 177, 106122], among others [Li et al., Free Radic. Biol. Med., 2020, 160, 303-318; Qin et al., Biomed. Pharmacother., 2021, 141, 111872].

Pathologies associated with ferroptosis affecting the central nervous system include strokes, notably ischemic stroke [Li *et al.,* 2020] or hemorrhagic stroke [Li et al., JCI Insight, 2017, 2(7):e90777], traumatic brain injury [Xie et al., CNS Neurosci Ther., 2019, 25:465-475], contusion spinal cord injury [Zhang et al., Neural Regen. Res., 2019, 14(3):532], epilepsy, including mitochondrial disease-related epilepsy and intractable epilepsy [Kahn-Kirby et al., PLoS One., 2019, 14(3)], and neurodegenerative disorders, in particular chronic neurodegenerative disorders, more particularly Alzheimer's disease [Li *et al.,* 2020], Huntington's disease [Mi et al., Neuromolecular Med., 2019, 21, 110-119], Parkinson's disease [Do Van et al., Neurobiol Dis., 2016, 94: 169-78], amyotrophic lateral sclerosis (Charcot's disease) [Li *et al.,* 2020], multiple sclerosis [Luoqian et al., Cell Mol Immunol., 2022, 19(8), 913-924], Friedreich's ataxia [Cotticelli et al., J Pharmacol Exp Ther., 2019, 369(1): 47-54], periventriculor leukomalacia [Skouta et al., J. Am. Chem. Soc., 2014, 136, 4551-4556] and dementia, which may be linked to one or several of the previous pathologies.

Pathologies associated with ferroptosis affecting the eyes include vision loss, in particular due to cataract [Wei et al., Free Radic Biol Med., 2021, 167, 94-108], and retinal disorders, notably Stargardt disease and age-related macular degeneration (AMD), in particular dry AMD [Sun et al., Invest Ophth Vis Sci., 2018, 59(9), 2482; Chen et al., J. Biol. Chem., 2021, 296, 100187].

Pathologies associated with ferroptosis affecting the liver include chronic liver diseases as well as acute liver injury and acute liver failure. Among chronic liver diseases, mention should be made of non-alcoholic steatohepatitis (NASH) [Qi et al., Am J Pathol., 2020, 190(1)], chronic infections such as hepatitis B and C [Cappelletti et al., Int J Mol Sci., 2020, 21(14)] and alcoholic cirrhosis [Zhou et al., Hepatol Commun., 2019, 3(5)]. Acute liver failure may notably result from a drug-induced liver injury (DILI), such as acetaminophen (APAP)-induced liver injury [Yamada et al., Cell Death Dis., 2020, 11(2)], from an ischemia-reperfusion injury induced by a septic or hemorrhagic shock [Friedmann Angeli et al., Nat Cell Biol., 2014, 16(12):1180-91], from fulminant viral hepatitis, from auto-immune origin or from alcohol intake.

Pathologies associated with ferroptosis affecting the skin include skin inflammatory diseases, such as psoriasis [Li et al., Cell Death Dis., 2020, 11(88)], and toxic epidermal necrolysis (Lyell syndrome) [Zhang et al., J Invest Dermatol., 2020, 140(7), S79].

Pathologies associated with ferroptosis affecting the kidneys include acute kidney injury (AKI) (also known as acute renal failure), such as crystal (oxalate)-, folic acid (FA)-induced AKI [Martin-Sanchez *et al.,* 2017] and cisplatin-induced AKI [Deng et al., J Clin Invest., 2019, 129(11); Mishima et al., J Am Soc Nephrol.,2020, 31(2); Hu et al., Cell Death Dis., 2020, 11(1)], renal ischemia-reperfusion injury [Li *et al.,* 2020], and acute tubular necrosis [Friedmann Angeli *et al.*, 2014].

Pathologies associated with ferroptosis affecting the lungs include chronic obstructive pulmonary disease (COPD) [Yoshida et al., Nat Commun., 2019, 10, 3145], bronchial asthma [Tao et al., Oxid Med Cell Longev., 2020], lung injury caused by a bacterial infection, notably by *Pseudomonas aeruginosa* [Dar et al., J Clin Invest., 2018, 128(10), 4639-4653] or *Mycobacterium tuberculosis* [Amaral et al., J Exp Med., 2019, 216(3): 556-570] and pulmonary fibrosis, such as radiation induced-lung fibrosis (RILF) [Li et al., J Inflamm., 2019, 16:11] and paraquat-induced pulmonary damage [Rashidipour et al., Toxicology, 2020, 433-434:152407].

Pathologies associated with ferroptosis affecting the gut include necrotizing enterocolitis [Subramanian et al., Acta Physiologica Sinica, 2020, 72(3)] and inflammatory bowel diseases, such as Crohn's disease [Mayr et al., Nat Commun., 2020, 11(1)] and ulcerative colitis.

Pathologies associated with ferroptosis affecting the whole body include haemochromatosis [Imoto et al., Transfus Apher Sci., 2018, 57(4), 524-531], β-thalassemia [Sposi, N. M., Oxidative Stress and Iron Overload in β-Thalassemia: An Overview, 2019, DOI: 10.5772/intechopen.90492], hemolytic disorders [Youssef et al., 2019, Ferroptosis in Hemolytic Disorders. In: Tang D. (eds) Ferroptosis in Health and Disease. Springer, Cham.], cytokinic storm during a viral infection [Edeas et al., Int J Infect Dis., 2020, 97; Yang and Lai, Cell Death Discov., 2020, 6], radiation-induced necrosis [Wu et al., Front Oncol., 2020, 10], rheumatoid arthritis [Xie et al., Inflammation., 2020, doi: 10.1007/s10753-020-01338-2], type I diabetes [Bruni et al., Cell Transplant., 2018, 27(6)], insulin resistance related to obesity, and pathologies related to stress-induced premature tissue senescence, such as atherosclerosis [Bai et al., Free Radic Biol Med., 2020, 160], hypertension [Yang et al., Clin Exp Hypertens., 2020, 42(8)] and type II diabetes [Li et al., Nutrients., 2020, 12(10)].

Therefore, inhibition of regulated necrotic cell death is a new and attractive therapeutic strategy for disorders associated with it.

The inventors have previously disclosed two new classes of regulated necrosis inhibitors (also designated herein as "inhibitors of regulated necrotic cell death"), the first one consisting of sibiriline derivatives (WO 2017/064217, WO 2017/064216, and WO 2022/157392), and the second one consisting of nigratine derivatives (WO 2018/073321).

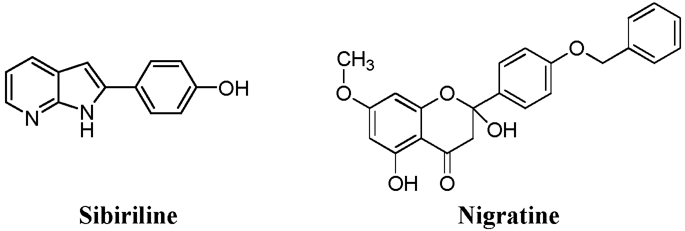

Now, the inventors have discovered that, surprisingly, when such regulated necrosis inhibitors (inhibitors of regulated necrotic cell death) are combined with N-acetylcysteine (NAC) or a derivative thereof, the combination produces cell protection effects that indicate super-additivity (i.e. synergy), achieving a much better therapeutic efficacy than when each active ingredient is used alone.

Hence, the present invention relates to a pharmaceutical composition comprising a combination of N-acetylcysteine (NAC) and/or a pharmaceutical salt and/or a derivative thereof with at least one inhibitor of regulated necrotic cell death, such as necroptosis and/or ferroptosis.

N-acetylcysteine (also named acetylcysteine or N-acetyl-L-cysteine, CAS number 616-91-1) corresponds to the following chemical formula:

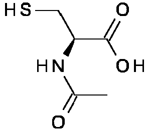

N-acetylcysteine The main therapeutic applications of NAC based on its pharmacological activity are listed below:
- In pneumology, NAC is a mucolytic-type mucomodifier. It acts on the gel phase of mucus, presumably by breaking the disulfide bridges of glycoproteins, and thus promotes expectoration.
- In ophthalmology, NAC is an inhibitor of collagenase, a proteolytic enzyme secreted in large quantities during any damage to the epithelium and causing the degradation of polypeptide fibers of corneal collagen.
- In toxicology, acetylcysteine is a precursor of glutathione, which can enter cells. This is the main way in which it protects hepatocytes. In fact, glutathione neutralizes the electrophilic entities produced by the metabolism of paracetamol.
- In neurology, several initial studies showed that administration of NAC increased glutathione levels in the brain. The benefit of NAC has recently been confirmed as part of a standard treatment for patients with Parkinson's disease. The study found improved levels of dopamine, the main neurotransmitter specifically reduced in the disease, as well as improved mental and physical abilities in patients [Monti et al., Clin Pharmacol Ther., 2019, 106(4), 884-890].

Pharmaceutically acceptable salts of NAC or derivative thereof such as N-acetylcysteine amide, N-acetylcysteine ethyl ester, N-acetylcysteine methyl ester include, but are not limited to, those formed with free amino groups such as those derived from hydrochloric, phosphoric, sulfuric, acetic, oxalic, tartaric acids, and the like, and those formed with free carboxyl groups such as those derived from sodium, potassium, ammonium, calcium, ferric hydroxides, isopropylamine, triethylamine, 2-(ethylamino)ethanol, histidine, procaine and the like.

**In** the framework of the present invention, the term "derivative of NAC" is intended to mean any compound which is structurally related to NAC and which possesses a similar pharmacological activity. For instance, the following compound, known as N-acetylcysteine amide (also named NAC amide or N-acetyl-L-cysteine amide or NACA, CAS number 38520-57-9) [Sunitha et al., Clin Pharmacol Ther., 2013, 47(5), 884-890] is a derivative of NAC:

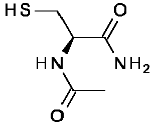

N-acetylcysteine amide In particular, the derivative of NAC of a pharmaceutical composition according to the invention is N-acetylcysteine amide.

For instance, the following compound, known as N-acetylcysteine ethyl ester (also named NAC ethyl ester or N-acetyl-L-cysteine ethyl ester, CAS number 59587-09-6) is a derivative of NAC: 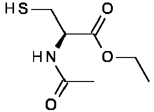 N-acetylcysteine ethyl ester

In particular, the derivative of NAC of a pharmaceutical composition according to the invention is N-acetylcysteine ethyl ester.

For instance, the following compound, known as N-acetylcysteine methyl ester (also named NAC methyl ester N-acetyl-L-cysteine methyl ester, CAS number 118398-49-5) is a derivative of NAC: 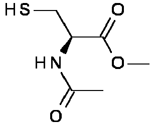 N-acetylcysteine methyl ester

In particular, the derivative of NAC of a pharmaceutical composition according to the invention is N-acetylcysteine methyl ester.

The invention thus relates to a pharmaceutical composition comprising a combination of N-acetylcysteine (NAC) and/or N-acetylcysteine amide and/or N-acetylcysteine ethyl ester and/or N-acetylcysteine methyl ester and/or a pharmaceutical salt thereof with at least one inhibitor of regulated necrotic cell death, such as necroptosis and/or ferroptosis.

For the purpose of the invention, the term "inhibitor of regulated necrotic cell death" refers to a compound which allows to preserve at least partially cells from a regulated necrotic death. In particular, when cells are exposed to a regulated necrotic cell death inducer, treatment with the inhibitor allows to improve cell viability, which can be easily verified by the skilled person using methods well known in the art, such as those described in the examples of the present description. The inhibitor of regulated necrotic cell death is a compound of general formula (I) or (II) as defined below.

The inhibitor of regulated necrotic cell death is advantageously a ferroptosis and/or a necroptosis inhibitor. In a particular embodiment, the inhibitor of regulated necrotic cell death is a ferroptosis inhibitor, preferably a ferroptosis and necroptosis inhibitor.

Tests for identifying ferroptosis inhibitors are for instance disclosed in WO2022157392, from pages 69 to 81 (experimental part, section "II. Biological Activity of the compounds of the invention). A preferred model is SH-SY5Y human neuroblastoma cell line treated with ferroptosis inducer RSL3 or erastin. A ferroptosis inhibitor is characterized by its ability to protect cells from cell death in a dose-dependent manner and to decrease lipid peroxidation induced by a ferroptosis inducer such as RSL3 or erastin for example in the model SH-SY5Y. Tests for identifying necroptosis inhibitors are for instance disclosed in page 29 of EP3362450B1, specially in paragraph [178]. A necroptosis inhibitor is characterized by its ability to protect cells from cell death induced by a necroptosis inducer, in a dose-dependent manner in this test, for example with an EC₅₀ of 25 µM or less (EC₅₀ is the half maximal effective concentration of the drug).

Examples of known ferroptosis and/or a necroptosis inhibitor are Resveratrol (CAS number 501-36-0), Ferrostatin 1 (CAS Number: 347174-05-4) or Liproxstatin (CAS Number 950455-15-9), and NEC1F (described in particular in (Tonnus et al. Nat Commun 12, 4402 (2021), DOI: 10.1038/s41467-021-24712-6). In particular, the inhibitor of regulated necrotic cell death inhibitor is NEC1F.

The inhibitor of regulated necrotic cell death (hereinafter, "the inhibitor") of a pharmaceutical composition according to the invention are sibiriline, nigratine or a derivative thereof.

### Sibiriline and derivatives thereof

In a particular embodiment, the inhibitor of a pharmaceutical composition according to the invention is sibiriline or a derivative thereof. Said inhibitor may thus correspond to any compound disclosed in WO 2017/064217, WO 2017/064216 or WO 2022/157392.

In this particular embodiment, the inhibitor is preferably a compound of general formula (I) 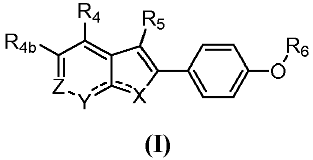 or a pharmaceutically acceptable salt and/or solvate thereof, wherein:
▪ 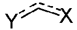 is 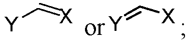
   (i) when 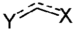 is 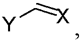 **X** is N, **Y** is N(**R₂**) and **Z** is C(H);
   (ii) when 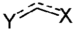 is 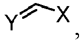
      - **X** is N(**R₁**), and
      - **Y** is N or N⁺(O⁻) and **Z** is C(**R₃**), or
         **Y** is CH and **Z** is N, or
         **Y** and **Z** are CH;
   and wherein:
   ▪ **R₁** and **R₂** represent, independently of each other, a hydrogen atom, CN, NO₂, OR₇, S**R₈**, N**R₉R₁₀**, C(O)**R₁₁**, CO₂**R₁₂**, OC(O)**R₁₃**, N**R₁₄**C(O)**R₁₅**, C(O)N**R₁₆R₁₇**, S(O)**R_{S}**, SO₂**R_{S}**', a (C₁-C₆)alkyl, a (C₁-C₆)haloalkyl, a -(C₁-C₆)alkyl-[O-(C₁-C₆)alkyl]ₘ-N**R_{N1}R_{N2}** group with m ranging from 1 to 6, an aryl, an aryl-(C₁-C₆)alkyl, a heterocyclyl, or a heterocyclyl-(C₁-C₆)alkyl group, wherein said aryl or heterocyclyl group is optionally substituted by one or more substituents selected from the group consisting of a halogen atom, CN, NO₂, O**R₁₈**, S**R₁₉**, N**R₂₀R₂₁**, C(O)**R₂₂**, CO₂**R₂₃**, OC(O)**R₂₄**, N**R₂₅**C(O)**R₂₆**, C(O)N**R₂₇R₂₈**, a (C₁-C₆)alkyl and a (C₁-C₆)haloalkyl group;
   ▪ **R₃, R₄, R_{4b}** and **R₅** represent, independently of each other, a hydrogen atom, a halogen atom, CN, **OR₂₉, SR₃₀, NR₃₁R₃₂,** C(O)**R₃₃**, CO₂**R₃₄**, OC(O)**R₃₅**, N**R₃₆**C(O)**R₃₇**, C(O)N**R₃₈R₃₉**, a (C₁-C₆)alkyl, a (C₁-C₆)haloalkyl group, said alkyl or haloalkyl group being optionally substituted by one or more substituents selected from the group consisting of O**R₄₀**, S**R₄₁** and N**R₄₂R₄₃**, an aryl, a heterocyclyl, an aryl-(C₁-C₆)alkyl or a heterocyclyl-(C₁-C₆)alkyl group, wherein said aryl or heterocyclyl group is optionally substituted by one or more substituents selected from the group consisting of a halogen atom, CN, NO₂, **OR₄₄, SR₄₅,** N**R₄₆R₄₇,** C(O)**R₄₈**, CO₂**R₄₉**, OC(O)**R₅₀**, N**R₅₁**C(O)**R₅₂**, C(O)N**R₅₃R₅₄**, a (C₁-C₆)alkyl and a (C₁-C₆)haloalkyl group;
   ▪ **R₆** represents a hydrogen atom, a (C₁-C₆)alkyl, an aryl-(C₁-C₆)alkyl, a heterocyclyl-(C₁-C₆)alkyl, a -(C₁-C₆)alkyl-[O-(C₁-C₆)alkyl]_{m'}-N**R**_{N'1}**R**_{N'2} group with m' ranging from 1 to 6, or a (C₁-C₆)alkylcarbonyl group, said (C₁-C₆)alkyl, aryl-(C₁-C₆)alkyl, and (C₁-C₆)alkylcarbonyl group being optionally substituted with one or more substituents selected from the group consisting of OH, SH, NH₂, a (C₁-C₆)alkoxy, a (C₁-C₆)thioalkoxy, a (C₁-C₆)alkylamino and a di((C₁-C₆)alkyl)amino group;
   ▪ **R_{S}** and **Rs'** represent, independently of each other a (C₁-C₆)alkyl, an aryl or an aryl-(C₁-C₆)alkyl group;
   ▪ **R₇-R₁₀, R₁₂, R₁₄** and **R₁₆-R₁₇** represent, independently of each other, a hydrogen atom, a (C₁-C₆)alkyl, an aryl or an aryl-(C₁-C₆)alkyl group;
   ▪ **R₁₁, R₁₃** and **R₁₅** represent, independently of each other, a hydrogen atom, a (C₁-C₆)alkyl, an aryl, an aryl-(C₁-C₆)alkyl, a (C₁-C₆)alkoxy, a (C₁-C₆)alkylamino or a di((C₁-C₆)alkyl)amino group;
   ▪ **R₁₈** to **R₂₈** represent, independently of each other, a hydrogen atom, a (C₁-C₆)alkyl or an aryl group;
   ▪ **R₂₉** to **R₃₉** represent, independently of each other, a hydrogen atom, a (C₁-C₆)alkyl, heterocyclyl-(C₁-C₆)alkyl, an aryl or an aryl-(C₁-C₆)alkyl group, said aryl group being optionally substituted by one or more substituents selected from the group consisting of a halogen atom, CN, NO₂, O**R₅₅,** S**R₅₆,** N**R₅₇R₅₈**, C(O)**R₅₉**, CO₂**R₆₀**, OC(O)**R₆₁**, N**R₆₂**C(O)**R₆₃**, C(O)N**R₆₄R₆₅**, a (C₁-C₆)alkyl and a (C₁-C₆)haloalkyl group;
   ▪ **R₄₀** to **R₄₃** represent, independently of each other, a hydrogen atom or a (C₁-C₆)alkyl group;
   ▪ **R₄₄** to **R₅₄** represent, independently of each other, a hydrogen atom, a (C₁-C₆)alkyl or an aryl group;
   ▪ **R₅₅** to **R₆₅** represent, independently of each other, a hydrogen atom, a (C₁-C₆)alkyl, aryl-(C₁-C₆)alkyl group or an aryl group; and
   **R_{N1}, R_{N'1}, R_{N2}** and **R_{N'2}** represent, independently of each other, a hydrogen atom, a (C₁-C₆)alkyl group, an aryl-(C₁-C₆)alkyl group or an aryl group.
▪ Preferably, in the compound of the following general formula **(I)** wherein:**R₁** and **R₂** represent, independently of each other, a hydrogen atom, CN, NO₂, O**R₇**, S**R₈**, N**R₉R₁₀**, C(O)**R₁₁**, CO₂**R₁₂**, OC(O)**R₁₃**, N**R₁₄**C(O)**R₁₅**, C(O)N**R₁₆R₁₇**, S(O)**R_{S}**, SO₂**R_{S}'**, a (C₁-C₆)alkyl, a (C₁-C₆)haloalkyl, an aryl, a heterocyclyl, an aryl-(C₁-C₆)alkyl or a heterocyclyl-(C₁-C₆)alkyl group, wherein said aryl or heterocyclyl group is optionally substituted by one or more substituents selected from the group consisting of a halogen atom, CN, NO₂, O**R**_{**18**,} S**R₁₉**, N**R₂₀R₂₁**, C(O)**R₂₂**, CO₂**R₂₃**, OC(O)**R₂₄**, N**R₂₅**C(O)**R₂₆**, C(O)N**R₂₇R₂₈**, a (C₁-C₆)alkyl and a (C₁-C₆)haloalkyl group;
▪ **R₃, R₄, R_{4b}** and **R₅** represent, independently of each other, a hydrogen atom, a halogen atom, CN, O**R₂₉,** S**R₃₀,** N**R₃₁R₃₂**, C(O)**R₃₃**, CO₂**R₃₄**, OC(O)**R₃₅**, N**R₃₆**C(O)**R₃₇**, C(O)N**R₃₈R₃₉**, a (C₁-C₆)alkyl, a (C₁-C₆)haloalkyl group, said alkyl or haloalkyl group being optionally substituted by one or more substituents selected from the group consisting of O**R₄₀**, S**R₄₁** and N**R₄₂R₄₃**, an aryl, a heterocyclyl, an aryl-(C₁-C₆)alkyl or a heterocyclyl-(C₁-C₆)alkyl group, wherein said aryl or heterocyclyl group is optionally substituted by one or more substituents selected from the group consisting of a halogen atom, CN, NO₂, O**R₄₄**, S**R₄₅**, N**R₄₆R₄₇**, C(O)**R₄₈**, CO₂**R₄₉**, OC(O)**R₅₀**, N**R₅₁**C(O)**R₅₂**, C(O)N**R₅₃R₅₄**, a (C₁-C₆)alkyl and a (C₁-C₆)haloalkyl group;
▪ **R₆** represents a hydrogen atom, a (C₁-C₆)alkyl, an aryl-(C₁-C₆)alkyl or a -CH₂-CH₂-O-CH₂-CH₂-NH₂ group, or a (C₁-C₆)alkylcarbonyl group optionally substituted with one or more substituents selected from the group consisting of OH, SH, NH₂, a (C₁-C₆)alkoxy, a (C₁-C₆)thioalkoxy, a (C₁-C₆)alkylamino and a di((C₁-C₆)alkyl)amino group;
▪ **R_{S}** and **Rs'** represent, independently of each other a (C₁-C₆)alkyl, an aryl or an aryl-(C₁-C₆)alkyl group;
▪ **R₇-R₁₀, R₁₂, R₁₄** and **R₁₆-R₁₇** represent, independently of each other, a hydrogen atom, a (C₁-C₆)alkyl, an aryl or an aryl-(C₁-C₆)alkyl group;
▪ **R₁₁, R₁₃** and **R₁₅** represent, independently of each other, a hydrogen atom, a (C₁-C₆)alkyl, an aryl, an aryl-(C₁-C₆)alkyl, a (C₁-C₆)alkoxy, a (C₁-C₆)alkylamino or a di((C₁-C₆)alkyl)amino group;
▪ **R₁₈** to **R₂₈** represent, independently of each other, a hydrogen atom, a (C₁-C₆)alkyl or an aryl group;
▪ **R₂₉** to **R₃₉** represent, independently of each other, a hydrogen atom, a (C₁-C₆)alkyl, an aryl or an aryl-(C₁-C₆)alkyl group, said aryl group being optionally substituted by one or more substituents selected from the group consisting of a halogen atom, CN, NO₂, O**R₅₅,** S**R₅₆**, N**R₅₇R₅₈**, C(O)**R₅₉**, CO₂**R₆₀**, OC(O)**R₆₁**, N**R₆₂**C(O)**R₆₃**, C(O)N**R₆₄R₆₅**, a (C₁-C₆)alkyl and a (C₁-C₆)haloalkyl group;
▪ **R₄₀** to **R₄₃** represent, independently of each other, a hydrogen atom or a (C₁-C₆)alkyl group;
▪ **R₄₄** to **R₅₄** represent, independently of each other, a hydrogen atom, a (C₁-C₆)alkyl or an aryl group; and
▪ **R₅₅** to **R₆₅** represent, independently of each other, a hydrogen atom, a (C₁-C₆)alkyl or an aryl group.

For the purpose of the invention, the term "pharmaceutically acceptable" is intended to mean what is useful to the preparation of a pharmaceutical composition, and what is generally safe and non-toxic, for a pharmaceutical use.

The term "pharmaceutically acceptable salt or solvate" is intended to mean, in the framework of the present invention, a salt or solvate of a compound which is pharmaceutically acceptable, as defined above, and which possesses the pharmacological activity of the corresponding compound.

The pharmaceutically acceptable salts comprise:
(1) acid addition salts formed with inorganic acids such as hydrochloric, hydrobromic, sulfuric, nitric and phosphoric acid and the like; or formed with organic acids such as acetic, benzenesulfonic, fumaric, glucoheptonic, gluconic, glutamic, glycolic, hydroxynaphtoic, 2-hydroxyethanesulfonic, lactic, maleic, malic, mandelic, methanesulfonic, muconic, 2-naphtalenesulfonic, propionic, succinic, dibenzoyl-L-tartaric, tartaric, p-toluenesulfonic, trimethylacetic, and trifluoroacetic acid and the like, and
(2) base addition salts formed when an acid proton present in the compound is either replaced by a metal ion, such as an alkali metal ion, an alkaline-earth metal ion, or an aluminium ion; or coordinated with an organic or inorganic base. Acceptable organic bases comprise diethanolamine, ethanolamine, N-methylglucamine, triethanolamine, tromethamine and the like. Acceptable inorganic bases comprise aluminium hydroxide, calcium hydroxide, potassium hydroxide, sodium carbonate and sodium hydroxide.

Acceptable solvates include conventional solvates such as those formed during the last step of the preparation of the compounds due to the presence of solvents.

The term "halogen", as used in the present invention, refers to a fluorine, bromine, chlorine or iodine atom.

The terms "(C₁-C₆)alkyl", as used in the present invention, refers to a straight or branched saturated hydrocarbon chain containing from 1 to 6 carbon atoms including, but not limited to, methyl, ethyl, n-propyl, iso-propyl, n-butyl, iso-butyl, sec-butyl, t-butyl, n-pentyl, n-hexyl, and the like.

The term "(C₁-C₆)haloalkyl", as used in the present invention, refers to a (C₁-C₆)alkyl group as defined above in which part or all of the hydrogen atoms is replaced with a halogen atom as defined above. This means that the (C₁-C₆)alkyl group is substituted by at least one halogen atom. It can be for example a trifluoromethyl group.

The term "aryl", as used in the present invention, refers to an aromatic hydrocarbon group comprising preferably 6 to 10 carbon atoms and comprising one or more, notably 1 or 2, fused rings, such as, for example, a phenyl or naphtyl group, advantageously a phenyl group.

The term "heterocyclic" as used in the present invention refers to a saturated, unsaturated (i.e. not aromatic) or aromatic monocyclic or bicyclic group comprising two fused, bridged or spiro rings, preferably fused rings, advantageously comprising 5 to 10, notably 5 or 6, atoms in each ring, in which the atoms of the ring(s) comprise one or more, advantageously 1 to 3, heteroatoms selected from O, S and N, preferably O and N, the remainder being carbon atoms.

A saturated heterocyclic group is more particularly a 5- or 6-membered saturated monocyclic heterocyclic group such as a pyrrolidinyl, tetrahydrofuranyl, tetrahydrothiophenyl, thiazolidinyl, isothiazolidinyl, oxazolidinyl, isoxazolidinyl, imidazolidinyl, pyrazolidinyl, triazolidinyl, piperidinyl, piperazinyl, morpholinyl or thiomorpholinyl group.

An unsaturated heterocyclic group is more particularly an unsaturated monocyclic or bicyclic heterocyclic group, each cycle comprising 5 or 6 members, such as a pyrrolinyl, dihydrofuranyl, dihydrothiophenyl, thiazolinyl, isothiazolinyl, oxazolinyl, isoxazolinyl, imidazolinyl, pyrazolinyl, triazolinyl, dihydropyridinyl, tetrahydropyridinyl, dihydropyrimidinyl, tetrahydropyrimidinyl, dihydropyridazinyl, tetrahydropyridazinyl, dihydropyrazinyl, tetrahydropyrazinyl, dihydrotriazinyl, tetrahydrotriazinyl, indolinyl, 2,3-dihydrobenzofuranyl, 2,3-dihydrobenzothiophenyl, 1,3-benzodioxolyl, 1,3-benzoxathiolyl, benzoxazolinyl, benzothiazolinyl, benzimidazolinyl, chromanyl or chromenyl group.

An aromatic heterocyclic group, also called heteroaryl group, is more particularly an aromatic monocyclic or bicyclic heterocyclic group, each cycle comprising 5 or 6 members, such as a pyrrolyl, furanyl, thiophenyl, thiazolyl, isothiazolyl, oxazolyl, isoxazolyl, imidazolyl, pyrazolyl, triazolyl, pyridinyl, pyrimidinyl, pyridazinyl, pyrazinyl, triazinyl (such as 1, 3, 5-triazinyl), indolyl, benzofuranyl, benzothiophenyl, benzothiazolyl, benzoxazolyl, benzimidazolyl, indazolyl, benzotriazolyl, purinyl, quinolinyl, isoquinolinyl, cinnolinyl, quinazolinyl or quinoxalinyl group.

The term "aryl-(C₁-C₆)alkyl", as used in the present invention, refers to an aryl group as defined above bound to the molecule via a (C₁-C₆)alkyl group as defined above. In particular, the -(C₁-C₆)alkyl-aryl group is a benzyl group.

The term "heterocyclyl -(C₁-C₆)alkyl", as used in the present invention, refers to a heterocyclyl group as defined above bound to the molecule via a (C₁-C₆)alkyl group as defined above. In particular, the -(C₁-C₆)alkyl- heterocyclyl group is 5- or 6-membered saturated monocyclic heterocyclic group as defined above bound to the molecule via a (C₁-C₆)alkyl group as defined above.

The term "(C₁-C₆)alkylcarbonyl", as used in the present invention, refers to a (C₁-C₆)alkyl group as defined above bound to the molecule via a -C(=O)- group, including, but not limited to, acetyl, propionyl, butanoyl, pentanoyl, hexanoyl and the like.

The term "(C₁-C₆)alkoxy", as used in the present invention, refers to a (C₁-C₆)alkyl group as defined above bound to the molecule via an oxygen atom, including, but not limited to, methoxy, ethoxy, n-propoxy, iso-propoxy, n-butoxy, iso-butoxy, sec-butoxy, t-butoxy, n-pentoxy, n-hexoxy, and the like.

The term "(C₁-C₆)thioalkoxy", as used in the present invention, refers to a (C₁-C₆)alkyl group as defined above bound to the molecule via a sulfur atom, including, but not limited to, thiomethoxy, thioethoxy, n-thiopropoxy, iso-thiopropoxy, n-thiobutoxy, iso-thiobutoxy, sec-thiobutoxy, t-thiobutoxy, n-thiopentoxy, n-thiohexoxy, and the like.

The term "(C₁-C₆)alkylamino", as used in the present invention, refers to a -NHAlk group with Alk representing a (C₁-C₆)alkyl group as defined above, including, but not limited to, methylamino, ethylamino, n-propylamino, iso-propylamino, n-butylamino, iso-butylamino, sec-butylamino, t-butylamino, n-pentylamino, n-hexylamino, and the like.

The term "di(C₁-C₆)alkylamino", as used in the present invention, refers to a -NAlk₁Alk₂ group with Alk₁ and Alk₂ representing, independently of one another, a (C₁-C₆)alkyl group as defined above, including, but not limited to, dimethylamino, diethylamino, ethylmethylamino and the like.

According to a particular embodiment of the present invention, **R₁** represents a hydrogen atom, CN, NO₂, O**R₇**, S**R₈**, N**R₉R₁₀**, C(O)**R₁₁**, CO₂**R₁₂**, OC(O)**R₁₃**, N**R₁₄**C(O)**R₁₅**, C(O)N**R₁₆R₁₇**, S(O)**R_{S}**, SO₂**R_{S}**', a (C₁-C₆)alkyl, a (C₁-C₆)haloalkyl, an aryl, a heterocyclyl, an aryl-(C₁-C₆)alkyl or a heterocyclyl-(C₁-C₆)alkyl group, wherein said aryl or heterocyclyl group (which may be part of a aryl-(C₁-C₆)alkyl or heterocyclyl-(C₁-C₆)alkyl group) is optionally substituted by one or more substituents, notably one susbtituent, selected from the group consisting of a halogen atom, CN, NO₂, O**R**_{**18**,} S**R₁₉**, N**R₂₀R₂₁**, a (C₁-C₆)alkyl and a (C₁-C₆)haloalkyl, notably a halogen atom, NO₂, O**R₁₈**, a (C₁-C₆)alkyl and a (C₁-C₆)haloalkyl group, in particular NO₂ and O**R₁₈**, and wherein **R₁₈** to **R₂₁** represent, independently of each other, a hydrogen atom or a (C₁-C₆)alkyl group.

According to another particular embodiment of the present invention, **R₁** represents a hydrogen atom, CN, NO₂, O**R₇**, S**R₈**, N**R₉R₁₀**, C(O)**R₁₁**, CO₂**R₁₂**, OC(O)**R₁₃**, N**R₁₄**C(O)**R₁₅**, C(O)N**R₁₆R₁₇**, a (C₁-C₆)alkyl, a (C₁-C₆)haloalkyl, an aryl, a heterocyclyl, an aryl-(C₁-C₆)alkyl or a heterocyclyl-(C₁-C₆)alkyl group, wherein said aryl or heterocyclyl group is optionally substituted by one or more substituents as defined above.

According to still another particular embodiment of the present invention, **R₁** represents a hydrogen atom, CN, O**R₇**, C(O)**R₁₁**, CO₂**R₁₂**, OC(O)**R₁₃**, SO₂**R**_{S}', a (C₁-C₆)alkyl, a heterocyclyl or a heterocyclyl-(C₁-C₆)alkyl group, wherein said heterocyclyl group (which may be part of a heterocyclyl-(C₁-C₆)alkyl group) is optionally substituted by one or more substituents, notably one substituent, selected from the group consisting of a halogen atom, CN, NO₂, O**R₁₈**, S**R₁₉**, N**R₂₀R₂₁**, a (C₁-C₆)alkyl and a (C₁-C₆)haloalkyl group, notably a halogen atom, NO₂, O**R₁₈**, a (C₁-C₆)alkyl and a (C₁-C₆)haloalkyl group, in particular NO₂ and O**R₁₈**, preferably said heterocyclyl group is optionally substituted by NO₂, and wherein **R₁₈** to **R₂₁** represent, independently of each other, a hydrogen atom or a (C₁-C₆)alkyl group.

According to yet another particular embodiment of the present invention, **R₁** represents a hydrogen atom, CN, O**R₇**, C(O)**R₁₁**, CO₂**R₁₂**, OC(O)**R₁₃**, a heterocyclyl or a heterocyclyl-(C₁-C₆)alkyl group, wherein said heterocyclyl group is optionally substituted by one or more substituents as defined above.

In a preferred embodiment, **R₁** represents a hydrogen atom or a (C₁-C₆)alkyl group, in particular a hydrogen atom or a (C₁-C₃)alkyl group, preferably a hydrogen atom.

In the above embodiments, the (C₁-C₆)alkyl group, which may be part of an aryl-(C₁-C₆)alkyl group or a heterocyclyl-(C₁-C₆)alkyl group, is preferably a (C₁-C₃)alkyl group.

In the above embodiments, the aryl group, which may be part of an aryl-(C₁-C₆)alkyl group, is preferably a phenyl group.

In the above embodiments, the heterocyclyl group, which may be part of a heterocyclyl-(C₁-C₆)alkyl group, is in particular a 5- or 6-membered, saturated, unsaturated (i.e. not aromatic) or aromatic, notably saturated or aromatic, monocyclic group, in which the atoms of the ring comprise one or more, advantageously 1 to 3, heteroatoms selected from O, S and N, preferably O and N, the remainder being carbon atoms, such as a morpholinyl, a pyridinyl or a piperazinyl, for instance a morpholinyl or pyridinyl group.

In the above embodiments, **R_{S}** and **R_{S}'** represent, independently of each other a (C₁-C₆)alkyl, an aryl or an aryl-(C₁-C₆)alkyl group, notably a (C₁-C₆)alkyl or an aryl group, in particular, an aryl group, such as a phenyl group.

In the above embodiments, **R₇-R₁₀, R₁₂, R₁₄** and **R₁₆-R₁₇** represent, independently of each other, a hydrogen atom, a (C₁-C₆)alkyl, an aryl or an aryl-(C₁-C₆)alkyl group, and **R₁₁, R₁₃** and **R₁₅** represent, independently of each other, a hydrogen atom, a (C₁-C₆)alkyl, an aryl, an aryl-(C₁-C₆)alkyl, a (C₁-C₆)alkoxy, a (C₁-C₆)alkylamino or a di((C₁-C₆)alkyl)amino group, notably a hydrogen atom, a (C₁-C₆)alkyl, an aryl, a (C₁-C₆)alkylamino or a di((C₁-C₆)alkyl)amino group, in particular a (C₁-C₃)alkyl, an aryl such as a phenyl, a (C₁-C₃)alkylamino or a di((C₁-C₃)alkyl)amino group.

In particular, in the above embodiments, **R₇** to **R₁₇** represent, independently of each other, a hydrogen atom, a (C₁-C₆)alkyl, an aryl or an aryl-(C₁-C₆)alkyl group, notably a hydrogen atom, a (C₁-C₆)alkyl or an aryl group, typically a hydrogen atom, a (C₁-C₃)alkyl or an aryl group, wherein the aryl group, which may be part of an aryl-(C₁-C₆)alkyl group, is preferably a phenyl group.

According to a particular embodiment of the present invention, **R₂** represents a hydrogen atom, CN, NO₂, O**R₇**, S**R₈**, N**R₉R₁₀**, C(O)**R₁₁**, CO₂**R₁₂**, OC(O)**R₁₃**, N**R₁₄**C(O)**R₁₅**, C(O)N**R₁₆R₁₇**, S(O)**R_{S}**, SO₂**R_{S}**', a (C₁-C₆)alkyl, a (C₁-C₆)haloalkyl, an aryl, a heterocyclyl, an aryl-(C₁-C₆)alkyl or a heterocyclyl-(C₁-C₆)alkyl group, wherein said aryl or heterocyclyl group (which may be part of a aryl-(C₁-C₆)alkyl or heterocyclyl-(C₁-C₆)alkyl group) is optionally substituted by one or more substituents, notably by one susbtituent, selected from the group consisting of a halogen atom, CN, NO₂, O**R**_{**18**,} S**R₁₉**, N**R₂₀R₂₁**, a (C₁-C₆)alkyl and a (C₁-C₆)haloalkyl, notably a halogen atom, NO₂, O**R**_{**18**,} a (C₁-C₆)alkyl and a (C₁-C₆)haloalkyl group, in particular NO₂ and O**R₁₈,** and wherein **R₁₈** to **R₂₁** represent, independently of each other, a hydrogen atom or a (C₁-C₆)alkyl group.

In the above embodiment, **R_{S}** and **R_{S}'** represent, independently of each other a (C₁-C₆)alkyl, an aryl or an aryl-(C₁-C₆)alkyl group, notably a (C₁-C₆)alkyl or an aryl group, in particular, an aryl group, such as a phenyl group.

According to another particular embodiment of the present invention, **R₂** represents a hydrogen atom, CN, NO₂, O**R₇**, S**R₈**, N**R₉R₁₀**, C(O)**R₁₁**, CO₂**R₁₂**, OC(O)**R₁₃**, N**R₁₄**C(O)**R₁₅**, C(O)N**R₁₆R₁₇**, a (C₁-C₆)alkyl, a (C₁-C₆)haloalkyl, an aryl, a heterocyclyl, an aryl-(C₁-C₆)alkyl or a heterocyclyl-(C₁-C₆)alkyl group, wherein said aryl or heterocyclyl group is optionally substituted by one or more substituents as defined above.

According to still another particular embodiment of the present invention, **R₂** represents C(O)**R₁₁**, CO₂**R₁₂,** C(O)N**R₁₆R₁₇**, a (C₁-C₆)alkyl, a (C₁-C₆)haloalkyl, an aryl, a heterocyclyl, an aryl-(C₁-C₆)alkyl or a heterocyclyl-(C₁-C₆)alkyl group, wherein said aryl or heterocyclyl group is optionally substituted by one or more substituents, notably one substituent, selected from the group consisting of a halogen atom O**R₁₈**, S**R₁₉**, N**R₂₀R₂₁**, a (C₁-C₆)alkyl and a (C₁-C₆)haloalkyl group, and wherein **R₁₈** to **R₂₁** represent, independently of each other, a hydrogen atom or a (C₁-C₆)alkyl group.

According to yet another particular embodiment of the present invention, **R₂** represents CO₂**R₁₂**, C(O)N**R₁₆R₁₇**, a (C₁-C₆)alkyl, an aryl or an aryl-(C₁-C₆)alkyl group, notably CO₂**R₁₂**, C(O)N**R₁₆R₁₇** or an aryl-(C₁-C₆)alkyl group, wherein said aryl group is optionally substituted by one or more substituents, notably one substituent, selected from the group consisting of a halogen atom, O**R₁₈**, S**R₁₉** and N**R₂₀R₂₁**, notably O**R₁₈**, and wherein **R₁₈** to **R₂₁** represent, independently of each other, a hydrogen atom or a (C₁-C₆)alkyl group.

In the above embodiments, the (C₁-C₆)alkyl group, which may be part of an aryl-(C₁-C₆)alkyl group or a heterocyclyl-(C₁-C₆)alkyl group, is preferably a (C₁-C₃)alkyl group.

In the above embodiments, the aryl group, which may be part of an aryl-(C₁-C₆)alkyl group, is preferably a phenyl group.

In the above embodiments, the heterocyclyl group, which may be part of a heterocyclyl-(C₁-C₆)alkyl group, is in particular a 5- or 6-membered, saturated, unsaturated (i.e. not aromatic) or aromatic, notably saturated, monocyclic group, in which the atoms of the ring comprise one or more, advantageously 1 to 3, heteroatoms selected from O, S and N, preferably O and N, the remainder being carbon atoms, such as a morpholinyl, a pyridinyl or a piperazinyl group, notably a piperazinyl group.

In the above embodiments, **R₇-R₁₀**, **R₁₂, R₁₄** and **R₁₆-R₁₇** represent, independently of each other, a hydrogen atom, a (C₁-C₆)alkyl, an aryl or an aryl-(C₁-C₆)alkyl group, and **R₁₁, R₁₃** and **R₁₅** represent, independently of each other, a hydrogen atom, a (C₁-C₆)alkyl, an aryl, an aryl-(C₁-C₆)alkyl, a (C₁-C₆)alkoxy, a (C₁-C₆)alkylamino or a di((C₁-C₆)alkyl)amino group, notably a hydrogen atom, a (C₁-C₆)alkyl, an aryl, a (C₁-C₆)alkylamino or a di((C₁-C₆)alkyl)amino group, in particular a (C₁-C₃)alkyl, an aryl such as a phenyl, a (C₁-C₃)alkylamino or a di((C₁-C₃)alkyl)amino group.

In particular, in the above embodiments, **R₇** to **R₁₇** represent, independently of each other, a hydrogen atom, a (C₁-C₆)alkyl, an aryl or an aryl-(C₁-C₆)alkyl group, notably a hydrogen atom, a (C₁-C₆)alkyl or an aryl-(C₁-C₆)alkyl group, typically a hydrogen atom, a (C₁-C₃)alkyl or an aryl group, wherein the aryl group, which may be part of an aryl-(C₁-C₆)alkyl group, is preferably a phenyl group.

According to another particular embodiment of the present invention, **R₃** represents a hydrogen atom, a halogen atom, a (C₁-C₆)alkyl group, CN, O**R₂₉,** S**R₃₀,** N**R₃₁R₃₂**, C(O)**R₃₃**, CO₂**R₃₄**, OC(O)**R₃₅**, N**R₃₆**C(O)**R₃₇**, C(O)N**R₃₈R₃₉**, an aryl, a heterocyclyl, an aryl-(C₁-C₆)alkyl or a heterocyclyl-(C₁-C₆)alkyl group, wherein said aryl or heterocyclyl group is optionally substituted by one or more substituents selected from the group consisting of a halogen atom, O**R₄₄**, S**R₄₅**, N**R₄₆R₄₇**, a (C₁-C₆)alkyl and a (C₁-C₆)haloalkyl group, and wherein **R₂₉** to **R₃₉** represent, independently of each other, a hydrogen atom, a (C₁-C₆)alkyl, an aryl, or an aryl-(C₁-C₆)alkyl group, notably a hydrogen atom or a (C₁-C₆)alkyl group, typically a hydrogen atom or a (C₁-C₃)alkyl group.

According to another particular embodiment of the present invention, **R₃** represents a hydrogen atom, a halogen atom, a (C₁-C₆)alkyl group, CN, O**R₂₉**, S**R₃₀**, NR₃₁**R₃₂**, C(O)**R₃₃**, CO₂**R₃₄**, OC(O)**R₃₅**, N**R₃₆**C(O)**R₃₇** or C(O)N**R₃₈R₃₉**, wherein **R₂₉** to **R₃₉** represent, independently of each other, a hydrogen atom, a (C₁-C₆)alkyl, an aryl, or an aryl-(C₁-C₆)alkyl group, notably a hydrogen atom or a (C₁-C₆)alkyl group.

According to still another particular embodiment of the present invention, **R₃** represents a hydrogen atom, a halogen atom, a (C₁-C₆)alkyl group, CN, O**R₂₉,** S**R₃₀**, N**R₃₁R₃₂**, OC(O)**R₃₅**, N**R₃₆**C(O)**R₃₇**, a heterocyclyl or a heterocyclyl-(C₁-C₆)alkyl group, wherein said heterocyclyl group is optionally substituted by one or more substituents selected from the group consisting of a halogen atom, O**R**_{**44**,} S**R₄₅**, N**R₄₆R₄₇** and a (C₁-C₆)alkyl a group, preferably, **R₃** represents a hydrogen atom, a halogen atom, CN, N**R₃₁R₃₂**, OC(O)**R₃₅**, a heterocyclyl or a heterocyclyl-(C₁-C₆)alkyl group, wherein said heterocyclyl group is optionally substituted by one or more substituents selected from the group consisting of a O**R**_{**44**,} S**R₄₅** and N**R₄₆R₄₇**, notably O**R₄₄,** and wherein **R₂₉** to **R₃₇** represent, independently of each other, a hydrogen atom, a (C₁-C₆)alkyl, an aryl, or an aryl-(C₁-C₆)alkyl group, notably a hydrogen atom or a (C₁-C₆)alkyl group, typically a hydrogen atom or a (C₁-C₃)alkyl group.

In the above embodiments, **R₄₄** to **R₄₇** represent, independently of each other, a hydrogen atom or a (C₁-C₆)alkyl group, notably a hydrogen atom or a (C₁-C₃)alkyl group.

According to yet another particular embodiment of the present invention, **R₃** represents a hydrogen atom, a halogen atom, a (C₁-C₆)alkyl group, O**R₂₉**, S**R₃₀**, N**R₃₁R₃₂**, C(O)**R₃₃**, CO₂**R₃₄,** OC(O)**R₃₅**, N**R₃₆**C(O)**R₃₇** or C(O)N**R₃₈R₃₉**, preferably a hydrogen atom, a halogen atom, O**R₂₉,** S**R₃₀**, N**R₃₁R₃₂**, OC(O)**R₃₅** or N**R₃₆**C(O)**R₃₇**, more preferably a hydrogen atom or OC(O)**R₃₅**, wherein **R₂₉** to **R₃₇** represent, independently of each other, a hydrogen atom, a (C₁-C₆)alkyl, an aryl, or an aryl-(C₁-C₆)alkyl group, notably a hydrogen atom or a (C₁-C₆)alkyl group, typically a hydrogen atom or a (C₁-C₃)alkyl group.

In a preferred embodiment, **R₃** represents a hydrogen atom, a halogen atom or a (C₁-C₆)alkyl group, in particular a hydrogen atom, a halogen atom or a (C₁-C₃)alkyl group, preferably a hydrogen atom or a halogen atom .

In the above embodiments, the aryl group, which may be part of an aryl-(C₁-C₆)alkyl group, is preferably a phenyl group.

In the above embodiments, the heterocyclyl group, which may be part of a heterocyclyl-(C₁-C₆)alkyl group, is in particular a 5- or 6-membered, saturated, unsaturated (i.e. not aromatic) or aromatic, notably aromatic, monocyclic group, in which the atoms of the ring comprise one or more, advantageously 1 to 3, heteroatoms selected from O, S and N, preferably O and N, the remainder being carbon atoms, such as a pyridinyl, a pyrimidinyl, a pyrazolyl, a piperazinyl or a piperidinyl group, for instance a a pyridinyl, a pyrimidinyl or a pyrazolyl group.

In the above embodiments, the (C₁-C₆)alkyl group, which may be part of an aryl-(C₁-C₆)alkyl group or a heterocyclyl-(C₁-C₆)alkyl group, is preferably a (C₁-C₃)alkyl group.

According to a particular embodiment of the present invention, **R₄** represents a hydrogen atom, a halogen atom, CN, O**R₂₉**, S**R₃₀**, N**R₃₁R₃₂**, a (C₁-C₆)alkyl, a (C₁-C₆)haloalkyl group, an aryl, a heterocyclyl, an aryl-(C₁-C₆)alkyl or a heterocyclyl-(C₁-C₆)alkyl group, wherein said aryl or heterocyclyl group (which may be part of a aryl-(C₁-C₆)alkyl or heterocyclyl-(C₁-C₆)alkyl group) is optionally substituted by one or more substituents, notably one substituent, selected from the group consisting of a halogen atom, CN, NO₂, O**R₄₄**, S**R₄₅**, N**R₄₆R₄₇**, C(O)**R₄₈**, CO₂**R₄₉**, OC(O)**R₅₀**, N**R₅₁**C(O)R₅₂, C(O)N**R₅₃R₅₄**, a (C₁-C₆)alkyl and a (C₁-C₆)haloalkyl group, notably a halogen atom, O**R₄₄**, S**R₄₅**, N**R₄₆R₄₇**, C(O)**R₄₈**, CO₂**R₄₉**, C(O)N**R₅₃R₅₄**, a (C₁-C₆)alkyl and a (C₁-C₆)haloalkyl group, in particular C(O)**R₄₈,** CO₂**R₄₉**, C(O)N**R₅₃R₅₄**, a (C₁-C₆)alkyl and a (C₁-C₆)haloalkyl group, preferably C(O)**R₄₈** and a (C₁-C₆)alkyl group.

According to another particular embodiment of the present invention, **R₄** represents a hydrogen atom, a halogen atom, CN, O**R₂₉**, S**R₃₀**, N**R₃₁R₃₂**, a (C₁-C₆)alkyl, a (C₁-C₆)haloalkyl group, an aryl, a heterocyclyl, an aryl-(C₁-C₆)alkyl or a heterocyclyl-(C₁-C₆)alkyl group, wherein said aryl or heterocyclyl group is optionally substituted by one or more substituents as defined above.

According to still another particular embodiment of the present invention, **R₄** represents a hydrogen atom, a halogen atom, O**R₂₉**, S**R₃₀**, N**R₃₁R₃₂**, a (C₁-C₆)alkyl, an aryl, or a heterocyclyl group, wherein said aryl or heterocyclyl group is optionally substituted by one or more substituents, notably one substituent, selected from the group consisting of a halogen atom, O**R₄₄**, S**R₄₅**, N**R₄₆R₄₇**, C(O)**R₄₈**, CO₂**R₄₉**, C(O)N**R₅₃R₅₄**, a (C₁-C₆)alkyl and a (C₁-C₆)haloalkyl group, in particular C(O)**R₄₈**, CO₂**R₄₉**, C(O)N**R₅₃R₅₄**, a (C₁-C₆)alkyl and a (C₁-C₆)haloalkyl group, preferably C(O)**R₄₈** and a (C₁-C₆)alkyl group.

According to yet another particular embodiment of the present invention, **R₄** represents a hydrogen atom, a halogen atom, N**R₃₁R₃₂**, a (C₁-C₆)alkyl, an aryl or a heterocyclyl group, wherein said aryl or heterocyclyl group is optionally substituted by one or more substituents, notably one substituent, selected from the group consisting of C(O)**R₄₈**, CO₂**R₄₉**, C(O)N**R₅₃R₅₄**, a (C₁-C₆)alkyl and a (C₁-C₆)haloalkyl group, preferably C(O)**R₄₈** and a (C₁-C₆)alkyl group.

In a preferred embodiment, **R₄** represents a hydrogen atom, a halogen atom or a (C₁-C₆)alkyl group, in particular a hydrogen atom or a (C₁-C₃)alkyl group, preferably a hydrogen atom.

In the above embodiments, the aryl group, which may be part of an aryl-(C₁-C₆)alkyl group, is preferably a phenyl group.

In the above embodiments, the heterocyclyl group, which may be part of a heterocyclyl-(C₁-C₆)alkyl group, is in particular a 5- or 6-membered, saturated, unsaturated (i.e. not aromatic) or aromatic, notably saturated, monocyclic group, in which the atoms of the ring comprise one or more, advantageously 1 to 3, heteroatoms selected from O, S and N, preferably O and N, the remainder being carbon atoms, such as a piperazinyl, a piperidinyl, a pyridinyl, a pyrimidinyl or a pyrazolyl group, for instance a piperazinyl or a piperidinyl group.

In the above embodiments, **R₂₉** to **R₃₂** represent, independently of each other, a hydrogen atom, a (C₁-C₆)alkyl, an aryl or an aryl-(C₁-C₆)alkyl group, said aryl group, which may be part of an aryl-(C₁-C₆)alkyl group, being preferably a phenyl and being optionally substituted by one or more substituents, notably one substituent, selected from the group consisting of a halogen atom, CN, NO₂, O**R₅₅**, S**R₅₆**, N**R₅₇R₅₈**, C(O)**R₅₉**, CO₂**R₆₀**, OC(O)**R₆₁**, N**R₆₂**C(O)**R₆₃**, C(O)N**R₆₄R₆₅**, a (C₁-C₆)alkyl and a (C₁-C₆)haloalkyl group, advantageously a halogen atom, O**R₅₅**, S**R₅₆**, N**R₅₇R₅₈**, C(O)**R₅₉**, CO₂**R₆₀**, OC(O)**R₆₁**, N**R₆₂**C(O)**R₆₃**, C(O)N**R₆₄R₆₅**, a (C₁-C₆)alkyl and a (C₁-C₆)haloalkyl group, notably C(O)R₅₉, CO₂**R₆₀**, C(O)N**R₆₄R₆₅**, a (C₁-C₆)alkyl and a (C₁-C₆)haloalkyl group, in particular C(O)**R₅₉**, wherein **R₅₅** to **R₆₅** represent, independently of each other, a hydrogen atom, a (C₁-C₆)alkyl or an aryl group, notably an aryl group, preferably a phenyl group.

In the above embodiments, **R₄₄** to **R₅₄** represent, independently of each other, a hydrogen atom, a (C₁-C₆)alkyl or an aryl group, notably an aryl group, preferably a phenyl group.

In the above embodiments, the (C₁-C₆)alkyl group, which may be part of an aryl-(C₁-C₆)alkyl group or a heterocyclyl-(C₁-C₆)alkyl group, is preferably a (C₁-C₃)alkyl group.

According to a particular embodiment of the present invention, **R_{4b}** represents a hydrogen atom, a halogen atom, O**R₂₉,** S**R₃₀**, N**R₃₁R₃₂**, a (C₁-C₆)alkyl, a (C₁-C₆)haloalkyl group, an aryl, a heterocyclyl, an aryl-(C₁-C₆)alkyl or a heterocyclyl-(C₁-C₆)alkyl group, wherein said aryl or heterocyclyl group (which may be part of a aryl-(C₁-C₆)alkyl or heterocyclyl-(C₁-C₆)alkyl group) is optionally substituted by one or more substituents, notably one substituent, selected from the group consisting of a halogen atom, CN, NO₂, O**R**_{**44**,} S**R₄₅**, N**R₄₆R₄₇,** C(O)**R₄₈**, CO₂**R₄₉**, OC(O)**R₅₀,** N**R₅₁**C(O)**R₅₂**, C(O)N**R₅₃R₅₄**, a (C₁-C₆)alkyl and a (C₁-C₆)haloalkyl group, notably a halogen atom, O**R**_{**44**,} S**R₄₅**, N**R₄₆R₄₇**, C(O)**R₄₈**, CO₂**R₄₉**, C(O)N**R₅₃R₅₄**, a (C₁-C₆)alkyl and a (C₁-C₆)haloalkyl group, in particular C(O)**R₄₈**, CO₂**R₄₉**, C(O)N**R₅₃R₅₄**, a (C₁-C₆)alkyl and a (C₁-C₆)haloalkyl group, preferably C(O)**R₄₈** and a (C₁-C₆)alkyl group.

According to a particular embodiment of the present invention, **R_{4b}** represents a hydrogen atom, a halogen atom, O**R₂₉**, S**R₃₀,** N**R₃₁R₃₂**, a (C₁-C₆)alkyl, an aryl, or a heterocyclyl group, wherein said aryl or heterocyclyl group is optionally substituted by one or more substituents, notably one substituent, selected from the group consisting of a halogen atom, O**R**_{**44**,} S**R₄₅**, N**R₄₆R₄₇**, C(O)**R₄₈**, CO₂**R₄₉**, C(O)N**R₅₃R₅₄**, a (C₁-C₆)alkyl and a (C₁-C₆)haloalkyl group, in particular C(O)**R₄₈**, CO₂**R₄₉,** C(O)N**R₅₃R₅₄**, a (C₁-C₆)alkyl and a (C₁-C₆)haloalkyl group, preferably C(O)**R₄₈** and a (C₁-C₆)alkyl group.

According to yet another particular embodiment of the present invention, **R_{4b}** represents a hydrogen atom, a halogen atom, N**R₃₁R₃₂**, a (C₁-C₆)alkyl, an aryl or a heterocyclyl group, wherein said aryl or heterocyclyl group is optionally substituted by one or more substituents, notably one substituent, selected from the group consisting of C(O)**R₄₈**, CO₂**R₄₉**, C(O)N**R₅₃R₅₄**, a (C₁-C₆)alkyl and a (C₁-C₆)haloalkyl group, preferably C(O)**R₄₈** and a (C₁-C₆)alkyl group.

According to still another particular embodiment of the present invention, **R_{4b}** represents a hydrogen atom, a halogen atom, a (C₁-C₆)alkyl group, O**R₂₉** or N**R₃₁R₃₂**, preferably a hydrogen atom, a halogen atom, a (C₁-C₃)alkyl group or O**R₂₉**, more preferably a hydrogen atom.

In a preferred embodiment, **R_{4b}** represents a hydrogen atom, a halogen atom or a (C₁-C₆)alkyl group, in particular a hydrogen atom or a (C₁-C₃)alkyl group, preferably a hydrogen atom.

In the above embodiments, the aryl group, which may be part of an aryl-(C₁-C₆)alkyl group, is preferably a phenyl group.

In the above embodiments, the heterocyclyl group, which may be part of a heterocyclyl-(C₁-C₆)alkyl group, is in particular a 5- or 6-membered, saturated, unsaturated (i.e. not aromatic) or aromatic, notably saturated, monocyclic group, in which the atoms of the ring comprise one or more, advantageously 1 to 3, heteroatoms selected from O, S and N, preferably O and N, the remainder being carbon atoms.

In the above embodiments, **R₂₉** to **R₃₂** represent, independently of each other, a hydrogen atom, a (C₁-C₆)alkyl, an aryl or an aryl-(C₁-C₆)alkyl group, said aryl group, which may be part of an aryl-(C₁-C₆)alkyl group, being preferably a phenyl and being optionally substituted by one or more substituents, notably one substituent, selected from the group consisting of a halogen atom, CN, NO₂, O**R₅₅**, S**R₅₆**, N**R₅₇R₅₈**, C(O)**R₅₉,** CO₂**R₆₀**, OC(O)**R₆₁,** N**R₆₂**C(O)**R₆₃**, C(O)N**R₆₄R₆₅**, a (C₁-C₆)alkyl and a (C₁-C₆)haloalkyl group, advantageously a halogen atom, O**R₅₅**, S**R₅₆**, N**R₅₇R₅₈**, C(O)**R₅₉**, CO₂**R₆₀,** OC(O)**R₆₁**, N**R₆₂**C(O)**R₆₃**, C(O)N**R₆₄R₆₅,** a (C₁-C₆)alkyl and a (C₁-C₆)haloalkyl group, notably C(O)**R₅₉**, CO₂**R₆₀**, C(O)N**R₆₄R₆₅**, a (C₁-C₆)alkyl and a (C₁-C₆)haloalkyl group, in particular C(O)**R₅₉**, wherein **R₅₅** to **R₆₅** represent, independently of each other, a hydrogen atom, a (C₁-C₆)alkyl or an aryl group, notably an aryl group, preferably a phenyl group. Preferably, R₂₉ to **R₃₂** represent, independently of each other, a hydrogen atom or a (C₁-C₆)alkyl group, notably a hydrogen atom.

In the above embodiments, **R₄₄** to **R₅₄** represent, independently of each other, a hydrogen atom, a (C₁-C₆)alkyl or an aryl group, notably an aryl group, preferably a phenyl group.

In the above embodiments, the (C₁-C₆)alkyl group, which may be part of an aryl-(C₁-C₆)alkyl group or a heterocyclyl-(C₁-C₆)alkyl group, is preferably a (C₁-C₃)alkyl group.

In a particular embodiment of the present invention, **R₄** is as defined above and **R_{4b}** represents a hydrogen atom, a halogen atom, O**R₂₉** or N**R₃₁R₃₂,** wherein **R₂₉** to **R₃₂** represent, independently of each other, a hydrogen atom or a (C₁-C₆)alkyl group, notably a hydrogen atom, preferably **R_{4b}** represents a hydrogen atom, a halogen atom or O**R₂₉**, more preferably a hydrogen atom.

In a particular embodiment of the present invention, **R₅** represents a hydrogen atom, a halogen atom, CN, O**R₂₉**, S**R₃₀**, N**R₃₁R₃₂**, a (C₁-C₆)alkyl, a (C₁-C₆)haloalkyl group, said alkyl or haloalkyl group being optionally substituted by one or more substituents selected from the group consisting of O**R_{40,}** S**R₄₁** and N**R₄₂R₄₃**, an aryl, a heterocyclyl, an aryl-(C₁-C₆)alkyl or a heterocyclyl-(C₁-C₆)alkyl group, wherein said aryl or heterocyclyl group (which may be part of a aryl-(C₁-C₆)alkyl or heterocyclyl-(C₁-C₆)alkyl group) is optionally substituted by one or more substituents, notably one substituent, selected from the group consisting of a halogen atom, CN, NO₂, O**R**_{**44**,} S**R₄₅,** N**R₄₆R₄₇**, C(O)**R₄₈**, CO₂**R₄₉**, OC(O)**R₅₀**, N**R₅₁**C(O)**R₅₂**, C(O)N**R₅₃R₅₄**, a (C₁-C₆)alkyl and a (C₁-C₆)haloalkyl group, notably a halogen atom, O**R₄₄**, S**R₄₅**, N**R₄₆R₄₇**, C(O)**R₄₈**, CO₂**R₄₉**, C(O)N**R₅₃R₅₄**, a (C₁-C₆)alkyl and a (C₁-C₆)haloalkyl group, in particular a halogen atom, O**R**_{**44**,} S**R₄₅**, N**R₄₆R₄₇**, a (C₁-C₆)alkyl and a (C₁-C₆)haloalkyl group, notably a halogen atom, a (C₁-C₆)alkyl and a (C₁-C₆)haloalkyl group, preferably a (C₁-C₆)alkyl group.

In the above embodiment, **R₂₉** to **R₃₂** represent, independently of each other, a hydrogen atom, a (C₁-C₆)alkyl, an aryl or an aryl-(C₁-C₆)alkyl group, said aryl group, which may be part of an aryl-(C₁-C₆)alkyl group, being preferably a phenyl and being optionally substituted by one or more substituents, notably one substituent, selected from the group consisting of a halogen atom, CN, NO₂, OR₅₅, SR₅₆, N**R₅₇R₅₈**, C(O)R₅₉, CO₂**R₆₀**, OC(O)**R₆₁**, N**R₆₂**C(O)**R₆₃**, C(O)N**R₆₄R₆₅**, a (C₁-C₆)alkyl and a (C₁-C₆)haloalkyl group, advantageously a halogen atom, OR₅₅, SR₅₆, N**R₅₇R₅₈**, C(O)R₅₉, CO₂**R₆₀**, OC(O)**R₆₁**, N**R₆₂**C(O)**R₆₃**, C(O)N**R₆₄R₆₅**, a (C₁-C₆)alkyl and a (C₁-C₆)haloalkyl group, notably C(O)R₅₉, CO₂**R₆₀**, C(O)N**R₆₄R₆₅**, a (C₁-C₆)alkyl and a (C₁-C₆)haloalkyl group, in particular C(O)R₅₉, wherein **R₅₅** to **R₆₅** represent, independently of each other, a hydrogen atom, a (C₁-C₆)alkyl or an aryl group, notably an aryl group, preferably a phenyl group. Preferably, **R₂₉** to **R₃₂** represent, independently of each other, independently of each other, a hydrogen atom or a (C₁-C₆)alkyl group.

In another particular embodiment of the present invention, **R₅** represents a hydrogen atom, a halogen atom, a (C₁-C₆)alkyl, a (C₁-C₆)haloalkyl group, said alkyl or haloalkyl group being optionally substituted by one or more substituents, notably one substituent, selected from the group consisting of O**R₄₀**, **SR₄₁** and N**R₄₂R₄₃**, an aryl-(C₁-C₆)alkyl or a heterocyclyl-(C₁-C₆)alkyl group, wherein said aryl or heterocyclyl group is optionally substituted by one or more substituents selected from the group consisting of a halogen atom, O**R₄₄**, S**R₄₅**, N**R₄₆R₄₇**, a (C₁-C₆)alkyl and a (C₁-C₆)haloalkyl group, notably a halogen atom, a (C₁-C₆)alkyl and a (C₁-C₆)haloalkyl group, preferably a (C₁-C₆)alkyl group.

In yet another particular embodiment of the present invention, **R₅** represents a hydrogen atom, a halogen atom, a (C₁-C₆)alkyl, a (C₁-C₆)haloalkyl group or a heterocyclyl-(C₁-C₆)alkyl group, said alkyl or haloalkyl group being optionally substituted by O**R₄₀**, and said heterocyclyl being optionally substituted by one or more (C₁-C₆)alkyl group.

In a preferred embodiment, **R₅** represents a hydrogen atom, a halogen atom or a (C₁-C₆)alkyl group, in particular a hydrogen atom or a (C₁-C₃)alkyl group.

Preferably, **R₅** represents a hydrogen atom.

In the above embodiments, the aryl group, which may be part of an aryl-(C₁-C₆)alkyl group, is preferably a phenyl group.

In the above embodiments, the heterocyclyl group, which may be part of a heterocyclyl-(C₁-C₆)alkyl group, is in particular a 5- or 6-membered, saturated, unsaturated (i.e. not aromatic) or aromatic, notably saturated, monocyclic group, in which the atoms of the ring comprise one or more, advantageously 1 to 3, heteroatoms selected from O, S and N, preferably O and N, the remainder being carbon atoms, such as a piperazinyl group.

In the above embodiments, **R₄₀** to **R₄₃** represent, independently of each other, a hydrogen atom or a (C₁-C₆)alkyl group, notably a hydrogen atom.

In the above embodiments, **R₄₄** to **R₅₄** represent, independently of each other, a hydrogen atom, a (C₁-C₆)alkyl or an aryl group, notably an aryl group, preferably a phenyl group.

In the above embodiments, the (C₁-C₆)alkyl group, which may be part of an aryl-(C₁-C₆)alkyl group or a heterocyclyl-(C₁-C₆)alkyl group, is preferably a (C₁-C₃)alkyl group.

In a particular embodiment of the present invention, **R₆** represents a hydrogen atom, a (C₁-C₃)alkyl, an aryl-(C₁-C₃)alkyl or a -CH₂-CH₂-O-CH₂-CH₂-NH₂ group, or a (C₁-C₆)alkylcarbonyl group optionally substituted with one or more substituents selected from the group consisting of OH, SH, NH₂, a (C₁-C₃)alkoxy, a (C₁-C₃)thioalkoxy and a (C₁-C₃)alkylamino group, preferably **R₆** represents a hydrogen atom, a methyl, an ethyl, a benzyl, a - CH₂-CH₂-O-CH₂-CH₂-NH₂ or a (C₁-C₆)alkylcarbonyl group optionally substituted with one or more substituents selected from the group consisting of OH, NH₂ and SH, in particular **R₆** represents a hydrogen atom, a -CH₂-CH₂-O-CH₂-CH₂-NH₂ or an ethyl group, notably an ethyl group.

In a preferred embodiment, **R₆** represents a hydrogen atom, a (C₁-C₃)alkyl or a -CH₂-CH₂-O-CH₂-CH₂-NH₂ group, in particular a hydrogen atom or a -CH₂-CH₂-O-CH₂-CH₂-NH₂ group.

In a first aspect of the present invention, 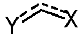 is 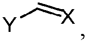 **X** is N, **Y** is N(**R₂**) and **Z** is C(H), and the inhibitor of a pharmaceutical composition according to the invention is thus of the following general formula **(I.i):** 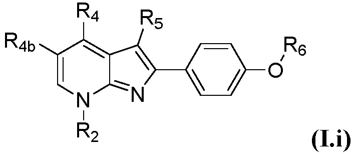 wherein **R₂, R₄, R_{4b}, R₅** and **R₆** are as defined in any one of the above embodiments.

In particular, **R₆** represents a hydrogen atom or a (C₁-C₆)alkyl group, preferably a (C₁-C₃)alkyl group, notably a methyl or an ethyl group, advantageously **R₆** represents an ethyl group.

In a second aspect of the present invention, 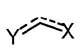 is 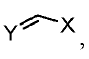 , **X** is N(**R₁**), Y is N and **Z** is C(**R₃**), and the inhibitor of a pharmaceutical composition according to the invention is thus of the following general formula **(I.ii.a):** 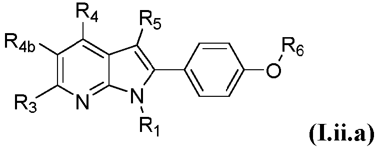 wherein **R₁, R₃, R₄, R_{4b}, R₅** and **R₆** are as defined in any one of the above embodiments.

In particular, **R₆** represents a hydrogen atom, a (C₁-C₃)alkyl such as an ethyl, a -CH₂-CH₂-O-CH₂-CH₂-NH₂ or a (C₁-C₆)alkylcarbonyl group optionally substituted with one or more substituents selected from the group consisting of OH, NH₂ and SH, advantageously **R₆** represents a hydrogen atom, a -CH₂-CH₂-O-CH₂-CH₂-NH₂ or a (C₁-C₃)alkyl such as an ethyl group.

In a third aspect of the present invention, 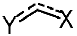 is 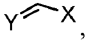 **X** is N**(R₁), Y** is N⁺(O⁻) and **Z** is C**(R₃),** and the inhibitor of a pharmaceutical composition according to the invention is thus of the following general formula **(I.ii.b):** 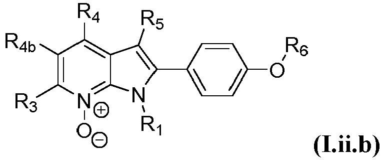 wherein **R₁, R₃, R₄, R_{4b}, R₅** and **R₆** are as defined in any one of the above embodiments.

In particular, **R₆** represents a hydrogen atom or a (C₁-C₆)alkyl group, preferably a (C₁-C₃)alkyl group, notably a methyl or an ethyl group, advantageously **R₆** represents an ethyl group.

In a fourth aspect of the present invention, 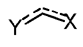 is 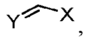 , **X** is N**(R₁), Y** is CH and **Z** is N, and the inhibitor of a pharmaceutical composition according to the invention is thus of the following general formula (I.ii.c): 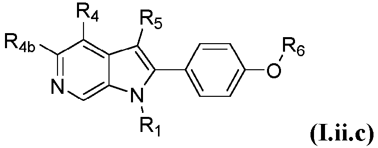 wherein **R₁, R₄, R_{4b}, R₅** and **R₆** are as defined in any one of the above embodiments.

In particular, **R₆** represents a hydrogen atom or a (C₁-C₆)alkyl group, preferably a (C₁-C₃)alkyl group, notably a methyl or an ethyl group, advantageously **R₆** represents an ethyl group.

In a fifth aspect of the present invention, 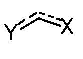 is 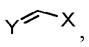 , **X** is N**(R₁)** and Y and **Z** are CH, and the inhibitor of a pharmaceutical composition according to the invention is thus of the following general formula **(I.ii.d):** 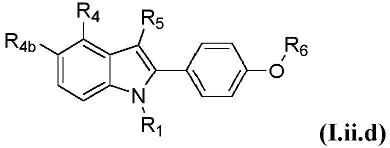 wherein **R₁, R₄, R_{4b}, R₅** and **R₆** are as defined in any one of the above embodiments.

In particular, **R₆** represents a hydrogen atom, a (C₁-C₆)alkyl group or an aryl-(C₁-C₆)alkyl group, preferably a hydrogen atom, a (C₁-C₃)alkyl group or an aryl-(C₁-C₃)alkyl group, notably a hydrogen atom, a methyl, an ethyl or a benzyl group, advantageously **R₆** represents a hydrogen atom, a methyl or a benzyl group, typically a hydrogen atom.

In a preferred embodiment, the inhibitor is a compound of the following general formula **(I.iii):** 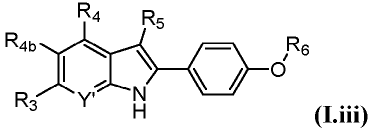 wherein **R₃, R₄, R_{4b}, R₅** and **R₆** are as defined in any one of the above embodiments, and wherein **Y'** is N or CH.

In particular:
- **R₃, R₄, R_{4b}** and **R₅** represent, independently of each other, a hydrogen atom, a halogen atom or a (C₁-C₆)alkyl group, in particular a hydrogen atom, a halogen atom or a (C₁-C₃)alkyl group, preferably a hydrogen atom or a halogen atom, and
- **R₆** represents a hydrogen atom, a (C₁-C₃)alkyl or a -CH₂-CH₂-O-CH₂-CH₂-NH₂ group, in particular a hydrogen atom or a -CH₂-CH₂-O-CH₂-CH₂-NH₂ group.

The inhibitor of a pharmaceutical composition according to the invention may notably be selected from the group consisting of compounds **1** to **45,** represented below, and the pharmaceutically acceptable salts and/or solvates thereof.

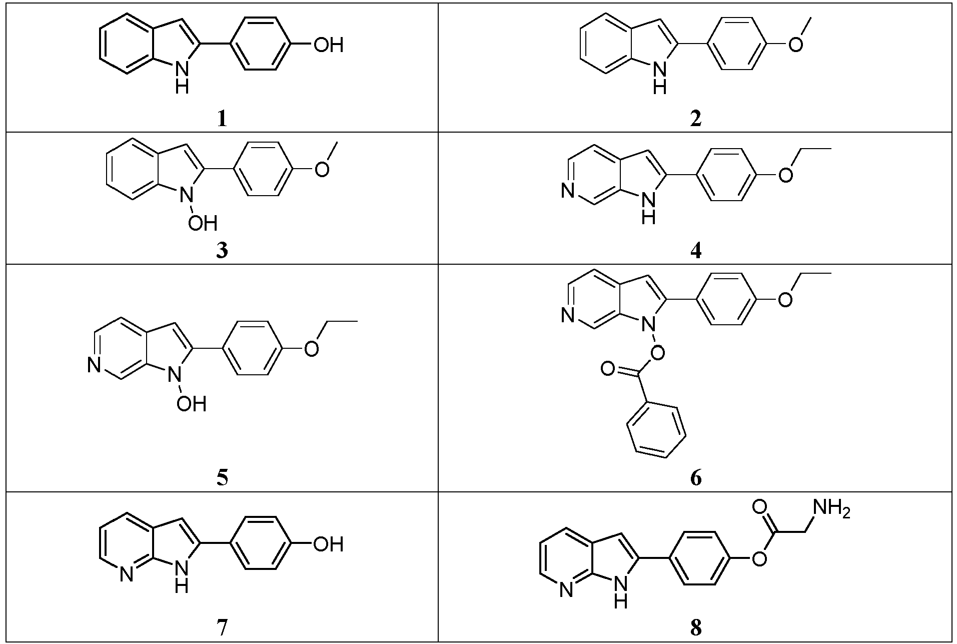

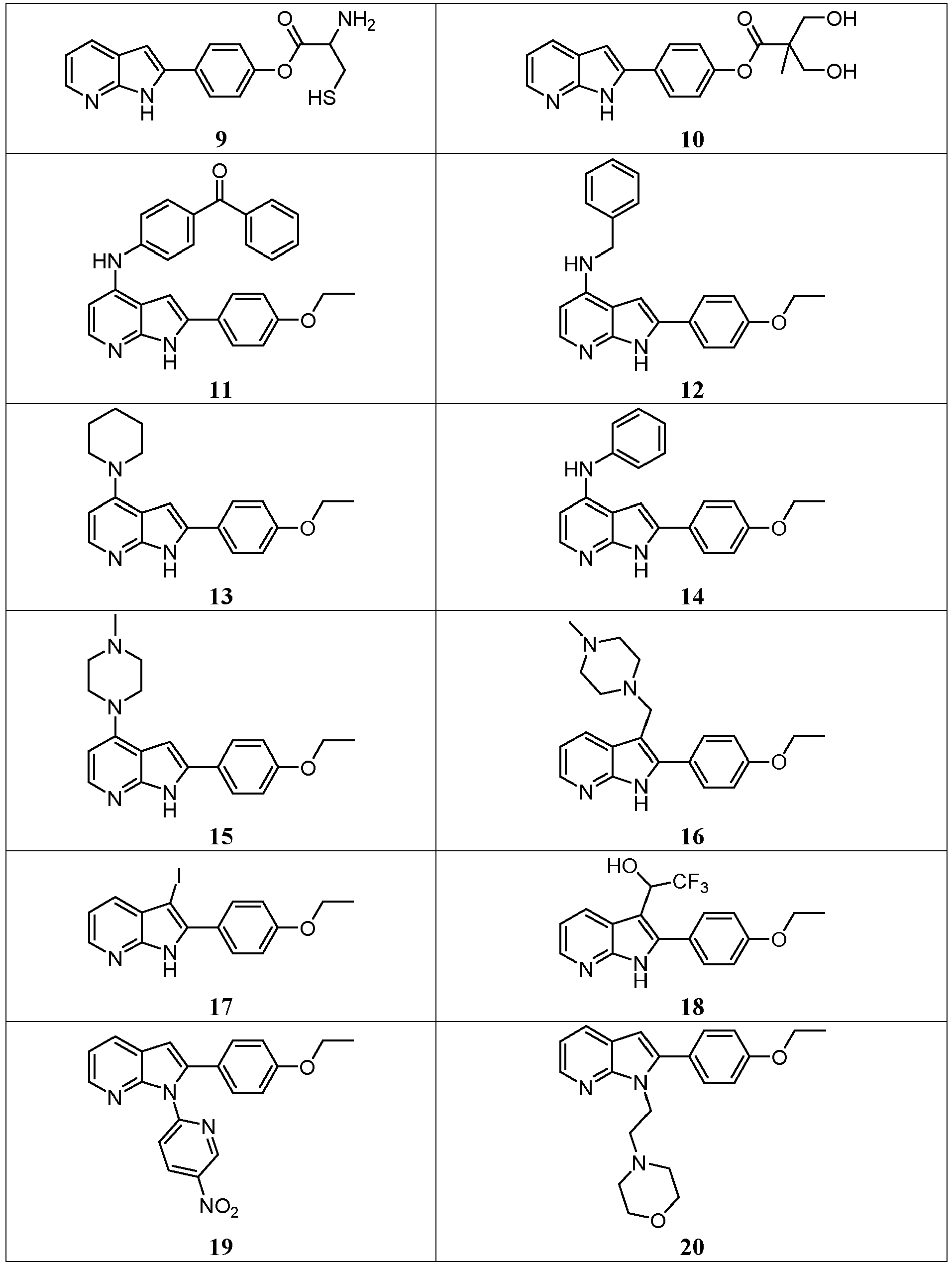

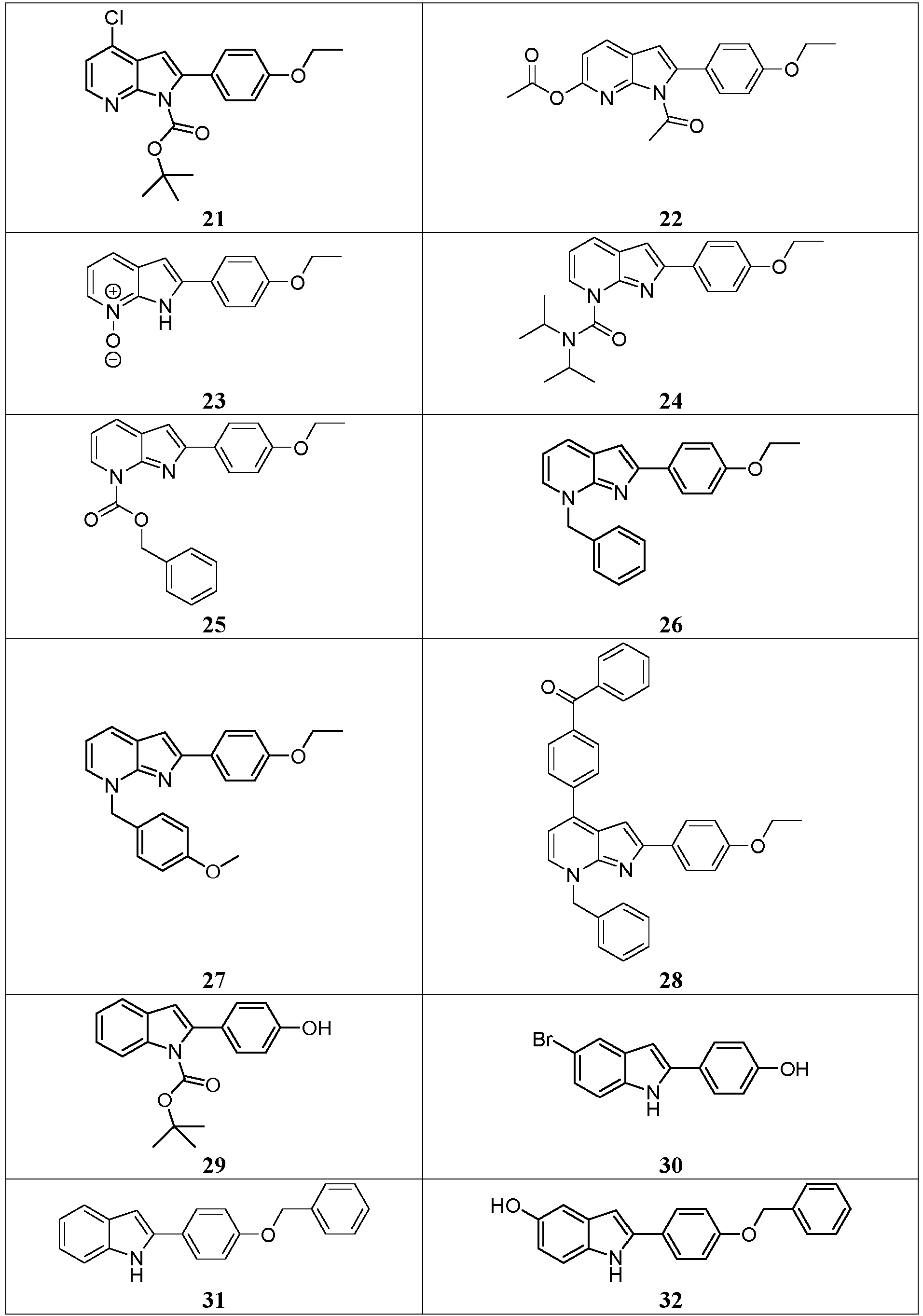

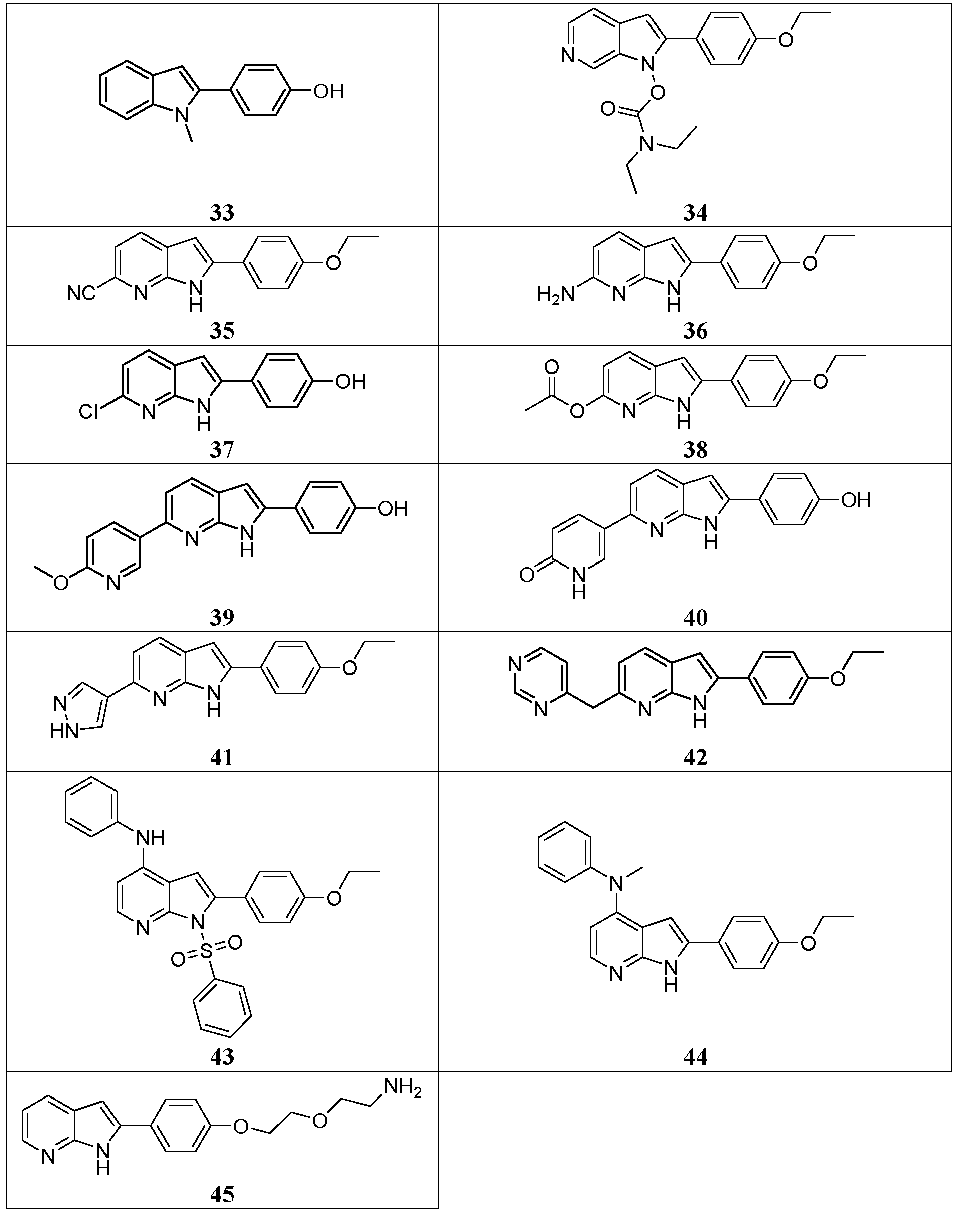

In particular, the inhibitor of a pharmaceutical composition according to the invention may be selected from the group consisting of compounds **1, 7, 37, 45** and the pharmaceutically acceptable salts and/or solvates thereof.

### Nigratine and derivatives thereof

In a particular embodiment, the inhibitor of a pharmaceutical composition according to the invention is nigratine or a derivative thereof.

In this particular embodiment, the inhibitor is preferably a compound of the following general formula **(II):** 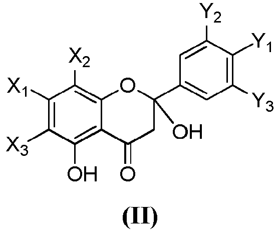 or a pharmaceutically acceptable salt and/or solvate thereof, wherein:
▪ **X₁, X₂** and **X₃** represent, independently of each other, a hydrogen atom, a (C₁-C₆)alkyl, an aryl, an aryl-(C₁-C₆)alkyl or an OH group, or a group selected from O**R_{X},** S**R_{X},** SO₂**R_{X}** and N**R_{X}R_{Z},**
   wherein at least one of **X₁, X₂** and **X₃** represents a (C₁-C₆)alkyl, an aryl or an aryl-(C₁-C₆)alkyl group, or a group selected from O**R_{X},** S**R_{X},** SO₂**R_{X}** and N**R_{X}R_{Z},** wherein
   **R_{X}** a (C₁-C₆)alkyl, an aryl or an aryl-(C₁-C₆)alkyl group,
   **R_{Z}** is a hydrogen atom or a (C₁-C₆)alkyl group, and
   the aryl groups are optionally substituted with one or several groups selected from a halogen atom, -O**R₆₆,** -N**R₆₇R₆₈,** -S**R₆₉,** -S(O)**R₇₀,** -SO₂**R₇₁,** -OCO**R₇₂,** -CO₂**R₇₃,** -CON**R₇₄R₇₅,** - CO₂**R₇₆,** nitro (-NO₂) and cyano (-CN);
▪ **Y₁, Y₂** and **Y₃** represent, independently of each other, a hydrogen atom, a (C₁-C₆)alkyl, an aryl, an aryl-(C₁-C₆)alkyl or an OH group, or a group selected from O**R_{Y},** S**R_{Y},** SO₂**R_{Y}** and N**R_{Y}R'_{Z},**
   wherein at least one of **Y₁, Y₂** and **Y₃** represents a (C₁-C₆)alkyl, an aryl or an aryl-(C₁-C₆)alkyl group, or a group selected from O**R_{Y},** S**R_{Y},** SO₂**R_{Y}** and N**R_{Y}R'_{Z},** wherein
   **R_{Y}** is a (C₁-C₆)alkyl, an aryl or an aryl-(C₁-C₆)alkyl group,
   **R'_{Z}** is a hydrogen atom or a (C₁-C₆)alkyl group, and
   the aryl groups are optionally substituted with one or several groups selected from a halogen atom, -O**R₆₆,** -N**R₆₇R₆₈,** -S**R₆₉,** -S(O)**R₇₀,** -SO₂**R₇₁,** -OCO**R₇₂,** -CO₂**R₇₃,** -CON**R₇₄R₇₅,** - CO₂**R₇₆,** nitro (-NO₂) and cyano (-CN);
      and
▪ **R₆₆** to **R₇₇** are, independently of one another, a hydrogen atom or a (C₁-C₆)alkyl group.

According to a particular embodiment of the present invention, **X₁, X₂** and **X₃** represent, independently of each other, a hydrogen atom, or a group selected from O**R_{X},** S**R_{X},** SO₂**R_{X}** and N**R_{X}R_{Z},** wherein at least one of **X₁, X₂** and **X₃** is not a hydrogen atom.

In another particular embodiment of the present invention, **X₁, X₂** and **X₃** represent, independently of each other, a hydrogen atom, a (C₁-C₆)alkyl, an aryl, an aryl-(C₁-C₆)alkyl group, an OH or an O**R_{X}** group, wherein at least one of **X₁, X₂** and **X₃** represents an O**R_{X}** group.

In still another particular embodiment of the present invention, **X₁, X₂** and **X₃** represent, independently of each other, a hydrogen atom or an O**R_{X}** group, wherein at least one of **X₁, X₂** and **X₃** represents an O**R_{X}** group.

In the above embodiments, **R_{X}** is preferably a (C₁-C₆)alkyl group, notably a (C₁-C₃)alkyl group such as methyl, ethyl, n-propyl, more preferably methyl.

In the above embodiments, the aryl groups are optionally substituted with one or several groups selected from a halogen atom, -O**R₆₆**, -N**R₆₇R₆₈**, -S**R₆₉**, -S(O)**R₇₀**, -SO₂**R₇₁**, -OCO**R₇₂**, - CO₂**R₇₃**, -CON**R₇₄R₇₅**, -CO₂**R₇₆**, nitro (-NO₂) and cyano (-CN).

In another embodiment, **X₁** represents a (C₁-C₆)alkyl, an aryl or an aryl-(C₁-C₆)alkyl group, or a group selected from O**R**_{X}, SR_{X}, SO₂**R_{X}** and N**R_{X}R_{Z}**, wherein **R_{X}** is selected from a (C₁-C₆)alkyl, an aryl and an aryl-(C₁-C₆)alkyl group, **R_{X}** being preferably a (C₁-C₆)alkyl group, notably a (C₁-C₃)alkyl group such as methyl, ethyl, n-propyl, more preferably methyl; and **X₂** and **X₃** represent, independently of each other, a hydrogen atom or a (C₁-C₆)alkyl group, preferably a hydrogen atom.

In still another embodiment, **X₁** represents a group selected from O**R_{X},** S**R_{X},** SO₂**R_{X}** and N**R_{X}R_{Z},** wherein **R_{X}** is selected from a (C₁-C₆)alkyl, an aryl and an aryl-(C₁-C₆)alkyl group, **R_{X}** being preferably a (C₁-C₆)alkyl group, notably a (C₁-C₃)alkyl group such as methyl, ethyl, n-propyl, more preferably methyl; and **X₂** and **X₃** represent, independently of each other, a hydrogen atom or a (C₁-C₆)alkyl group, preferably a hydrogen atom.

In a preferred embodiment, **X₁** represents an O**R_{X}** group, **R_{X}** being advantageously a (C₁-C₆)alkyl group.

In another preferred embodiment, **X₂** and **X₃** each represent a hydrogen atom.

In a yet another preferred embodiment, **X₁** represents an O**R_{X}** group, wherein **R_{X}** is selected from a (C₁-C₆)alkyl, an aryl and an aryl-(C₁-C₆)alkyl group, **R_{X}** being advantageously a (C₁-C₆)alkyl group, notably a (C₁-C₃)alkyl group such as methyl, ethyl, n-propyl, more advantageously methyl; and **X₂** and **X₃** represent, independently of each other, a hydrogen atom or a (C₁-C₆)alkyl group, advantageously a hydrogen atom.

In the above embodiments, the aryl groups are optionally substituted with one or several groups selected from a halogen atom, -O**R₆₆,** -N**R₆₇R₆₈,** -S**R₆₉,** -S(O)**R₇₀,** -SO₂**R₇₁,** -OCO**R₇₂,** - CO₂**R₇₃,** -CON**R₇₄R₇₅,** -CO₂**R₇₆,** nitro (-NO₂) and cyano (-CN).

According to a particular embodiment of the present invention, **Y₁**, **Y₂** and **Y₃** represent, independently of each other, a hydrogen atom, or a group selected from O**R_{Y},** S**R_{Y},** SO₂**R_{Y}** and N**R_{Y}R'_{Z},** wherein at least one of **Y₁, Y₂** and **Y₃** is not a hydrogen atom.

In another particular embodiment of the present invention, **Y₁, Y₂** and **Y₃** represent, independently of each other, a hydrogen atom, a (C₁-C₆)alkyl, an aryl, an aryl-(C₁-C₆)alkyl group, an OH, or an O**R_{Y}** group, wherein at least one of **Y₁, Y₂** and **Y₃** represents an O**R_{Y}** group.

In still another particular embodiment of the present invention, **Y₁, Y₂** and **Y₃** represent, independently of each other, a hydrogen atom or an O**R_{Y}** group, wherein at least one of **Y₁, Y₂** and **Y₃** represents an O**R_{Y}** group.

In the above embodiments, **R_{Y}** is preferably a -(C₁-C₆)alkyl-aryl group, such as benzyl or -CH₃-naphtyl, more preferably benzyl.

In another embodiment, **Y₁** represents a (C₁-C₆)alkyl, an aryl or an aryl-(C₁-C₆)alkyl group, or a group selected from O**R_{Y},** S**R_{Y},** SO₂**R_{Y}** and N**R_{Y}R'_{Z},** wherein **R_{Y}** is selected from a (C₁-C₆)alkyl, an aryl and an aryl-(C₁-C₆)alkyl group, **R_{Y}** being preferably a -(C₁-C₆)alkyl-aryl group, such as benzyl or -CH₃-naphtyl, more preferably benzyl; and **Y₂** and **Y₃** represent, independently of each other, a hydrogen atom or a (C₁-C₆)alkyl group, preferably a hydrogen atom.

In still another embodiment, **Y₁** represents a group selected from O**R_{Y},** S**R_{Y},** SO₂**R_{Y}** and N**R_{Y}R'_{Z},** wherein **R_{Y}** is selected from a (C₁-C₆)alkyl, an aryl and an aryl-(C₁-C₆)alkyl group, **R_{Y}** being preferably -(C₁-C₆)alkyl-aryl group, such as benzyl or -CH₃-naphtyl, more preferably benzyl, and **Y₂** and **Y₃** represent, independently of each other, a hydrogen atom or a (C₁-C₆)alkyl group, preferably a hydrogen atom.

In a preferred embodiment, **Y₁** represents an O**R_{Y}** group, wherein **R_{Y}** is selected from a (C₁-C₆)alkyl, an aryl and an aryl-(C₁-C₆)alkyl group, **R_{Y}** being advantageously a -(C₁-C₆)alkylaryl group, such as benzyl or -CH₃-naphtyl, more preferably benzyl; and **Y₂** and **Y₃** represent, independently of each other, a hydrogen atom or a (C₁-C₆)alkyl group, advantageously a hydrogen atom.

In the above embodiments, the aryl groups are optionally substituted with one or several groups selected from a halogen atom, -O**R₆₆**, -N**R₆₇R₆₈**, -S**R₆₉**, -S(O)**R₇₀**, -SO₂**R₇₁**, -OCO**R₇₂**, - CO₂**R₇₃,** -CON**R₇₄R₇₅**, -CO₂**R₇₆**, nitro (-NO₂) and cyano (-CN).

According to a particular embodiment of the present invention:
▪ **X₁, X₂** and **X₃** represent, independently of each other, a hydrogen atom, a (C₁-C₆)alkyl, an aryl, an aryl-(C₁-C₆)alkyl or an O**R_{X}** group, wherein at least one of **X₁, X₂** and **X₃** is not a hydrogen atom, and wherein **R_{X}** is selected from a (C₁-C₆)alkyl, an aryl and an aryl-(C₁-C₆)alkyl group; and
▪ **Y₁, Y₂** and **Y₃** represent, independently of each other, a hydrogen atom, a (C₁-C₆)alkyl, an aryl, an aryl-(C₁-C₆)alkyl group, an OH or an O**R_{Y}** group, wherein at least one of **Y₁, Y₂** and **Y₃** represents a (C₁-C₆)alkyl, an aryl, an aryl-(C₁-C₆)alkyl group or an O**R_{y}** group, wherein **R_{Y}** is selected from a (C₁-C₆)alkyl, an aryl and an aryl-(C₁-C₆)alkyl group.

According to another particular embodiment:
▪ **X₁** represents a (C₁-C₆)alkyl, an aryl, an aryl-(C₁-C₆)alkyl or an O**R_{X}** group, wherein **R_{X}** is a (C₁-C₆)alkyl, an aryl or an aryl-(C₁-C₆)alkyl group;
▪ **X₂** and **X₃** represent, independently of each other, a hydrogen atom or a (C₁-C₆)alkyl group; and
▪ **Y₁, Y₂** and **Y₃** represent, independently of each other, a hydrogen atom, a (C₁-C₆)alkyl, an aryl, an aryl-(C₁-C₆)alkyl, an OH or an O**R_{Y}** group, wherein at least one of **Y₁, Y₂** and**Y₃** represents a (C₁-C₆)alkyl, an aryl, an aryl-(C₁-C₆)alkyl or an O**R_{Y}** group, wherein **R_{Y}** is selected from a (C₁-C₆)alkyl, an aryl and an aryl-(C₁-C₆)alkyl group.

According to yet another particular embodiment:
▪ **X₁** represents an O**R_{X}** group, wherein **R_{X}** is selected from a (C₁-C₆)alkyl, an aryl and an aryl-(C₁-C₆)alkyl group, **R_{X}** being advantageously a (C₁-C₆)alkyl group, notably a (C₁-C₃)alkyl group such as methyl, ethyl, n-propyl, more advantageously methyl;
▪ **X₂** and **X₃** represent, independently of each other, a hydrogen atom or a (C₁-C₆)alkyl group, advantageously a hydrogen atom;
▪ **Y₁** represents an O**R_{Y}** group, wherein **R_{Y}** is selected from a (C₁-C₆)alkyl, an aryl and an aryl-(C₁-C₆)alkyl group, **R_{Y}** being advantageously a -(C₁-C₆)alkyl-aryl group, such as benzyl or -CH₃-naphtyl, more preferably benzyl; and
▪ **Y₂** and **Y₃** each represent, independently of each other, a hydrogen atom or a (C₁-C₆)alkyl group, advantageously a hydrogen atom.

According to a preferred embodiment, the inhibitor of a pharmaceutical composition according to the invention is of the following formula **(II.i):** 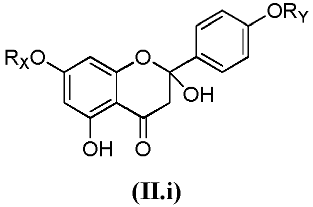 or a pharmaceutically acceptable salt and/or solvate thereof, wherein:
**R_{X}** represents a (C₁-C₆)alkyl group, notably a (C₁-C₃)alkyl group such as methyl, ethyl, n-propyl, more advantageously methyl, and
**R_{Y}** represents an aryl-(C₁-C₆)alkyl group, such as benzyl or -CH₃-naphtyl, more preferably benzyl.

The inhibitor of a pharmaceutical composition according to the invention may notably be selected from the group consisting of compounds **46** to **49,** represented below, and the pharmaceutically acceptable salts and/or solvates thereof.

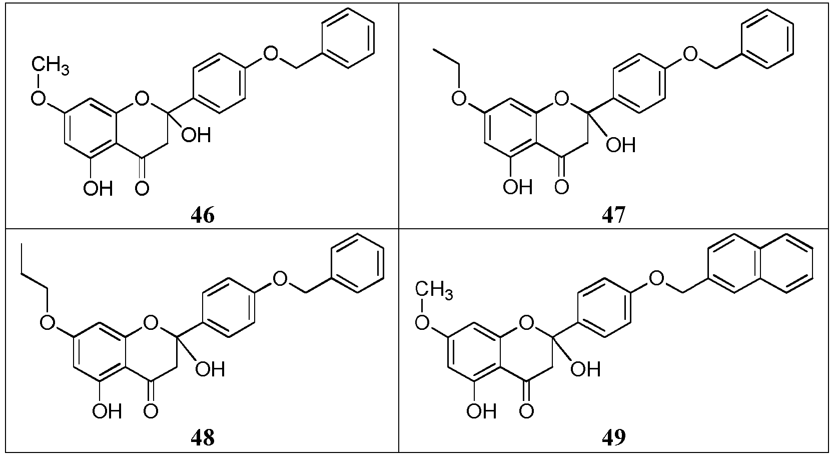

In particular, the inhibitor of a pharmaceutical composition according to the invention is compound **46** or a pharmaceutically acceptable salt and/or solvate thereof.

In particular, the inhibitor of a pharmaceutical composition according to the invention may be selected from the group consisting of compounds **1, 7, 37, 45, 46** and the pharmaceutically acceptable salts and/or solvates thereof.

### Pharmaceutical composition

The pharmaceutical composition according to the invention comprises a combination of N-acetylcysteine (NAC) or a pharmaceutical salt or a derivative thereof as defined above, with at least one inhibitor of regulated necrotic cell death, such as necroptosis and/or ferroptosis, wherein the inhibitor of regulated necrotic cell death is a compound of formula (I) or (II) as defined above.

In a particular embodiment, the inhibitor of regulated necrotic cell death is a ferroptosis inhibitor, preferably a ferroptosis and necroptosis inhibitor. Said inhibitor may be, in particular, sibiriline, nigratine or a derivative thereof, as defined above.

In this particular embodiment, the NAC / inhibitor molar ratio is advantageously comprised between 500:1 and 1:1, preferably between 200:1 and 1:1, notably between 100:1 and 1:1, in particular between 50:1 and 1:1, for example between 20:1 and 1:1.

In a preferred embodiment, the inhibitor of regulated necrotic cell death is sibiriline (compound 7) and the NAC / inhibitor weight ratio is advantageously comprised between 100:1 and 1:1, in particular between 50:1 and 1:1, notably between 30:1 and 1:1, for example 20:1.

In a particular embodiment, the or N-acetylcysteine amide (NACA) / inhibitor molar ratio is advantageously comprised between 200:1 and 1:1, preferably between 100:1 and 1:1, notably between 50:1 and 1:1, in particular between 30:1 and 1:1, for example between 20:1 and 1:1.

In a particular embodiment, the or N-acetylcysteine ethyl ester (NACET / inhibitor molar ratio is advantageously comprised between 500:1 and 1:1, preferably between 200:1 and 1:1, notably between 100:1 and 1:1, in particular between 50:1 and 1:1, for example between 20:1 and 1:1.

The pharmaceutical composition according to the invention may further comprise at least one pharmaceutically acceptable excipient.

The term "pharmaceutically acceptable excipient" is intended to mean, in the framework of the present invention, a substance which is pharmaceutically acceptable, as defined above, formulated alongside the active ingredients of the pharmaceutical composition, included for the purpose of long-term stabilization, bulking up solid formulations that contain potent active ingredients in small amounts, to confer a therapeutic improvement on the active ingredient in the final dosage form (such as facilitating drug absorption, reducing viscosity, or enhancing solubility), or to enhance the taste or the appearance of the pharmaceutical composition. The appropriate excipients can be easily and wisely selected by the skilled person, taking into account notably the dosage form and the route of administration.

The pharmaceutical compositions according to the invention may be formulated notably for oral administration, for topical administration or for injection, wherein said compositions are intended for mammals, including humans.

The pharmaceutical composition can be administered orally in a solid or liquid (solution or suspension) form.

A solid composition can be in the form of tablets, gelatin capsules, powders, granules and the like. When a solid composition is prepared in the form of tablets, the active ingredients are mixed with a pharmaceutical vehicle such as gelatin, starch, lactose, magnesium stearate, talc, gum arabic and the like. The tablets may be coated with sucrose or with other suitable materials, or they may be treated in such a way that they have a prolonged or delayed activity and they continuously release a predetermined amount of active principle. In powders or granules, the active ingredients can be mixed or granulated with dispersing agents, wetting agents or suspending agents and with flavor correctors or sweeteners. In gelatin capsules, the active ingredients can be introduced into soft or hard gelatin capsules in the form of a powder or granules such as mentioned previously or in the form of a liquid composition such as mentioned below.

A liquid composition can contain the active ingredients together with a sweetener, a taste enhancer or a suitable coloring agent in a solvent such as water. The liquid composition can also be obtained by suspending or dissolving a powder or granules, as mentioned above, in a liquid such as water, juice, milk, etc. It can be for example a syrup or an elixir.

For topical administration, the pharmaceutical composition may be in any form allowing an application to the surface of the skin or mucous membranes: cream, gel, ointment, patch, etc.

For administration by injection, aqueous suspensions, isotonic saline solutions or sterile and injectable solutions which contain pharmacologically compatible dispersing agents and/or wetting agents are used.

The inhibitor of the pharmaceutical composition may be used in doses ranging between 0.01 mg and 2,000 mg per day, given in a single dose once per day or administered in several doses throughout the day, for example twice a day in equal doses. The dose administered per day is advantageously between 5 mg and 500 mg, even more advantageously between 10 mg and 200 mg. The effective dose of the inhibitor can be determined by one skilled in the art by routine tests including assessment of the effect of administration of the inhibitor on the disorders which are sought to be prevented and/or treated by said administration. For example, such tests can be implemented by analyzing both quantitative and qualitative effect of the administration of different amounts of the inhibitor on a set of markers (biological and/or clinical) characteristics of said disorder, in particular from a biological sample of a person. Besides, as is well-known to the skilled person, the suitable dose and the associated dosing regimen for treating a given disease in a given patient will depend on several other factors, such as the stage of the disease as well as the physical and medical condition of the patient.

N-acetylcysteine (NAC), N-acetylcysteine amide or N-acetylcysteine ethyl ester or N-acetylcysteine methyl ester may be used in doses ranging between 50 mg and 5,000 mg per day, given in a single dose once per day or administered in several doses throughout the day, for example twice or three times a day in equal doses. The dose administered per day is advantageously between 100 mg and 2000 mg, even more advantageously between 500 mg and 1500 mg.

### Applications

The present invention is also directed to a pharmaceutical composition as defined above, for preventing and/or treating a disorder associated with regulated necrotic cell death, such as necroptosis and/or ferroptosis.

In a particular embodiment, said disorder is associated with ferroptosis, notably with both ferroptosis and necroptosis.

The disorder associated with ferroptosis may be myocardial ischemia-reperfusion injury, notably occurring after artery ligation or myocardial necrosis in myocardial infarction; cardiomyopathy, notably doxorubicin-induced cardiomyopathy; strokes, notably ischemic stroke or hemorrhagic stroke; cardiovascular disease, such as aortic dissection; traumatic brain injury; contusion spinal cord injury; neurodegenerative disorders, in particular chronic neurodegenerative disorders, more particularly Alzheimer's disease, Huntington's disease, Parkinson's disease, amyotrophic lateral sclerosis (Charcot's disease), multiple sclerosis, Friedreich's ataxia and dementia; vision loss, in particular due to retinal detachment or cataract; retinal disorders, notably Stargardt disease or age-related macular degeneration (AMD), in particular dry AMD; chronic liver diseases, notably non-alcoholic steatohepatitis (NASH), chronic infections such as hepatitis, and alcoholic cirrhosis; acute liver injury and acute liver failure, notably resulting from a drug-induced liver injury (DILI), such as acetaminophen (APAP)-induced liver injury, from an ischemia-reperfusion injury induced by a septic or hemorrhagic shock, from fulminant viral hepatitis, from auto-immune origin or from alcohol intake; skin inflammatory diseases, such as psoriasis; toxic epidermal necrolysis (Lyell syndrome); acute kidney injury (AKI) or acute renal failure, such as oxalate-, folic acid (FA)-and cisplatin-induced AKI, renal ischemia-reperfusion injury and acute tubular necrosis; chronic obstructive pulmonary disease (COPD); bronchial asthma; lung injury caused by a bacterial infection, notably by *Pseudomonas aeruginosa* or *Mycobacterium tuberculosis;* pulmonary fibrosis, such as radiation induced-lung fibrosis (RILF) and paraquat-induced pulmonary damage; necrotizing enterocolitis; inflammatory bowel diseases, such as Crohn's disease and ulcerative colitis; haemochromatosis; β-thalassemia; hemolytic disorders; cytokinic storm during a viral infection; radiation-induced necrosis; rheumatoid arthritis; type I diabetes; insulin resistance related to obesity; epilepsy, including mitochondrial disease-related epilepsy and intractable epilepsy; and pathologies related to stress-induced premature tissue senescence, such as atherosclerosis, hypertension and type II diabetes.

Preferably, the disorder associated with ferroptosis is selected from the group consisting of myocardial ischemia-reperfusion injury, notably occurring after artery ligation or myocardial necrosis in myocardial infarction; strokes, notably ischemic stroke or hemorrhagic stroke; traumatic brain injury; neurodegenerative disorders, in particular chronic neurodegenerative disorders, more particularly Alzheimer's disease, Huntington's disease, Parkinson's disease, amyotrophic lateral sclerosis (Charcot's disease) and multiple sclerosis; vision loss, in particular due to retinal detachment or cataract; retinal disorders, notably Stargardt disease or age-related macular degeneration (AMD), in particular dry AMD; chronic liver diseases, notably non-alcoholic steatohepatitis (NASH); acute liver injury and acute liver failure, notably resulting from a drug-induced liver injury (DILI), such as acetaminophen (APAP)-induced liver injury, or from an ischemia-reperfusion injury induced by a septic or hemorrhagic shock; and acute kidney injury (AKI) or acute renal failure, such as folic acid (FA)-induced AKI, cisplatin-induced AKI, renal ischemia-reperfusion injury and acute tubular necrosis.

In particular, the disorder associated with ferroptosis is selected from the group consisting of neurodegenerative disorders, in particular chronic neurodegenerative disorders, more particularly Alzheimer's disease, Huntington's disease, Parkinson's disease, amyotrophic lateral sclerosis (Charcot's disease) and multiple sclerosis; vision loss, in particular due to retinal detachment or cataract; retinal disorders, notably Stargardt disease or age-related macular degeneration (AMD), in particular dry AMD; acute liver injury and acute liver failure, notably resulting from a drug-induced liver injury (DILI), such as acetaminophen (APAP)-induced liver injury, or from an ischemia-reperfusion injury induced by a septic or hemorrhagic shock; and acute kidney injury (AKI) or acute renal failure, such as folic acid (FA)-induced AKI and cisplatin-induced AKI.

In particular, disorder associated with regulated necrotic cell death is a disorder associated with ferroptosis and necroptosis and can be selected from the group consisting of: brain diseases or disorders including neurodegenerative diseases or disorders (notably Alzheimer's disease, Huntington's disease, Parkinson's disease, amyotrophic lateral sclerosis (Charcot's disease), dementia, Friedreich's ataxia and multiple sclerosis), stroke (notably ischemic stroke and hemorrhagic stroke), traumatic brain injury, epilepsy; eye diseases or disorders including retinopathy, degenerative eye diseases or disorders such as retinal degenerative diseases or disorders (notably Stargardt disease and age-related macular degeneration (AMD)); infectious diseases including diseases caused by virus or bacteria (including lung injury caused by a bacterial infection, cytokinic storm during a viral infection); autoimmune diseases including psoriasis and rheumatoid arthritis; inflammatory diseases including chronic liver diseases (notably non-alcoholic steatohepatitis (NASH)), toxic epidermal necrolysis (Lyell's syndrome), bronchial asthma, pulmonary fibrosis, necrotizing enterocolitis, inflammatory bowel diseases (such as Crohn's disease), type I and II diabetes, haemochromatosis, atherosclerosis, insulin resistance related to obesity; pathologies related to stress-induced premature tissue senescence including age-related disorders such as atherosclerosis, hypertension and type II diabetes; liver injury including acute liver failure (notably resulting from a drug-induced liver injury (DILI), such as acetaminophen (APAP)-induced liver injury, or from an ischemia-reperfusion injury induced by a septic or hemorrhagic shock) and chronic liver diseases; hypertension; haemochromatosis; hemolytic disorders; ischemic disorders affecting the heart, brain, or kidneys; kidney injury including acute kidney injury (AKI, also known as acute renal failure (ARF)) such as folic acid (FA)-induced AKI, cisplatin-induced AKI, renal ischemia-reperfusion injury, acute tubular necrosis, liver fibrosis; heart injury including myocardial infarction, myocardial ischemia-reperfusion injury, notably occurring after artery ligation or myocardial necrosis in myocardial infarction; aortic aneurysm; pancreatitis; radiation-induced necrosis; chronic obstructive pulmonary disease, acute respiratory distress disorder; diseases related to transplantation; cancers including liver cancers (notably hepatocellular carcinoma), eye cancers, gastric cancers, colorectal cancers, pancreatic cancers, brain cancers, lung cancers, adrenocortical carcinomas, kidney cancers (notably clear cell renal cell carcinomas).

More particularly, disorder associated with regulated necrotic cell death is a disorder associated with ferroptosis and necroptosis and can be selected from the group consisting of: brain diseases or disorders including neurodegenerative diseases or disorders, stroke, traumatic brain injury, epilepsy; eye diseases or disorders including retinopathy, degenerative eye diseases or disorders; infectious diseases; autoimmune diseases; inflammatory diseases; pathologies related to stress-induced premature tissue senescence; liver injury including acute liver failure and chronic liver diseases; hypertension; haemochromatosis; hemolytic disorders; ischemic disorders affecting the heart, brain, or kidneys; kidney injury including acute kidney injury, renal ischemia-reperfusion injury, acute tubular necrosis, liver fibrosis; heart injury; aortic aneurysm; pancreatitis; radiation-induced necrosis; chronic obstructive pulmonary disease, acute respiratory distress disorder; diseases related to transplantation; cancers including liver cancers, eye cancers, brain cancers, kidney cancers.

In another embodiment, disorder associated with regulated necrotic cell death is a disorder associated with ferroptosis and necroptosis and can be selected from the group consisting of: brain diseases or disorders including neurodegenerative diseases or disorders, stroke, traumatic brain injury; eye diseases and disorders including retinopathy; liver diseases or disorders including acute liver injury such as acute liver failure; kidney diseases or disorders including acute kidney injury; diseases related to transplantation; cancers including liver cancers (such as hepatocellular carcinoma), eye cancer, kidney cancers (such as clear cell renal cell carcinomas).

The disorder associated with both ferroptosis and necroptosis may be acute liver injury and acute liver failure, acute renal failure or acute kidney injury (AKI), acute tubular necrosis, radiation-induced necrosis, ischemic disorders affecting the heart, brain, or kidneys, rheumatoid arthritis, psoriasis, neurodegenerative diseases such as Alzheimer's disease, Parkinson's diseases, lateral amyotrophic sclerosis (Charcot's disease) and dementia, dry (atrophic) age-related macular degeneration (AMD), haemochromatosis, necrotizing enterocolitis, non-alcoholic steatohepatitis (NASH), Friedreich's ataxia, inflammatory bowel diseases such as Crohn's disease, toxic epidermal necrolysis (Lyell's syndrome), type I diabetes, and diseases related to stress-induced premature tissue senescence, including age-related disorders such as atherosclerosis, hypertension and type II diabetes.

The present invention also relates to a pharmaceutical composition as defined above for use in a method for inhibiting regulated necrotic cell death, in particular for inhibiting ferroptosis, more particularly for inhibiting both ferroptosis and necroptosis, comprising the administration to a person in need thereof of an effective dose of a pharmaceutical composition as defined above. In particular, the present invention relates to a pharmaceutical composition as defined above for use in a method for preventing and/or treating a disorder associated with regulated necrotic cell death, such as necroptosis and/or ferroptosis, comprising the administration to a person in need thereof of an effective dose of a pharmaceutical composition as defined above.

In a particular embodiment, said disorder is associated with ferroptosis, notably with both ferroptosis and necroptosis, and is as defined above.

The present invention also relates to pharmaceutical compositions as defined above for use in a method for inhibiting lysosomal permeabilization and/or lysosome-dependent cell death, in particular in a subject suffering from a neurodegenerative disease, comprising the administration to a person in need thereof of an effective dose of a pharmaceutical composition as defined above.

The present invention also relates to the use of a pharmaceutical composition as defined above for the manufacture of a drug, said drug being notably intended for preventing and/or treating a disorder associated with regulated necrotic cell death, such as necroptosis and/or ferroptosis. In a particular embodiment, said disorder is associated with ferroptosis, notably with both ferroptosis and necroptosis, and is as defined above.

The present invention also relates to the use, i.e. non-therapeutic use, of a pharmaceutical composition as defined above, for the *in vitro* preservation and/or protection of biological materials such as cells, tissues, body fluids and organs.

In the context of the present invention, *"in vitro"* means outside of the organism from which the biological material derives.

As used herein, the expression "preservation and/or protection of biological materials" refers to an improved survival of said biological material, allowing its conservation over time. As is clear from the present description, this improved survival is obtained by preventing ferroptosis-induced cell death in said biological materials.

Hence, the present invention also relates to the *in vitro* use of a pharmaceutical composition as defined above as an agent for inhibiting regulated necrotic cell death, such as necroptosis and/or ferroptosis, in a biological material.

The present invention is also directed to a method for inhibiting regulated necrotic cell death, such as necroptosis and/or ferroptosis in a biological material *in vitro,* which comprises exposing said biological material to a pharmaceutical composition as defined above.

In the above aspects of the present invention, the biological material is preferably a cell sample or a tissue sample.

The active ingredients of the pharmaceutical composition according to the invention, namely N-acetylcysteine or a pharmaceutical salt or a derivative thereof and the inhibitor of regulated necrotic cell death as defined above, which is in particular a ferroptosis inhibitor, preferably a ferroptosis and necroptosis inhibitor, may be administered simultaneously, separately or sequentially to a person in need thereof, in particular for inhibiting regulated necrotic cell death, such as necroptosis and/or ferroptosis, more particularly for preventing and/or treating a disorder associated with regulated necrotic cell death, such as necroptosis and/or ferroptosis.

Therefore, the present invention also relates to a pharmaceutical composition comprising:
- N-acetylcysteine or a pharmaceutical salt or a derivative thereof as defined in claim 1, and
- at least one inhibitor of regulated necrotic cell death, in particular at least one ferroptosis inhibitor, preferably at least one ferroptosis and necroptosis inhibitor,
as a combination product for simultaneous, separate or sequential administration, in particular for inhibiting regulated necrotic cell death, such as necroptosis and/or ferroptosis, more particularly for preventing and/or treating a disorder associated with regulated necrotic cell death, such as necroptosis and/or ferroptosis.

The present invention also relates to a pharmaceutical composition as defined above for use as a therapeutically active ingredient in a combination or in an add-on therapeutic regimen in a person in need thereof, in particular for inhibiting regulated necrotic cell death, such as necroptosis and/or ferroptosis, more particularly for preventing and/or treating a disorder associated with regulated necrotic cell death, such as necroptosis and/or ferroptosis. Also provided is a pharmaceutical composition as defined above for use as a therapeutically active ingredient in a combination or in an add-on therapeutic regimen in a patient in need thereof.

In some embodiments, the pharmaceutical composition of the present invention is administered simultaneously, separately or sequentially to a person in need thereof with a third active ingredient.

The pharmaceutical composition as defined above may be provided in a combination product, comprising additional products, in particular a third active ingredient, particularly intended for simultaneous, separate or sequential administration.

The third active ingredient is typically relevant for the disorder to be prevented and/or treated.

The present invention also relates to a kit comprising a pharmaceutical composition as defined above and a delivery device (a device allowing administration of said composition), in particular suitable for parenteral, enteral administration or local administration. Examples of delivery devices include but are not limited to autoinjectors, in particular multichamber syringes, transdermal patchs, pre-filled syringe or a needle free device.

### BRIEF SUMMARY OF THE FIGURES

Figure 1 represents the maximal viability of ARPE19 cells treated with sodium iodate in the presence of NAC, compound **45** or in co-treatment.
Figure 2 represents the maximal viability of ARPE19 cells treated with sodium iodate in the presence of NAC, compound **46** or in co-treatment.
Figure 3 represents the maximal viability of SH-SY5Y cells treated with erastin in the presence of NAC, compound **46** or in co-treatment.
Figure 4 represents the maximal viability of SH-SY5Y cells treated with erastin in the presence of NAC, compound **1** or in co-treatment.
Figure 5 represents the maximal viability of HT-22 cells treated with erastin in the presence of NAC, compound **7** or in co-treatment.
Figure 6 represents the maximal viability of HT-22 cells treated with erastin in the presence of NAC, compound **37** or in co-treatment.
Figure 7 represents the maximal viability of HT-22 cells treated with erastin in the presence of NAC, compound **1** or in co-treatment.
Figure 8 represents the alanine transferase (ALT) concentrations (UI/L) quantified in mouse plasma for 8 groups of mice after APAP intoxication, treated with NAC, increasing doses of compound 7 or in co-treatment.
Figures **9a, 9b** and **9c** show the synergic effect of co-treatment of NAC and compound 7 on *in vivo* mice model of APAP intoxication; ALT values are reported as percent of reduction of maximal ALT obtained in plasma of mice treated with 400 mg/kg APAP only; control mice (Ctrl) were mice without APAP injection.
Figure 10 represents the maximal viability of LLC-PK1 cells treated with RSL3 in the presence of NAC, compound **7** or in co-treatment.
Figure 11 represents the maximal viability of LLC-PK1 cells treated with RSL3 in the presence of NAC, compound **1** or in co-treatment.
Figure 12 represents the maximal viability of LLC-PK1 cells treated with RSL3 in the presence of NAC, compound **46** or in co-treatment.
Figure 13 represents the maximal viability of ARPE19 cells treated with sodium iodate in the presence of N-acetylcysteine amide (NACA), compound **46** or in co-treatment.
Figure 14 represents the maximal viability of SHSY5Y cells treated with erastin in the presence of N-acetylcysteine ethyl ester, compound **7** or in co-treatment.
Figure 15 represents the maximal viability of SHSY5Y cells treated with erastin in the presence of N-acetylcysteine ethyl ester, compound **1** or in co-treatment.
Figure 16 represents the maximal viability of SHSY5Y cells treated with erastin in the presence of N-acetylcysteine ethyl ester, compound **46** or in co-treatment.

### EXAMPLES

The following abbreviations, commonly used in this field of art, are used in the following examples:
- ALT :: alanine aminotransferase
- AMD :: age-related macular degeneration
- APAP :: Acetaminophen
- CI :: calculation index
- DMEM :: Dulbecco's Modified Eagle Medium
- DMSO :: Dimethylsulfoxyde
- H&E :: haematoxylin and eosin
- i.p :: intraperitoneal
- MTS :: 3-[4,5- dimethylthiazol-2-yl]-5-[3-carboxymethoxy-phenyl]-2-[4-sulfophenyl]-2H-tetrazolium
- n :: number of replicates in an experiment
- NAC :: N-acetylcysteine
- PBS :: Phosphate buffered saline
- SD :: Standard deviation
- SEM :: Standard error of mean

### I. In vitro cellular models of diseases

The synergistic efficacy of the combination of NAC and regulated necrosis inhibitors was evaluated *in vitro* in cellular models of pathologies. Regulated necrosis is involved in several pathologies or dysfunctions of the organism and notably in liver damage linked to drug toxicity. Regulated necrosis is also involved in the pathophysiology of degenerative diseases, such as retinal degeneration, or neurodegenerative diseases such as Parkinson's disease, or in neurological disorders associated with excitotoxicity such as trauma or stroke.

### 1.1. Materials and methods

### - Cell culture

The SH-SY5Y neuronal cells (Human Neuroblastoma Cell line) and HT-22 cells (Mouse Hippocampal Neuronal Cell line) were maintained in standard DMEM with GlutaMAX medium (GIBCO), supplemented with 10% fetal bovine serum (GIBCO), at 37 °C in presence of 5% CO2.

The ARPE-19 cells (Human Retinal pigment epithelial cell line) were cultured in standard DMEM/F12 medium (GIBCO), supplemented with 10% fetal bovine serum (GIBCO), at 37 °C in presence of 5% CO2.

The LLC-PK1 cells (Pig Kidney Epithelial cells) were cultured in DMEM/F12(GIBCO), supplemented with 10% fetal bovine serum (GIBCO), at 37°C in presence of 5% CO2.

### - Cell viability assays

SH-SY5Y, LLC-PK1, ARPE-19 and HT22 cells were seeded in 96-well plates at a density of 10 000 or 5000 cells per well respectively, following overnight incubation. Cells were treated with 10 µM (SH-SY5Y cells) or 0.5 µM (HT22 cells) of erastin or 2µM of RSL3 (LLCPK1) or 10 mM of sodium iodate (ARPE-19 cells) for 24 h.

Erastin and RSL3 was purchased from Selleck Chemical and sodium iodate from Sigma Aldrich. Cell viability was assessed by MTS assay (CellTiter 96^{®} AQueous Non-Radioactive Cell Proliferation Assay; Promega, Fitchburg, WI, USA) according to the manufacturer's instructions. This assay is based on the reduction of the 3-(4,5-dimethylthiazol-2-yl)-5-(3-carboxymethoxyphenyl)-2-(4-sulfophenyl)-2H-tetrazolium (MTS) by viable cells to form a colored formazan product. After treatment, cells were incubated 3 h at 37 °C, 5% CO₂ with MTS. The absorbance was measured using a microplate reader at 490 and 630 nm and the percentage of viability was calculated by dividing the absorbance of testing compound by the absorbance of DMSO treated cells (control).

N-acetylcysteine amide (NACA) was purchased from MedChemExpress (ref: HY-110256). N-acetylcysteine ethyl ester was purchased from MedChemExpress (ref: HY-134495).

### - Analysis of synergistic effects of molecules

Cells (HT-22, SHSY-5Y, LLC-PK1 and ARPE 19) were seeded in a 96-well plate in their respective medium overnight. The next day they were treated with NAC or a derivative thereof including NACA or N-acetylcysteine ethyl ester (compound A), with inhibitor compound (compound B) or with a combination of the two compounds. The analysis of the effects of the combination of the association of compound A (NAC or a derivative thereof including NACA and N-acetylcysteine ethyl ester) with another compound B is based on the independence model of Bliss. In this approach, the effect of compound alone (EA or EB) or in combination is expressed as a probability (0≤E≤1). The combined effect of the compounds is defined by: $EAB = EA + EB \left(1-EA\right)$
wherein EA and EB represent the effects of compound A and compound B respectively, and EAB the combined effect of the combination of A and B (expected effect on histogram). Calculation index CI is calculated as follows: CI = (EA + EB - EA*EB) / EAB
wherein CI is synergistic, antagonistic or additive when CI is under, above or equal to 1 respectively [Duarte *et al., Current Research in Pharmacology and Drug Discovery,* 2022].

### 1.2. Model of eye disease

Age-related macular degeneration, or AMD, is characterized by vision loss caused by degeneration of the central cells of the retina, called the macula. Oxidative stress has been shown to play an important role in retinal cell loss through the initiation of non-apoptotic cell death, including ferroptosis [Totsuka et al., Exp. Eye Res., 2019, 181-316-324]. One model used to study retinal cell death is that of human ARPE-19 cells, a retinal pigment epithelial cell line, in the presence of sodium iodate (NaIO₃, a potent oxidizing agent) [Hanus et al. Cell Death Discov. 2016, 2, 16054] [Chan et al., J. Biomed. Sci., 2019, 26:40].

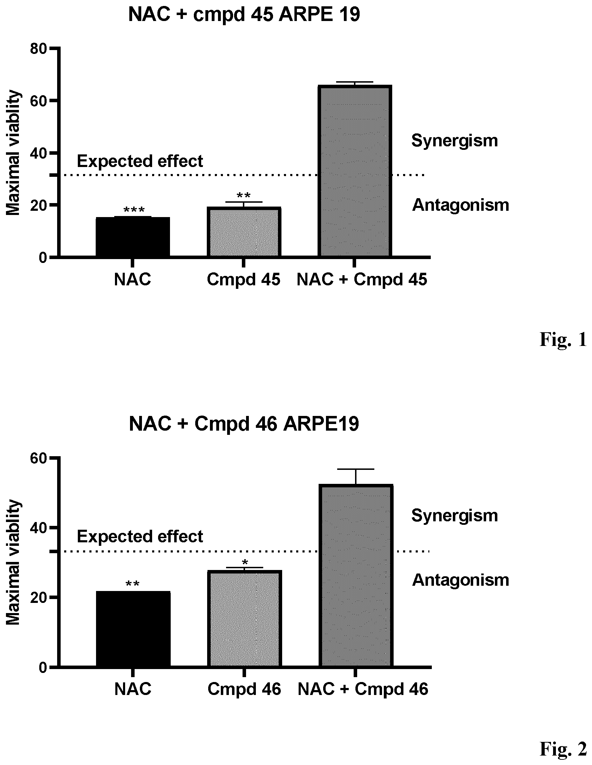

Compounds **45** (Fig. 1) and **46** (Fig. 2) were tested in this assay alone and in combination with NAC. Figures 1 (10 µM cmpd 45; 100 µM NAC) and 2 (5 µM cmpd 46; 50 µM NAC) showed a significant effect of the tested molecules alone and a synergistic effect when combined with NAC (n=2, mean ± SD, * *P <* 0.05, ** *P <* 0.01, *** *P <* 0.001).

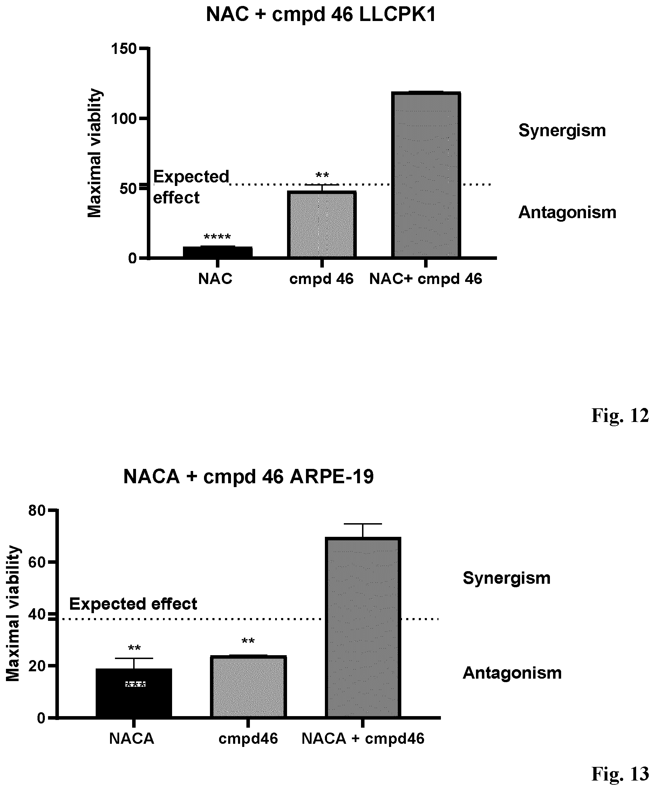

Compound **46** (Fig. 13) was tested in this assay alone and in combination with NACA (N-acetylcysteine amide). Figures 13 (10 µM cmpd 46; 125 µM NACA) showed a significant effect of the tested molecule Compound **46** alone and a synergistic effect when combined with NACA at the tested doses (n=2, mean ± SD, * *P* < 0.05, ** *P* < 0.01, *** *P* < 0.001 and **** *P <* 0.0001).

### 1.3. Neurotoxicity and excitotoxicity models

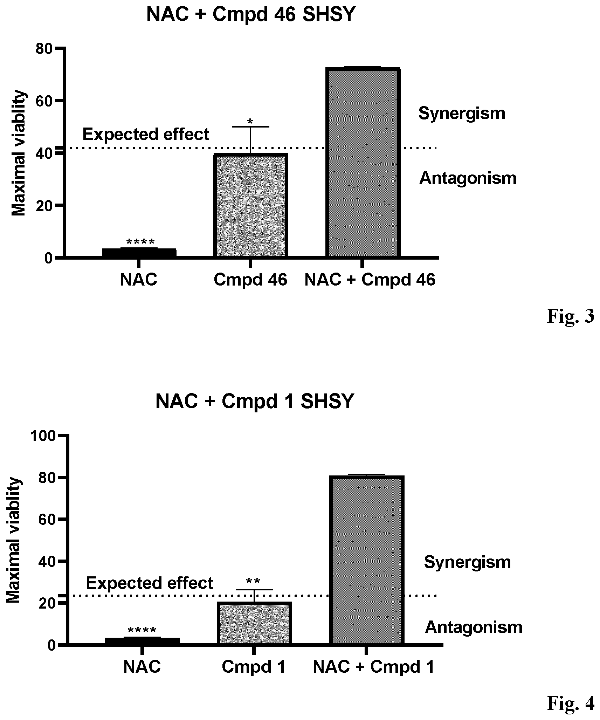

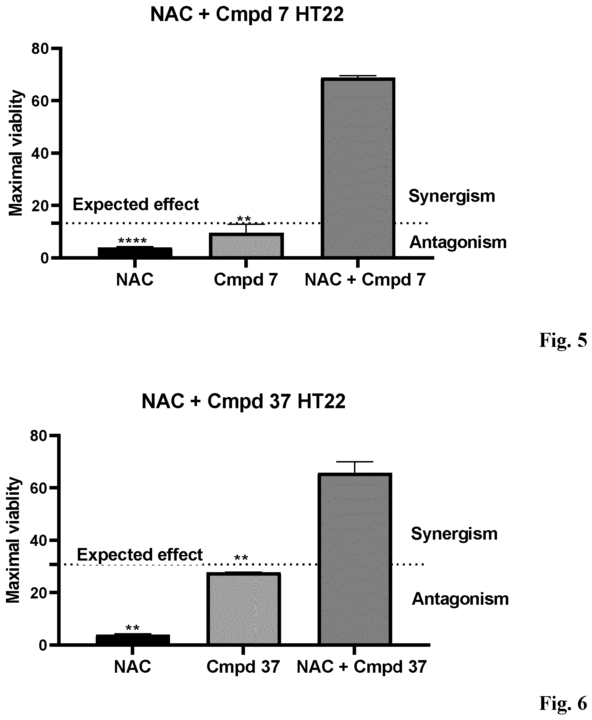

The biological activity of interest was demonstrated in two neuronal cell lines, (i) SH-SY5Y, a human neuroblastoma cell line (Fig. 3-4) and (ii) HT22, a mouse hippocampal cell line (Fig. 5-7). Cell death by ferroptosis was induced by erastin [Dixon et al., Cell, 2012, 149(5), 1060-1072] in both cell lines. Erastin is a well described ferroptosis inducer and also a molecular tool to study neuronal pathologies [Lewerenz et al., Front. Neurosci., 2018, 12: 214].

The results obtained, summarized in Figures 3 to 7, clearly showed the efficacy of the tested compounds on these neurotoxicity models and a synergistic efficacy when combined with NAC. Compounds **46** (Fig. 3) and **1** (Fig. 4) were added to SH-SY5Y cells in combination or not with NAC in the presence of a ferroptosis inducer (erastin). Figures 3 (25 µM cmpd 46; 100 µM NAC) and 4 (1 µM cmpd 1; 100 µM NAC) showed a significant effect of the tested molecules alone and a synergistic effect when combined with NAC at the tested doses (n=2, mean ± SD, * *P* < 0.05, ** *P* < 0.01, *** *P* < 0.001 and **** *P* < 0.0001). HT22 cells were treated with compound **7, 37** or **1** (Fig. **5, 6** and **7** respectively) in combination or not with NAC and in the presence of a ferroptosis inducer (erastin). Figures 5 (25 µM cmpd 7; 100 µM NAC), 6 (50 µM cmpd 37; 100 µM NAC) and 7 (5 µM cmpd 1; 100 µM NAC) showed a significant effect of the tested molecules alone and a synergistic effect when combined with NAC at the tested doses (n=2, mean ± SD, * *P* < 0.05, ** *P* < 0.01, *** *P* < 0.001 and **** *P* < 0.0001).

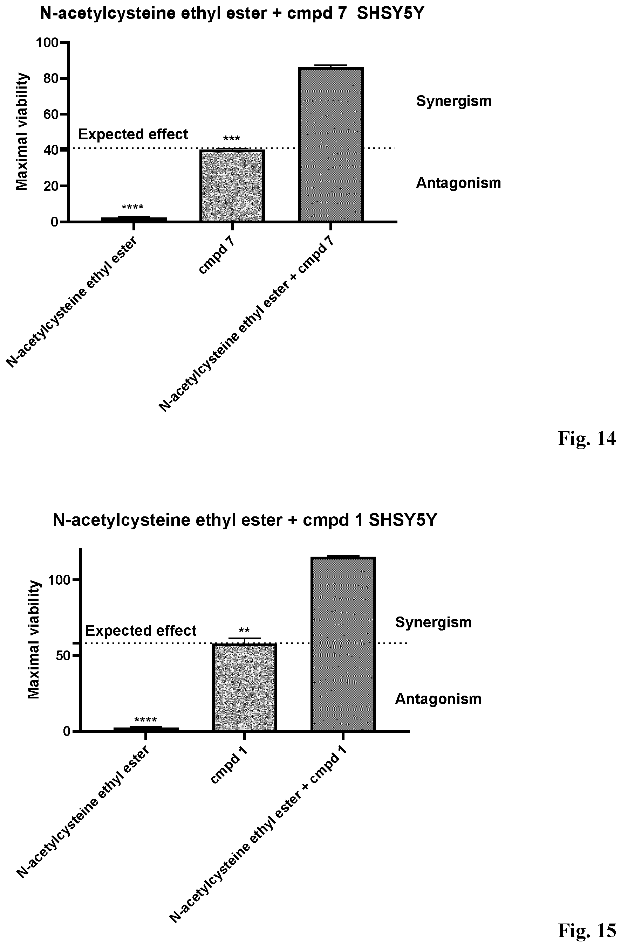

The biological activity of interest was also demonstrated with NAC derivatives in neuronal cell line SH-SY5Y. The results obtained, summarized in Figures **14** to **16****,** clearly showed the efficacy of the tested compounds on this neurotoxicity model and a synergistic efficacy when combined with N-acetylcysteine ethyl ester.

Compound **7** (Fig. **14****)** was added to SH-SY5Y cells in combination or not with N-acetylcysteine ethyl ester in the presence of a ferroptosis inducer (erastin). Figure **14** (10 µM Compound **7** ; 100 µM N-acetylcysteine ethyl ester) showed a significant effect of the tested molecules alone and a synergistic effect when combined with NACET (n=2, mean ± SD, * *P* < 0.05, ** *P* < 0.01, *** *P* < 0.001).

Compound **1** (Fig. **15****)** was added to SH-SY5Y cells in combination or not with N-acetylcysteine ethyl ester in the presence of a ferroptosis inducer (erastin). Figure **15** (2.5 µM Compound 1 ; 100 µM N-acetylcysteine ethyl ester) showed a significant effect of the tested molecules alone and a synergistic effect when combined with N-acetylcysteine ethyl ester (n=2, mean ± SD, * *P* < 0.05, ** *P* < 0.01, *** *P <* 0.001).

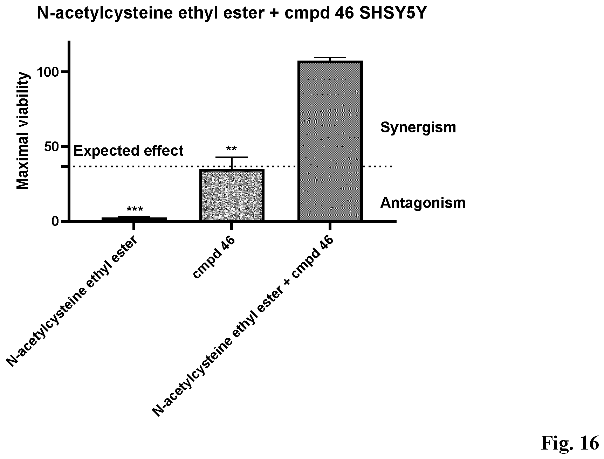

Compound **46** (Fig. **16****)** was added to SH-SY5Y cells in combination or not with N-acetylcysteine ethyl ester in the presence of a ferroptosis inducer (erastin). Figure **16** (25 µM Compound **46 ;** 100 µM N-acetylcysteine ethyl ester) showed a significant effect of the tested molecules alone and a synergistic effect when combined with N-acetylcysteine ethyl ester (n=2, mean ± SD, * *P <* 0.05, ** *P <* 0.01, *** *P <* 0.001).

### 1.4. Pig Kidney Epithelial cells models

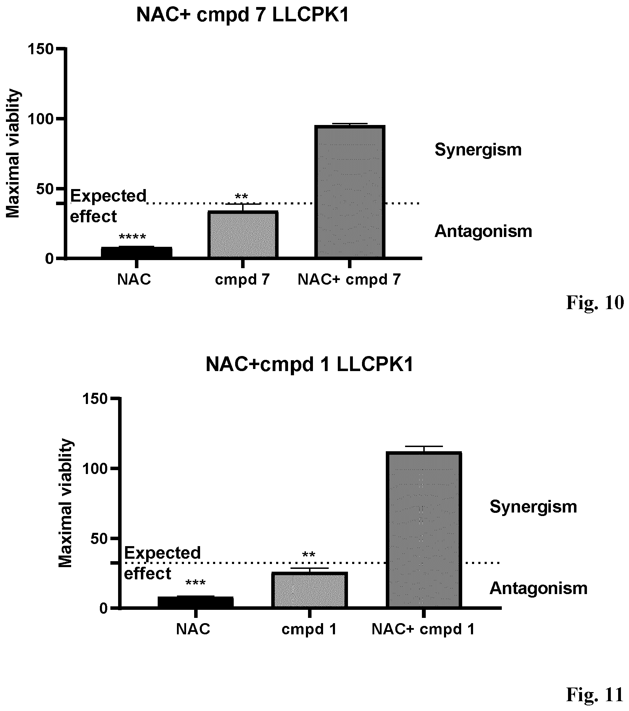

Compound **7** (Fig. **10****),** Compound **1** (Fig. **11****)** and Compound **46 (****Fig.12****)** were tested in this assay alone and in combination with NAC. Figures **10** (2.5 µM Compound **7;** 500 µM NAC), figures **11** (1 µM Compound **1;** 500 µM NAC) and **12** (25 µM Compound **46;** 500 µM NAC) showed a significant effect of the tested molecules alone and a synergistic effect when combined with NAC (n=2, mean ± SD, * *P* < 0.05, ** *P <* 0.01, *** *P <* 0.001).

### II. In vivo model of pathologies

### II.1. Materials and methods

All animal experiments were conducted in compliance with French laws and the institution's guidelines for animal welfare. This project was approved by the "Comité Régional d'Ethique et d'Expérimentation Animal" (CREAA), under the authorization APAFIS #32246-2021061616397414 v7, given by the "Ministère de l'Enseignement Supérieur de la Recherche et de l'Innovation".

Nine-week-old C57B1/6J male mice were purchased from Janvier Labs (Le Genest St Isle, France). After one week of acclimatization in the animal house, mice were fasted overnight before experimentation. Acetaminophen (APAP) was purchased from Sigma-Aldrich (A70-85-100g, batch #SCLF8273) and diluted at 20 mg/mL in PBS (preheated at 45°C). Acetaminophen intoxication was induced in mice by intraperitoneal (*i.p.*) injection of APAP (400mg/kg). One hour after APAP injection, mice were treated with inhibitors by intraperitoneal injection. N-acetylcysteine (NAC, A9165-25g, Sigma-Aldrich, batch #SLCJ1628), the reference treatment for paracetamol intoxication, was diluted at 40 mg/mL in PBS Tween80 5% and injected *via* intraperitoneal route at a dose of 200 mg/kg. Compound 7 was diluted at 1 mg/ml or 2 mg/ml in PBS Tween80 5% and injected by intraperitoneal injection with increasing concentrations (2.5, 5 or 10 mg/kg) or in combination with NAC (200 mg/kg). There were two groups of control mice: the first one was injected via *i.p.* route with PBS Tween80 5% (Tween80 5%) and the second group, treated with APAP, was administered one hour after APAP with PBS Tween80 5% (APAP-Tween80 5%) via *i.p..* Mice were slaughtered eight hours after APAP injection. Blood and liver were collected.

### - Biochemical Parameters

Serum alanine aminotransferase (ALT) plasma levels were measured according to the International Federation of Clinical Chemistry and Laboratory Medicine primary reference procedures using an Olympus AU2700 Autoanalyser (Olympus Optical).

### - Histological Analysis

Liver fragments were fixed in 4% paraformaldehyde and embedded in paraffin. Sections of 4 µm were used for haematoxylin and eosin (H&E) staining. All paraffin-embedded liver sections were scanned with a digital slide scanner (Nanozoomer 2.0-RS, Hamamatsu Photonics, Massy, France) and files were analysed with the NDP viewer 2.5 software (Hamamatsu).

### - Statistical Analysis

Results were expressed as means ± SEM. Mean differences between two experimental groups were assessed using the non-parametric Mann-Whitney U-test. All statistical analyses were achieved with the GraphPad Prism5 software. Calculated *P* values are integrated on histograms and graphs. Significance is shown as follows: $ *P* < 0.05, $$ *P* < 0.01, $$$ *P* < 0.001 and $$$ *P* < 0.0001 (comparison between APAP mice and APAP mice treated with NAC and/or compound 7); * *P* < 0.05, ** *P* < 0.01, *** *P* < 0.001 and **** *P* < 0.0001 (comparison between treated groups).

### II.2. Results

Nine groups of mice were studied:
- Tween80 5%, n=15
- APAP (400 mg/kg) + Tween 80 5%, n=31
- APAP (400 mg/kg) + NAC (200 mg/kg), n=15
- APAP (400 mg/kg) + compound 7 (2.5 mg/kg), n=23
- APAP (400 mg/kg) + compound 7 (5 mg/kg), n=21
- APAP (400 mg/kg) + compound 7 (10 mg/kg), n=21
- APAP (400 mg/kg) + compound 7 (2.5mg /kg) + (NAC 200 mg/kg), n=9
- APAP (400 mg/kg) + compound 7 (5mg /kg) + NAC (200 mg/kg), n=9
- APAP (400 mg/kg) + compound 7 (10 mg/kg) + NAC (200 mg/kg), n=13

APAP overdose induced liver injury and the release of alanine aminotransferase (ALT) in mouse plasma. Mice treated with APAP (400 mg/kg) for 8 hours showed significant increase of plasma ALT level (~3000 IU/L) in comparison with control mice only injected with Tween80 5% (~100 IU/L).

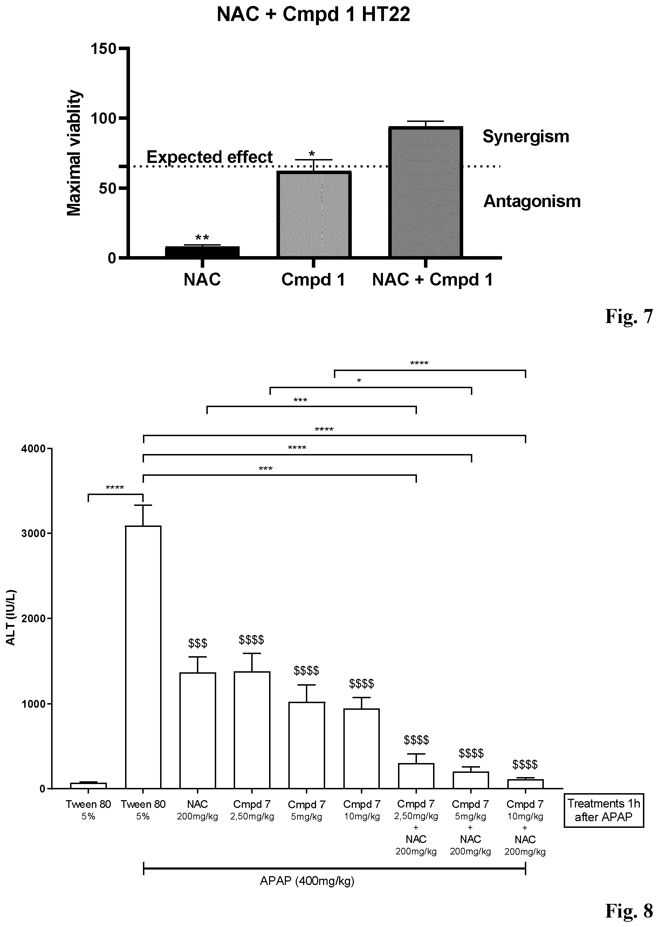

N-acetylcysteine (NAC) is an effective antidote to limit liver injury in patients with APAP intoxication. Mice treated with NAC (200 mg/kg) one hour after APAP (400 mg/kg) injection were partly protected from APAP toxicity as shown by significant reduction of ALT plasma level (~1300 IU/L) in comparison with mice treated with APAP (~3000 IU/L). Compound 7 treatment one hour after APAP was also effective to protect mice liver from APAP toxicity by reducing ALT plasma levels in a dose-dependent manner with a maximum effect observed at the dose of compound 7 (10mg/kg) (~1000 IU/L) (Fig. 8).

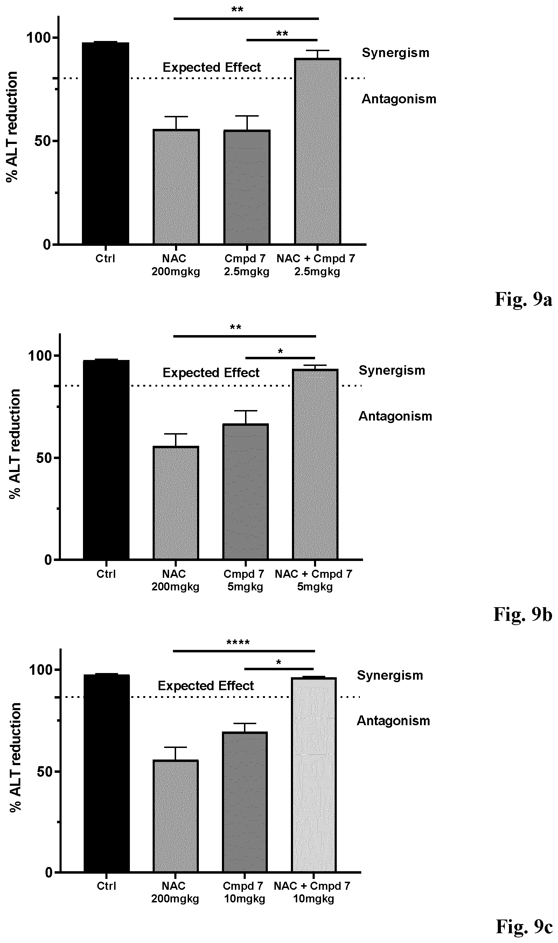

In a remarkable way, the treatment with NAC (200 mg/kg) in combination with increased concentrations of compound 7 (2.5, 5 or 10mg/kg) had a better and synergistic protective effect against APAP toxicity than the treatment with NAC alone or compound 7 alone (Fig. 9a-c). This protective effect was dependent on the dose of compound 7 with an almost complete protection for the combination of NAC (200 mg/kg) with compound 7 (10 mg/kg) since the ALT plasma level was closed similar (~200 IU/L) to the one measured in control mice only treated with Tween80 5% (~100 IU/L) (Fig. 8).

## Claims

1. A pharmaceutical composition comprising a combination of :
N-acetylcysteine and/or a pharmaceutical salt and/or a derivative thereof, the derivative of N-acetylcysteine being N-acetylcysteine amide, and/or N-acetylcysteine ethyl ester and/or N-acetylcysteine methyl ester, and/or salt(s) thereof,
with at least one inhibitor of regulated necrotic cell death, wherein the inhibitor of regulated necrotic cell death is:
**(A)** a compound of the following general formula **(I):** 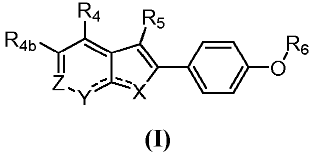
or a pharmaceutically acceptable salt and/or solvate thereof,
wherein:
▪ 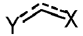 is 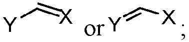
(i) when 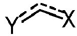 is 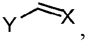 **X** is N, **Y** is N**(R₂)** and **Z** is C(H);
(ii) when 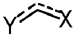 is 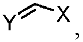
- **X** is N**(R₁),** and
- **Y** is Nor N⁺(O⁻) and **Z** is C**(R₃),** or
**Y** is CH and **Z** is N, or
**Y** and **Z** are CH;
and wherein:
▪ **R₁** and **R₂** represent, independently of each other, a hydrogen atom, CN, NO₂, O**R₇,** S**R₈,** N**R₉R₁₀,** C(O)**R₁₁,** CO₂**R₁₂,** OC(O)**R₁₃,** N**R₁₄**C(O)**R₁₅,** C(O)N**R₁₆R₁₇,** S(O)**R_{S},** SO₂**R_{S}',** a (C₁-C₆)alkyl, a (C₁-C₆)haloalkyl, a -(C₁-C₆)alkyl-[O-(C₁-C₆)alkyl]ₘ-N**R_{N1}R_{N2}** group with m ranging from 1 to 6, an aryl, an aryl-(C₁-C₆)alkyl, a heterocyclyl, or a heterocyclyl-(C₁-C₆)alkyl group, wherein said aryl or heterocyclyl group is optionally substituted by one or more substituents selected from the group consisting of a halogen atom, CN, NO₂, O**R₁₈,** S**R₁₉,** N**R₂₀R₂₁,** C(O)**R₂₂,** CO₂**R₂₃,** OC(O)**R₂₄,** N**R₂₅**C(O)**R₂₆,** C(O)N**R₂₇R₂₈,** a (C₁-C₆)alkyl and a (C₁-C₆)haloalkyl group;
▪ **R₃, R₄, R_{4b}** and **R₅** represent, independently of each other, a hydrogen atom, a halogen atom, CN, O**R₂₉**, S**R₃₀**, N**R₃₁R₃₂**, C(O)**R₃₃**, CO₂**R₃₄**, OC(O)**R₃₅**, N**R₃₆**C(O)**R₃₇**, C(O)N**R₃₈R₃₉**, a (C₁-C₆)alkyl, a (C₁-C₆)haloalkyl group, said alkyl or haloalkyl group being optionally substituted by one or more substituents selected from the group consisting of O**R₄₀**, S**R₄₁** and N**R₄₂R₄₃**, an aryl, a heterocyclyl, an aryl-(C₁-C₆)alkyl or a heterocyclyl-(C₁-C₆)alkyl group, wherein said aryl or heterocyclyl group is optionally substituted by one or more substituents selected from the group consisting of a halogen atom, CN, NO₂, O**R₄₄**, S**R₄₅**, N**R₄₆R₄₇**, C(O)**R₄₈**, CO₂**R₄₉**, OC(O)**R₅₀**, N**R₅₁**C(O)**R₅₂**, C(O)N**R₅₃R₅₄**, a (C₁-C₆)alkyl and a (C₁-C₆)haloalkyl group;
▪ **R₆** represents a hydrogen atom, a (C₁-C₆)alkyl, an aryl-(C₁-C₆)alkyl, a heterocyclyl-(C₁-C₆)alkyl, a -(C₁-C₆)alkyl-[O-(C₁-C₆)alkyl]_{m'}-N**R_{N'1}R_{N'2}** group with m' ranging from 1 to 6, or a (C₁-C₆)alkylcarbonyl group, said (C₁-C₆)alkyl, aryl-(C₁-C₆)alkyl, and (C₁-C₆)alkylcarbonyl group being optionally substituted with one or more substituents selected from the group consisting of OH, SH, NH₂, a (C₁-C₆)alkoxy, a (C₁-C₆)thioalkoxy, a (C₁-C₆)alkylamino and a di((C₁-C₆)alkyl)amino group;
▪ **R_{S}** and **R_{S}**' represent, independently of each other a (C₁-C₆)alkyl, an aryl or an aryl-(C₁-C₆)alkyl group;
▪ **R₇-R₁₀, R₁₂, R₁₄** and **R₁₆-R₁₇** represent, independently of each other, a hydrogen atom, a (C₁-C₆)alkyl, an aryl or an aryl-(C₁-C₆)alkyl group;
▪ **R₁₁, R₁₃** and **R₁₅** represent, independently of each other, a hydrogen atom, a (C₁-C₆)alkyl, an aryl, an aryl-(C₁-C₆)alkyl, a (C₁-C₆)alkoxy, a (C₁-C₆)alkylamino or a di((C₁-C₆)alkyl)amino group;
▪ **R₁₈** to **R₂₈** represent, independently of each other, a hydrogen atom, a (C₁-C₆)alkyl or an aryl group;
▪ **R₂₉** to **R₃₉** represent, independently of each other, a hydrogen atom, a (C₁-C₆)alkyl, heterocyclyl-(C₁-C₆)alkyl, an aryl or an aryl-(C₁-C₆)alkyl group, said aryl group being optionally substituted by one or more substituents selected from the group consisting of a halogen atom, CN, NO₂, O**R₅₅**, S**R₅₆**, N**R₅₇R₅₈**, C(O)**R₅₉**, CO₂**R₆₀**, OC(O)**R₆₁**, N**R₆₂**C(O)**R₆₃**, C(O)N**R₆₄R₆₅**, a (C₁-C₆)alkyl and a (C₁-C₆)haloalkyl group;
▪ **R₄₀** to **R₄₃** represent, independently of each other, a hydrogen atom or a (C₁-C₆)alkyl group;
▪ **R₄₄** to **R₅₄** represent, independently of each other, a hydrogen atom, a (C₁-C₆)alkyl or an aryl group;
▪ **R₅₅** to **R₆₅** represent, independently of each other, a hydrogen atom, a (C₁-C₆)alkyl, aryl-(C₁-C₆)alkyl group or an aryl group; and
**R_{N1}, R_{N'1}, R_{N2}** and **R_{N'2}** represent, independently of each other, a hydrogen atom, a (C₁-C₆)alkyl group, an aryl-(C₁-C₆)alkyl group or an aryl group;
or
**(B)** a compound of the following general formula **(II):** 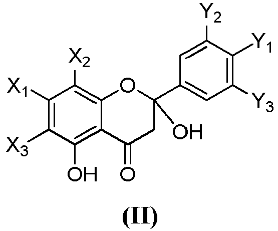
or a pharmaceutically acceptable salt and/or solvate thereof, wherein:
▪ **X₁, X₂** and **X₃** represent, independently of each other, a hydrogen atom, a (C₁-C₆)alkyl, an aryl, an aryl-(C₁-C₆)alkyl or an OH group, or a group selected from O**R_{X},** S**R_{X}**, SO₂**R_{X}** and N**R_{X}R_{Z}**,
wherein at least one of **X₁, X₂** and **X₃** represents a (C₁-C₆)alkyl, an aryl or an aryl-(C₁-C₆)alkyl group, or a group selected from O**R_{X},** S**R_{X}**, SO₂**R_{X}** and N**R_{X}R_{Z}**, wherein
**R_{X}** a (C₁-C₆)alkyl, an aryl or an aryl-(C₁-C₆)alkyl group,
**R_{Z}** is a hydrogen atom or a (C₁-C₆)alkyl group, and
the aryl groups are optionally substituted with one or several groups selected from a halogen atom, -O**R₆₆,** -N**R₆₇R₆₈,** -S**R₆₉,** -S(O)**R₇₀,** -SO₂**R₇₁,** -OCO**R₇₂,** -CO₂**R₇₃,** -CON**R₇₄R₇₅,** - CO₂**R₇₆,** nitro (-NO₂) and cyano (-CN);
▪ **Y₁, Y₂** and **Y₃** represent, independently of each other, a hydrogen atom, a (C₁-C₆)alkyl, an aryl, an aryl-(C₁-C₆)alkyl or an OH group, or a group selected from O**R_{Y}**, S**R_{Y},** SO₂**R_{Y}** and N**R_{Y}R'_{Z},**
wherein at least one of **Y₁, Y₂** and **Y₃** represents a (C₁-C₆)alkyl, an aryl or an aryl-(C₁-C₆)alkyl group, or a group selected from O**R_{Y}**, S**R_{Y},** SO₂**R_{Y}** and N**R_{Y}R'_{Z}**, wherein
**R_{Y}** is a (C₁-C₆)alkyl, an aryl or an aryl-(C₁-C₆)alkyl group,
**R'_{Z}** is a hydrogen atom or a (C₁-C₆)alkyl group, and
the aryl groups are optionally substituted with one or several groups selected from a halogen atom, -O**R₆₆**, -N**R₆₇R₆₈**, -S**R₆₉**, -S(O)**R₇₀**, -SO₂**R₇₁**, -OCO**R₇₂**, -CO₂**R₇₃**, -CON**R₇₄R₇₅,** - CO₂**R₇₆,** nitro (-NO₂) and cyano (-CN);
and
▪ **R₆₆** to **R₇₇** are, independently of one another, a hydrogen atom or a (C₁-C₆)alkyl group.

2. The pharmaceutical composition according to claim 1, wherein the inhibitor of regulated necrotic cell death is a compound of formula **(I),** wherein:
▪ **R₁** and **R₂** represent, independently of each other, a hydrogen atom, CN, NO₂, O**R₇**, S**R₈**, N**R₉R₁₀**, C(O)**R₁₁**, CO₂**R₁₂**, OC(O)**R₁₃**, N**R₁₄**C(O)**R₁₅**, C(O)N**R₁₆R₁₇**, S(O)**R_{S}**, SO₂**R_{S}'**, a (C₁-C₆)alkyl, a (C₁-C₆)haloalkyl, an aryl, a heterocyclyl, an aryl-(C₁-C₆)alkyl or a heterocyclyl-(C₁-C₆)alkyl group, wherein said aryl or heterocyclyl group is optionally substituted by one or more substituents selected from the group consisting of a halogen atom, CN, NO₂, O**R₁₈**, S**R₁₉**, N**R₂₀R₂₁**, C(O)**R₂₂**, CO₂**R₂₃**, OC(O)**R₂₄**, N**R₂₅**C(O)**R₂₆**, C(O)N**R₂₇R₂₈**, a (C₁-C₆)alkyl and a (C₁-C₆)haloalkyl group;
▪ **R₃, R₄, R_{4b}** and **R₅** represent, independently of each other, a hydrogen atom, a halogen atom, CN, O**R₂₉**, S**R₃₀**, N**R₃₁R₃₂**, C(O)**R₃₃**, CO₂**R₃₄**, OC(O)**R₃₅**, N**R₃₆**C(O)**R₃₇**, C(O)N**R₃₈R₃₉**, a (C₁-C₆)alkyl, a (C₁-C₆)haloalkyl group, said alkyl or haloalkyl group being optionally substituted by one or more substituents selected from the group consisting of O**R₄₀**, S**R₄₁** and N**R₄₂R₄₃**, an aryl, a heterocyclyl, an aryl-(C₁-C₆)alkyl or a heterocyclyl-(C₁-C₆)alkyl group, wherein said aryl or heterocyclyl group is optionally substituted by one or more substituents selected from the group consisting of a halogen atom, CN, NO₂, O**R₄₄**, S**R₄₅**, N**R₄₆R₄₇**, C(O)**R₄₈**, CO₂**R₄₉**, OC(O)**R₅₀**, N**R₅₁**C(O)**R₅₂,** C(O)N**R₅₃R₅₄**, a (C₁-C₆)alkyl and a (C₁-C₆)haloalkyl group;
▪ **R₆** represents a hydrogen atom, a (C₁-C₆)alkyl, an aryl-(C₁-C₆)alkyl or a -CH₂-CH₂-O-CH₂-CH₂-NH₂ group, or a (C₁-C₆)alkylcarbonyl group optionally substituted with one or more substituents selected from the group consisting of OH, SH, NH₂, a (C₁-C₆)alkoxy, a (C₁-C₆)thioalkoxy, a (C₁-C₆)alkylamino and a di((C₁-C₆)alkyl)amino group;
▪ **R_{S}** and **R_{S}'** represent, independently of each other a (C₁-C₆)alkyl, an aryl or an aryl-(C₁-C₆)alkyl group;
▪ **R₇-R₁₀, R₁₂, R₁₄** and **R₁₆-R₁₇** represent, independently of each other, a hydrogen atom, a (C₁-C₆)alkyl, an aryl or an aryl-(C₁-C₆)alkyl group;
▪ **R₁₁, R₁₃** and **R₁₅** represent, independently of each other, a hydrogen atom, a (C₁-C₆)alkyl, an aryl, an aryl-(C₁-C₆)alkyl, a (C₁-C₆)alkoxy, a (C₁-C₆)alkylamino or a di((C₁-C₆)alkyl)amino group;
▪ **R₁₈** to **R₂₈** represent, independently of each other, a hydrogen atom, a (C₁-C₆)alkyl or an aryl group;
▪ **R₂₉** to **R₃₉** represent, independently of each other, a hydrogen atom, a (C₁-C₆)alkyl, an aryl or an aryl-(C₁-C₆)alkyl group, said aryl group being optionally substituted by one or more substituents selected from the group consisting of a halogen atom, CN, NO₂, O**R₅₅**, S**R₅₆**, N**R₅₇R₅₈**, C(O)**R₅₉**, CO₂**R₆₀**, OC(O)**R₆₁**, N**R₆₂**C(O)**R₆₃**, C(O)N**R₆₄R₆₅**, a (C₁-C₆)alkyl and a (C₁-C₆)haloalkyl group;
▪ **R₄₀** to **R₄₃** represent, independently of each other, a hydrogen atom or a (C₁-C₆)alkyl group;
▪ **R₄₄** to **R₅₄** represent, independently of each other, a hydrogen atom, a (C₁-C₆)alkyl or an aryl group; and
▪ **R₅₅** to **R₆₅** represent, independently of each other, a hydrogen atom, a (C₁-C₆)alkyl or an aryl group.

3. The pharmaceutical composition according to claim 1, wherein the inhibitor of regulated necrotic cell death is a compound of formula **(I),** wherein:
▪ **R₁** represents a hydrogen atom, CN, NO₂, O**R₇**, S**R₈**, N**R₉R₁₀**, C(O)**R₁₁**, CO₂**R₁₂**, OC(O)**R₁₃**, N**R₁₄**C(O)**R₁₅**, C(O)N**R₁₆R₁₇**, S(O)**R_{S}**, SO₂**R_{S}'**, a (C₁-C₆)alkyl, a (C₁-C₆)haloalkyl, an aryl, a heterocyclyl, an aryl-(C₁-C₆)alkyl or a heterocyclyl-(C₁-C₆)alkyl group, wherein said aryl or heterocyclyl group is optionally substituted by one substituent selected from the group consisting of a halogen atom, CN, NO₂, O**R₁₈**, S**R₁₉**, N**R₂₀R₂₁,** a (C₁-C₆)alkyl and a (C₁-C₆)haloalkyl group, wherein **R₁₈** to **R₂₁** represent, independently of each other, a hydrogen atom or a (C₁-C₆)alkyl group and **R_{S}** and **R_{S}'** represent, independently of each other a (C₁-C₆)alkyl or an aryl group, preferably an aryl group,
preferably, **R₁** represents a hydrogen atom, CN, O**R₇,** C(O)**R₁₁**, CO₂**R₁₂**, OC(O)**R₁₃**, SO₂**R_{S}**', a (C₁-C₆)alkyl, a heterocyclyl or a heterocyclyl-(C₁-C₆)alkyl group, wherein said heterocyclyl group is optionally substituted by NO₂,
more preferably, **R₁** represents a hydrogen atom or a (C₁-C₆)alkyl group, in particular a hydrogen atom or a (C₁-C₃)alkyl group, advantageously a hydrogen atom;
▪ **R₂** represents C(O)**R₁₁**, CO₂**R₁₂**, C(O)N**R₁₆R₁₇**, a (C₁-C₆)alkyl, a (C₁-C₆)haloalkyl, an aryl, a heterocyclyl, an aryl-(C₁-C₆)alkyl or a heterocyclyl-(C₁-C₆)alkyl group, wherein said aryl or heterocyclyl group is optionally substituted by one or more substituents selected from the group consisting of a halogen atom O**R₁₈**, S**R₁₉**, N**R₂₀R₂₁**, a (C₁-C₆)alkyl and a (C₁-C₆)haloalkyl group, wherein **R₁₈** to **R₂₁** represent, independently of each other, a hydrogen atom or a (C₁-C₆)alkyl group,
preferably **R₂** represents CO₂**R₁₂**, C(O)N**R₁₆R₁₇** or an aryl-(C₁-C₆)alkyl group, wherein said aryl group is optionally substituted by one or more substituents selected from the group consisting of a halogen atom, O**R₁₈**, S**R₁₉** and N**R₂₀R₂₁**, notably O**R₁₈**;
▪ **R₃** represents a hydrogen atom, a halogen atom, a (C₁-C₆)alkyl group, CN, O**R₂₉**, S**R₃₀**, N**R₃₁R₃₂**, C(O)**R₃₃**, CO₂**R₃₄**, OC(O)**R₃₅**, N**R₃₆**C(O)**R₃₇**, C(O)N**R₃₈R₃₉**, an aryl, a heterocyclyl, an aryl-(C₁-C₆)alkyl or a heterocyclyl-(C₁-C₆)alkyl group, wherein said aryl or heterocyclyl group is optionally substituted by one or more substituents selected from the group consisting of a halogen atom, O**R₄₄**, S**R₄₅**, N**R₄₆R₄₇**, a (C₁-C₆)alkyl and a (C₁-C₆)haloalkyl group, wherein **R₂₉** to **R₃₇** represent, independently of each other, a hydrogen atom or a (C₁-C₆)alkyl group, and **R₄₄** to **R₄₇** represent, independently of each other, a hydrogen atom or a (C₁-C₆)alkyl group,
preferably **R₃** represents a hydrogen atom, a halogen atom, a (C₁-C₆)alkyl group, CN, O**R₂₉**, S**R₃₀**, N**R₃₁R₃₂**, OC(O)**R₃₅**, N**R₃₆**C(O)**R₃₇**, a heterocyclyl or a heterocyclyl-(C₁-C₆)alkyl group, wherein said heterocyclyl group is optionally substituted by one or more substituents selected from the group consisting of a halogen atom, O**R₄₄**, S**R₄₅**, N**R₄₆R₄₇** and a (C₁-C₆)alkyl a group,
more preferably **R₃** represents a hydrogen atom, a halogen atom, a (C₁-C₆)alkyl group, CN, N**R₃₁R₃₂**, OC(O)**R₃₅**, a heterocyclyl or a heterocyclyl-(C₁-C₆)alkyl group, wherein said heterocyclyl group is optionally substituted by one or more substituents selected from the group consisting of a O**R₄₄**, S**R₄₅** and N**R₄₆R₄₇**, notably O**R₄₄**,
even more preferably **R₃** represents a hydrogen atom, a halogen atom or a (C₁-C₃)alkyl group, advantageously a hydrogen atom or a halogen atom;
▪ **R₄** represents a hydrogen atom, a halogen atom, CN, O**R₂₉**, S**R₃₀**, N**R₃₁R₃₂**, a (C₁-C₆)alkyl, a (C₁-C₆)haloalkyl group, an aryl, a heterocyclyl, an aryl-(C₁-C₆)alkyl or a heterocyclyl-(C₁-C₆)alkyl group, wherein said aryl or heterocyclyl group is optionally substituted by one or more substituents selected from the group consisting of a halogen atom, CN, NO₂, O**R₄₄**, S**R₄₅**, N**R₄₆R₄₇**, C(O)**R₄₈**, CO₂**R₄₉**, OC(O)**R₅₀**, N**R₅₁**C(O)**R₅₂**, C(O)N**R₅₃R₅₄**, a (C₁-C₆)alkyl and a (C₁-C₆)haloalkyl group,
preferably **R₄** represents a hydrogen atom, a halogen atom, O**R₂₉**, S**R₃₀**, N**R₃₁R₃₂**, a (C₁-C₆)alkyl, an aryl or a heterocyclyl group, wherein said aryl or heterocyclyl group is optionally substituted by one or more substituents selected from the group consisting of a halogen atom, O**R₄₄**, S**R₄₅**, N**R₄₆R₄₇**, C(O)**R₄₈**, CO₂**R₄₉**, C(O)N**R₅₃R₅₄**, a (C₁-C₆)alkyl and a (C₁-C₆)haloalkyl group,
more preferably **R₄** represents a hydrogen atom, a halogen atom, N**R₃₁R₃₂**, a (C₁-C₆)alkyl, an aryl or a heterocyclyl group, wherein said aryl or heterocyclyl group is optionally substituted by one or more substituents selected from the group consisting of C(O)**R₄₈** and a (C₁-C₆)alkyl group,
even more preferably **R₄** represents a hydrogen atom or a (C₁-C₃)alkyl group, advantageously a hydrogen atom,
**R_{4b}** represents a hydrogen atom, a halogen atom, a (C₁-C₆)alkyl group, O**R₂₉** or N**R₃₁R₃₂**, preferably a hydrogen atom, a halogen atom, a (C₁-C₃)alkyl group or O**R₂₉**, more preferably a hydrogen atom or a (C₁-C₃)alkyl group, advantageously a hydrogen atom,
wherein, in the above definitions of **R₄** and **R_{4b}**, **R₂₉** to **R₃₂** represent, independently of each other, a hydrogen atom, a (C₁-C₆)alkyl, an aryl or an aryl-(C₁-C₆)alkyl group, said aryl group being optionally substituted by one or more substituents selected from the group consisting of a halogen atom, O**R₅₅**, S**R₅₆**, N**R₅₇R₅₈**, C(O)**R₅₉**, CO₂**R₆₀**, OC(O)**R₆₁**, N**R₆₂**C(O)**R₆₃**, C(O)N**R₆₄R₆₅**, a (C₁-C₆)alkyl and a (C₁-C₆)haloalkyl group, notably C(O)**R₅₉**, CO₂**R₆₀**, C(O)N**R₆₄R₆₅**, a (C₁-C₆)alkyl and a (C₁-C₆)haloalkyl group, in particular C(O)**R₅₉**, **R₄₈** represents a hydrogen atom, a (C₁-C₆)alkyl or an aryl group, notably an aryl group, and **R₅₅** to **R₆₅** represent, independently of each other, a hydrogen atom, a (C₁-C₆)alkyl or an aryl group, notably an aryl group;
▪ **R₅** represents a hydrogen atom, a halogen atom, CN, O**R₂₉**, S**R₃₀**, N**R₃₁R₃₂**, a (C₁-C₆)alkyl, a (C₁-C₆)haloalkyl group, said alkyl or haloalkyl group being optionally substituted by one or more substituents selected from the group consisting of O**R₄₀**, S**R₄₁** and N**R₄₂R₄₃**, an aryl, a heterocyclyl, an aryl-(C₁-C₆)alkyl or a heterocyclyl-(C₁-C₆)alkyl group, wherein said aryl or heterocyclyl group is optionally substituted by one or more substituents selected from the group consisting of a halogen atom, O**R₄₄**, S**R₄₅**, N**R₄₆R₄₇**, a (C₁-C₆)alkyl and a (C₁-C₆)haloalkyl group, wherein **R₂₉** to **R₃₂** represent, independently of each other, a hydrogen atom or a (C₁-C₆)alkyl group, and **R₄₀** to **R₄₃** represent, independently of each other, a hydrogen atom or a (C₁-C₆)alkyl group, notably a hydrogen atom,
preferably **R₅** represents a hydrogen atom, a halogen atom, a (C₁-C₆)alkyl, a (C₁-C₆)haloalkyl group, said alkyl or haloalkyl group being optionally substituted by one or more substituents selected from the group consisting of O**R₄₀**, S**R₄₁** and N**R₄₂R₄₃**, an aryl-(C₁-C₆)alkyl or a heterocyclyl-(C₁-C₆)alkyl group, wherein said aryl or heterocyclyl group is optionally substituted by one or more substituents selected from the group consisting of a halogen atom, a (C₁-C₆)alkyl and a (C₁-C₆)haloalkyl group,
more preferably, **R₅** represents a hydrogen atom, a halogen atom, a (C₁-C₆)alkyl, a (C₁-C₆)haloalkyl group or a heterocyclyl-(C₁-C₆)alkyl group, said alkyl or haloalkyl group being optionally substituted by O**R₄₀**, and said heterocyclyl being optionally substituted by one or more (C₁-C₆)alkyl group,
even more preferably, **R₅** represents a hydrogen atom or a (C₁-C₃)alkyl group, advantageously a hydrogen atom; and
▪ **R₆** represents a hydrogen atom, a (C₁-C₃)alkyl, an aryl-(C₁-C₃)alkyl or a -CH₂-CH₂-O-CH₂-CH₂-NH₂ group, or a (C₁-C₆)alkylcarbonyl group optionally substituted with one or more substituents selected from the group consisting of OH, SH, NH₂, a (C₁-C₃)alkoxy, a (C₁-C₃)thioalkoxy and a (C₁-C₃)alkylamino group,
preferably **R₆** represents a hydrogen atom, a methyl, an ethyl, a benzyl, a -CH₂-CH₂-O-CH₂-CH₂-NH₂ or a (C₁-C₆)alkylcarbonyl group optionally substituted with one or more substituents selected from the group consisting of OH, NH₂ and a thiomethyl group, in particular **R₆** represents a hydrogen atom or a -CH₂-CH₂-O-CH₂-CH₂-NH₂ group.

4. The pharmaceutical composition according to claim 1, wherein the inhibitor of regulated necrotic cell death is a compound of formula **(II),** wherein:
▪ **X₁** represents a (C₁-C₆)alkyl, an aryl, an aryl-(C₁-C₆)alkyl or an O**R_{X}** group, wherein **R_{X}** is a (C₁-C₆)alkyl, an aryl or an aryl-(C₁-C₆)alkyl group;
▪ **X₂** and **X₃** represent, independently of each other, a hydrogen atom or a (C₁-C₆)alkyl group; and
▪ **Y₁, Y₂** and **Y₃** represent, independently of each other, a hydrogen atom, a (C₁-C₆)alkyl, an aryl, an aryl-(C₁-C₆)alkyl, an OH or an O**R_{Y}** group, wherein at least one of **Y₁, Y₂** and **Y₃** represents a (C₁-C₆)alkyl, an aryl, an aryl-(C₁-C₆)alkyl or an O**R_{Y}** group, wherein **R_{Y}** is selected from a (C₁-C₆)alkyl, an aryl and an aryl-(C₁-C₆)alkyl group.

5. The pharmaceutical composition according to claim 4, wherein:
▪ **X₁** represents an O**R_{X}** group, **R_{X}** being advantageously a (C₁-C₆)alkyl group;
▪ **Y₁** represents an **OR_{Y}** group, **R_{Y}** being advantageously an aryl-(C₁-C₆)alkyl group, and **Y₂** and **Y₃** each represent a hydrogen atom.

6. The pharmaceutical composition according to claim 1, wherein the inhibitor of regulated necrotic cell death is:
**(a)** a compound of the following general formula **(I.iii):** 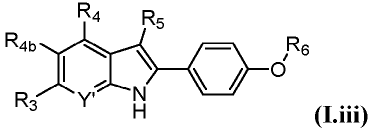 or a pharmaceutically acceptable salt and/or solvate thereof, wherein:
- **R₃, R₄**, **R_{4b}** and **R₅** represent, independently of each other, a hydrogen atom, a halogen atom or a (C₁-C₆)alkyl group, in particular a hydrogen atom, a halogen atom or a (C₁-C₃)alkyl group, preferably a hydrogen atom or a halogen atom, and
- **R₆** represents a hydrogen atom, a (C₁-C₃)alkyl or a -CH₂-CH₂-O-CH₂-CH₂-NH₂ group, in particular a hydrogen atom or a -CH₂-CH₂-O-CH₂-CH₂-NH₂ group;
or
(b) a compound of the following general formula **(II.i):** 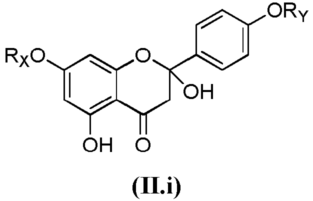 or a pharmaceutically acceptable salt and/or solvate thereof, wherein:
- **R_{X}** represents a (C₁-C₆)alkyl group, notably a (C₁-C₃)alkyl group such as methyl, ethyl, n-propyl, more advantageously methyl, and
- **R_{Y}** represents an aryl-(C₁-C₆)alkyl group, such as benzyl or -CH₃-naphtyl, more preferably benzyl.

7. The pharmaceutical composition according to any one of claims 1 to 6, wherein the inhibitor of regulated necrotic cell death is selected from the group consisting of: 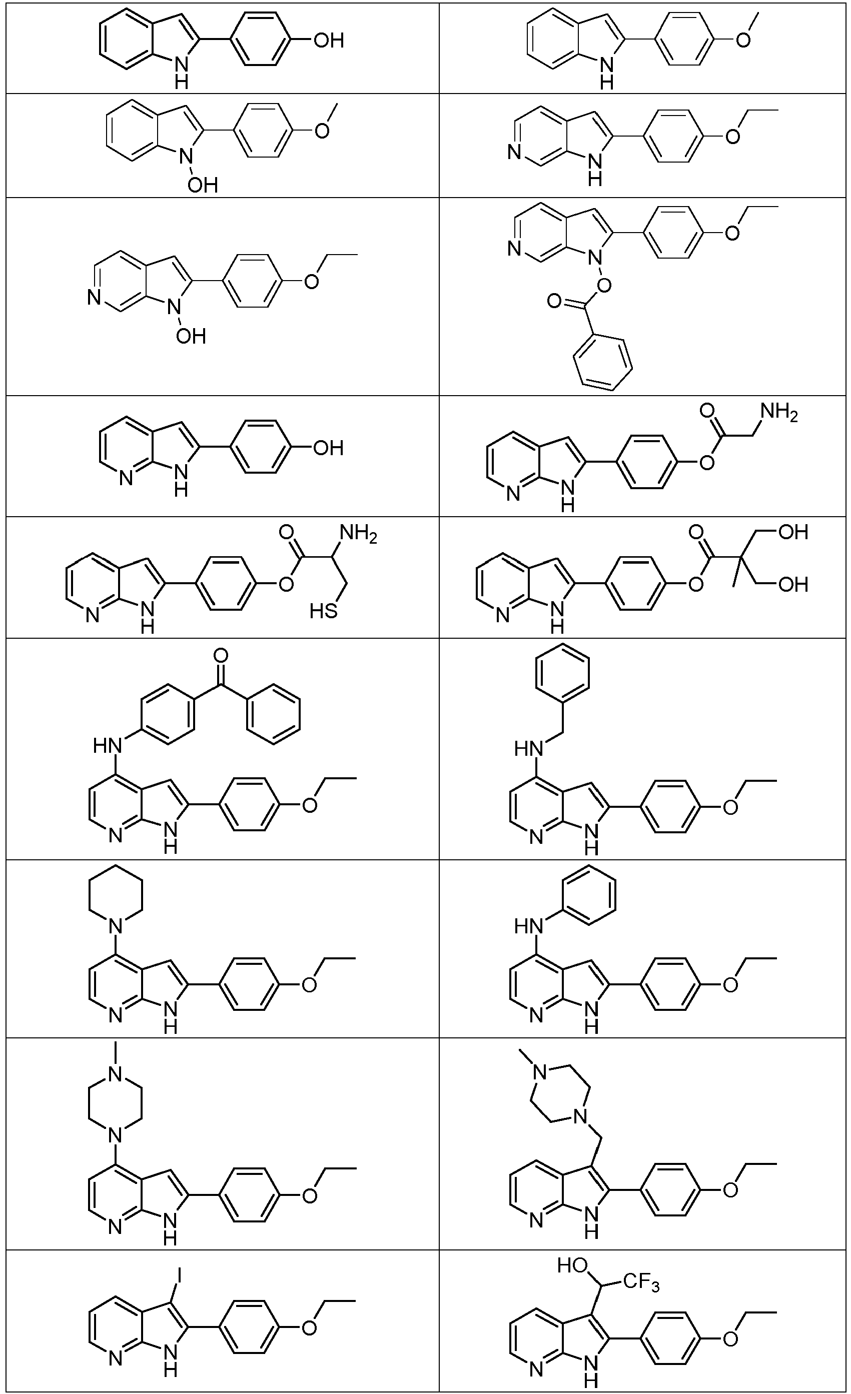 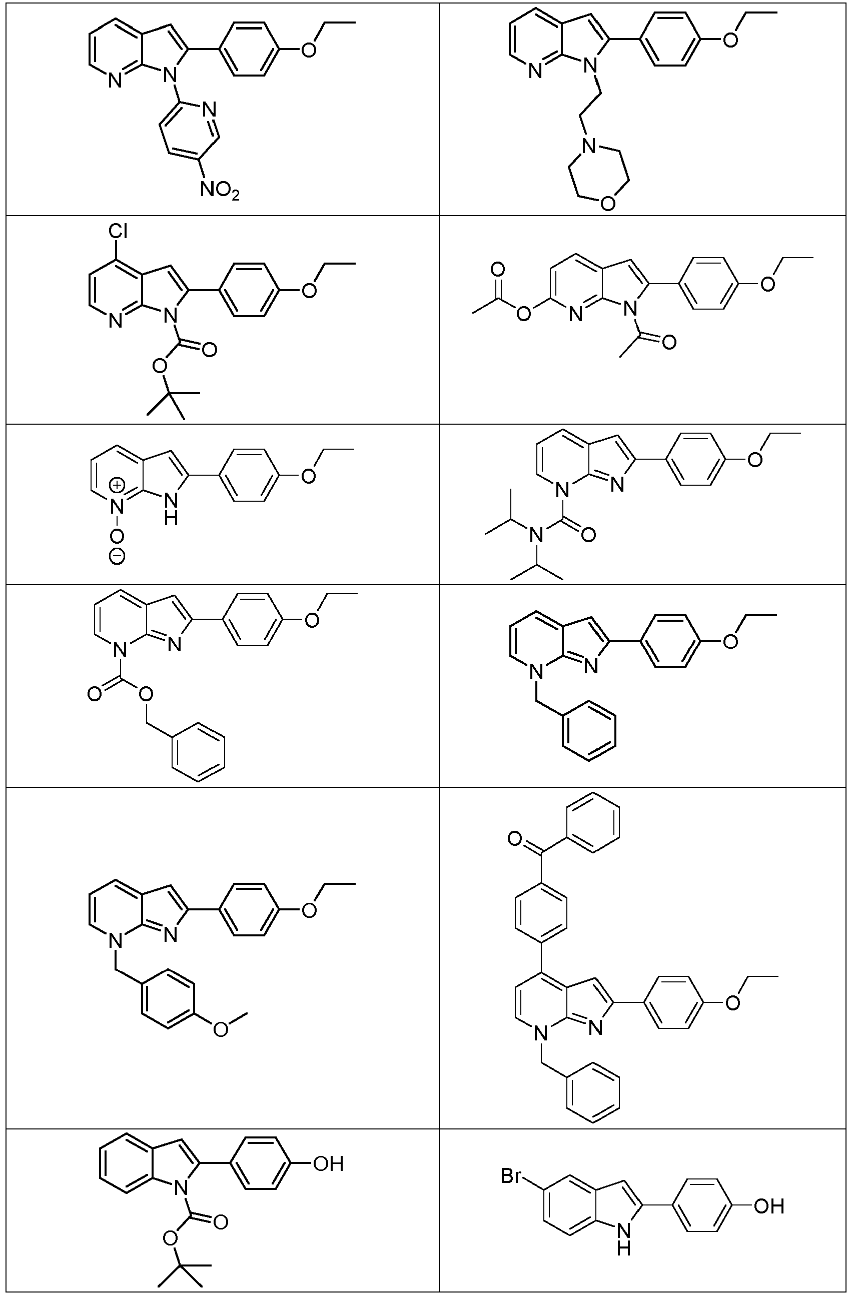 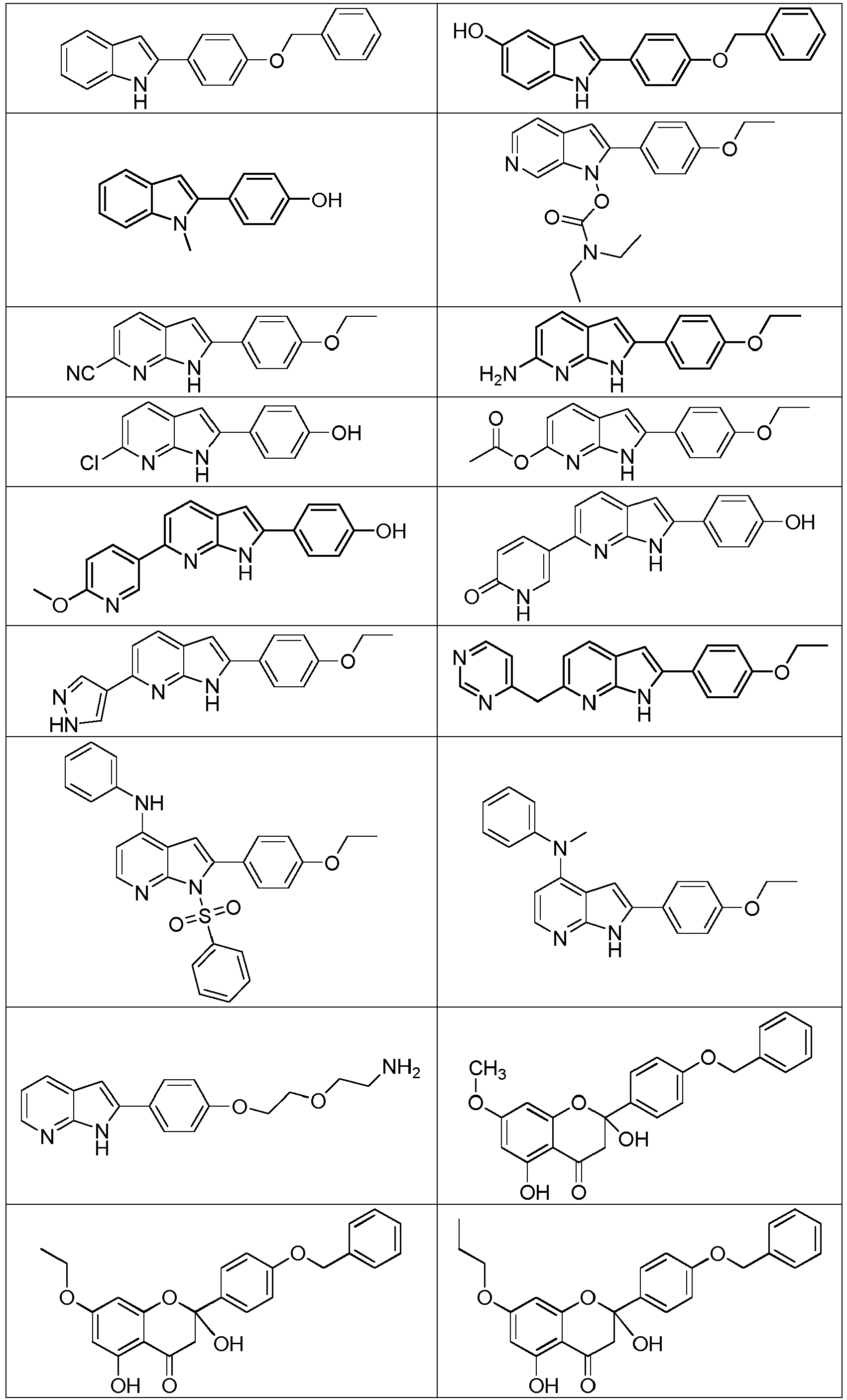 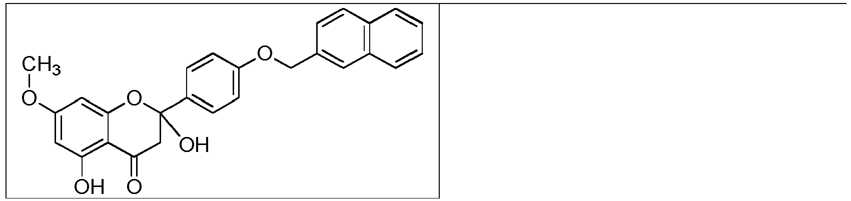 and the pharmaceutically acceptable salts and/or solvates thereof.

8. The pharmaceutical composition according to any one of claims 1 to 7, wherein the inhibitor of regulated necrotic cell death is selected from the group consisting of: 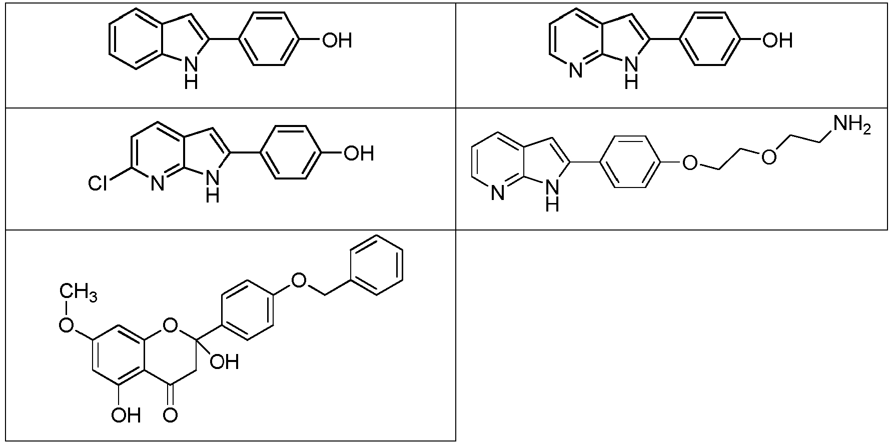 and the pharmaceutically acceptable salts and/or solvates thereof.

9. The pharmaceutical composition according to any one of claims 1 to 8 comprising:
- N-acetylcysteine and/or a pharmaceutical salt and/or a derivative thereof, the derivative being as defined in claim 1, and
- at least one inhibitor of regulated necrotic cell death
as a combination product for simultaneous, separate or sequential administration.

10. The pharmaceutical composition according to any one of claims 1 to 9, for use in preventing and/or treating a disorder associated with regulated necrotic cell death, wherein the disorder is selected from the group consisting of brain diseases or disorders including neurodegenerative diseases or disorders, stroke, traumatic brain injury, epilepsy; eye diseases or disorders including retinopathy, degenerative eye diseases or disorders; infectious diseases; autoimmune diseases; inflammatory diseases; liver injury including acute liver failure and chronic liver diseases; hypertension; haemochromatosis; hemolytic disorders; ischemic disorders affecting the heart, brain, or kidneys; kidney injury including acute kidney injury, renal ischemia-reperfusion injury, acute tubular necrosis, liver fibrosis; heart injury; aortic aneurysm; pancreatitis; radiation-induced necrosis; chronic obstructive pulmonary disease, acute respiratory distress disorder; diseases related to transplantation; cancers including liver cancers, eye cancers, brain cancers, kidney cancers.

11. Use of a pharmaceutical composition according to any one of claims 1 to 9, for the *in vitro* preservation and/or protection of biological materials such as cells, tissues, body fluids and organs.

## Patentansprüche

1. Pharmazeutische Zusammensetzung, die eine Kombination umfasst aus:
N-Acetylcystein und/oder einem pharmazeutischen Salz und/oder einem Derivat davon, wobei das Derivat von N-Acetylcystein N-Acetylcysteinamid und/oder N-Acetylcysteinethylester und/oder N-Acetylcysteinmethylester und/oder Salz/e davon ist,
mit mindestens einem Inhibitor des regulierten nekrotischen Zelltods, wobei der Inhibitor des regulierten nekrotischen Zelltods ist:
**(A)** eine Verbindung mit der folgenden allgemeinen Formel **(I):** 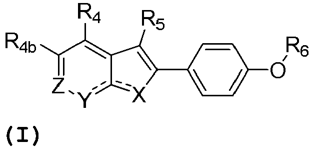
oder ein pharmazeutisch verträgliches Salz und/oder Solvat davon,
wobei:
▪ 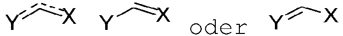 ist;
(i) wenn 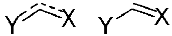 ist, ist **X** N, ist **Y** N**(R₂)** und ist **Z** C(H);
(ii) wenn 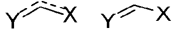 ist,
- ist **X** N(**R₁**) , und
- ist **Y** N oder N⁺(O⁻) und ist **Z** C(**R₃**), oder
ist **Y** CH und ist **Z** N, oder
sind **Y** und **Z** CH;
und wobei:
▪ **R₁** und **R₂** unabhängig voneinander ein Wasserstoffatom, CN, NO₂, O**R₇**, S**R₈,** N**R₉R₁₀**, C(O)**R₁₁**, CO₂**R₁₂**, OC (O)**R₁₃**, N**R₁₄**C(O)**R₁₅**, C (O) N**R₁₆R₁₇**, S (O)**R_{S}**, SO₂**R_{S}**' , eine (C₁-C₆)Alkyl-, eine (C₁-C₆) Halogenalkyl-, eine - (C₁-C₆)Alkyl- [O- (C₁-C₆) Alkyl]ₘ-N**R_{N1}R_{N2}-**Gruppe, wobei m zwischen 1 und 6 beträgt, eine Aryl-, eine Aryl- (C₁-C₆) alkyl-, eine Heterocyclyl- oder eine Heterocyclyl-(C₁-C₆) alkyl-Gruppe darstellen, wobei die Aryl- oder Heterocyclyl-Gruppe wahlweise durch einen oder mehrere Substituenten substituiert ist, der bzw. die aus der Gruppe ausgewählt ist bzw. sind, die besteht aus einem Halogenatom, CN, NO₂, O**R₁₈**, S**R₁₉**, N**R₂₀R₂₁**, C(O)**R₂₂**, CO₂**R₂₃**, OC(O)**R₂₄**, N**R₂₅**C(O)**R₂₆**, C (O) N**R₂₇R₂₈**, einer (C₁-C₆) Alkyl- und einer (C₁-C₆) Halogenalkyl-Gruppe;
▪ **R₃, R₄**, **R_{4b}** und **R₅** unabhängig voneinander ein Wasserstoffatom, ein Halogenatom, CN, O**R₂₉**, S**R₃₀**, N**R₃₁R₃₂**, C(O)**R₃₃**, CO₂**R₃₄**, OC (O)**R₃₅**, N**R₃₆**C(O)**R₃₇**, C (O) N**R₃₈R₃₉**, eine (C₁-C₆) Alkyl-, eine (C₁-C₆)Halogenalkyl-Gruppe darstellen, wobei die Alkyl- oder Halogenalkyl-Gruppe wahlweise durch einen oder mehrere Substituenten substituiert ist, der bzw. die aus der Gruppe ausgewählt ist bzw. sind, die besteht aus O**R₄₀**, S**R₄₁** und N**R₄₂R₄₃**, einer Aryl-, einer Heterocyclyl-, einer Aryl- (C₁-C₆) alkyl- oder einer Heterocyclyl-(C₁-C₆)Alkyl-Gruppe, wobei die Aryl- oder Heterocyclyl-Gruppe wahlweise durch einen oder mehrere Substituenten substituiert ist, der bzw. die aus der Gruppe ausgewählt ist bzw. sind, die besteht aus einem Halogenatom, CN, NO₂, O**R₄₄**, S**R₄₅**, N**R₄₆R₄₇**, C(O)**R₄₈**, CO₂**R₄₉**, OC (O)**R₅₀**, N**R₅₁**C(O)**R₅₂**, C (O) N**R₅₃R₅₄**, einer (C₁-C₆) Alkyl- und einer (C₁-C₆)Halogenalkyl-Gruppe;
▪ **R₆** ein Wasserstoffatom, eine (C₁-C₆)Alkyl-, eine Aryl- (C₁-C₆) alkyl-, eine Heterocyclyl- (C₁-C₆) alkyl-, eine - (C₁-C₆) Alkyl- [O- (C₁-C₆) alkyl]ₘ,-N**R_{N'1}R_{N'2}-**Gruppe, wobei m' im zwischen 1 und 6 beträgt, oder eine (C₁-C₆)Alkylcarbonyl-Gruppe darstellt, wobei die (C₁-C₆) Alkyl-, Aryl- (C₁-C₆) Alkyl- und (C₁-C₆)Alkylcarbonyl-Gruppe wahlweise mit einem oder mehreren Substituenten substituiert sind, der bzw. die aus der Gruppe ausgewählt ist bzw. sind, die besteht aus OH, SH, NH₂, einer (C₁-C₆)Alkoxy-, einer (C₁-C₆) Thioalkoxy-, einer (C₁-C₆) Alkylamino- und einer Di ( (C₁-C₆) alkyl) amino-Gruppe;
▪ **R_{S}** und **R_{S}**' unabhängig voneinander eine (C₁-C₆)Alkyl-, eine Aryl- oder eine Aryl-(C₁-C₆)Alkyl-Gruppe darstellen;
▪ **R₇-R₁₀, R₁₂, R₁₄** und **R₁₆-R₁₇** unabhängig voneinander ein Wasserstoffatom, eine (C₁-C₆)Alkyl-, eine Aryl- oder eine Aryl-(C₁-C₆)Alkyl-Gruppe darstellen;
▪ **R₁₁**, **R₁₃** und **R₁₅** unabhängig voneinander ein Wasserstoffatom, eine (C₁-C₆)Alkyl-, eine Aryl-, eine Aryl- (C₁-C₆) Alkyl-, eine (C₁-C₆)Alkoxygruppe, eine (C₁-C₆) Alkylamino- oder eine Di ( (C₁-C₆)alkyl)amino-Gruppe darstellen;
▪ **R₁₈** bis **R₂₈** unabhängig voneinander ein Wasserstoffatom, eine (C₁-C₆)Alkyl- oder eine Aryl-Gruppe darstellen;
▪ **R₂₉** bis **R₃₉** unabhängig voneinander ein Wasserstoffatom, eine (C₁-C₆)Alkyl-, Heterocyclyl-(C₁-C₆) Alkyl-, eine Aryl- oder eine Aryl- (C₁-C₆) Alkyl-Gruppe darstellen, wobei die Aryl-Gruppe wahlweise durch einen oder mehrere Substituenten substituiert ist, der bzw. die aus der Gruppe ausgewählt ist bzw. sind, die besteht aus einem Halogenatom, CN, NO₂, O**R₅₅**, S**R₅₆**, N**R₅₇R₅₈**, C(O)**R₅₉**, CO₂**R₆₀**, OC(O)**R₆₁**, N**R₆₂**C(O)**R₆₃**, C(O)N**R₆₄R₆₅**, einer (C₁-C₆)Alkyl- und einer (C₁-C₆) Halogenalkyl-Gruppe;
▪ **R₄₀** bis **R₄₃** unabhängig voneinander ein Wasserstoffatom oder eine (C₁-C₆)Alkyl-Gruppe darstellen;
▪ **R₄₄** bis **R₅₄** unabhängig voneinander ein Wasserstoffatom, eine (C₁-C₆) Alkyl- oder eine Arylgruppe darstellen;
▪ **R₅₅** bis **R₆₅** unabhängig voneinander ein Wasserstoffatom, eine (C₁-C₆) Alkyl-, Aryl-(C₁-C₆)alkyl- oder eine Arylgruppe darstellen; und
**R_{N1}, R_{N'1}, R_{N2}** und **R_{N'2}** unabhängig voneinander ein Wasserstoffatom, eine (C₁-C₆) Alkyl-Gruppe, eine Aryl- (C₁-C₆)alkyl-Gruppe oder eine Arylgruppe darstellen;
oder
**(B)** eine Verbindung mit der folgenden allgemeinen Formel **(II):** 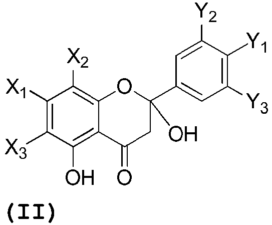
oder ein pharmazeutisch verträgliches Salz und/oder Solvat davon, wobei:
▪ **X₁, X₂** und **X₃** unabhängig voneinander ein Wasserstoffatom, eine (C₁-C₆)Alkyl-, eine Aryl-, eine Aryl-(C₁-C₆)alkyl- oder eine OH-Gruppe oder eine Gruppe darstellen, die ausgewählt ist aus **OR_{X}, SR_{X},** SO₂**R_{X}** und **NR_{X}R_{Z},**
wobei mindestens eines von **X₁, X₂** und **X₃** eine (C₁-C₆)Alkyl-, eine Aryl- oder eine Aryl-(C₁-C₆)Alkyl-Gruppe oder eine Gruppe darstellt, die ausgewählt ist aus O**R_{X},** S**R_{X},** SO₂**R_{X}** und N**R_{X}R_{Z},** wobei
**R_{X}** eine (C₁-C₆)Alkyl-, eine Aryl- oder eine Aryl-(C₁-C₆) alkyl-Gruppe darstellt,
**R_{Z}** ein Wasserstoffatom oder eine (C₁-C₆)Alkyl-Gruppe ist, und
die Arylgruppen wahlweise mit einer oder mehreren Gruppen substituiert sind, die ausgewählt ist bzw. sind aus einem Halogenatom, -O**R₆₆**, -N**R₆₇R₆₈**, -S**R₆₉**, -S (O)**R₇₀**, - SO₂**R₇₁**, -OCO**R₇₂**, -CO₂**R₇₃**, -CON**R₇₄R₇₅**, -CO₂**R₇₆**, Nitro (-NO₂) und Cyano (-CN);
▪ **Y₁, Y₂** und **Y₃** unabhängig voneinander ein Wasserstoffatom, eine (C₁-C₆)Alkyl-, eine Aryl-, eine Aryl-(C₁-C₆)alkyl oder eine OH-Gruppe oder eine Gruppe darstellen, die ausgewählt ist aus O**R_{Y}**, S**R_{Y}**, SO₂**R_{Y}** und N**R_{Y}R**' **_{Z}**,
wobei mindestens eines von **Y₁, Y₂** und **Y₃** eine (C₁-C₆)Alkyl-, eine Aryl- oder eine Aryl- (C₁-C₆) alkyl oder eine Gruppe darstellt, die ausgewählt ist aus O**R_{Y}**, S**R_{Y}**, SO₂**R_{Y}** und N**R_{Y}R'_{Z}**, wobei
**R_{Y}** eine (C₁-C₆) Alkyl-, eine Aryl- oder eine Aryl-(C₁-C₆) alkyl-Gruppe ist,
**R_{Z}** ein Wasserstoffatom oder eine (C₁-C₆)Alkyl-Gruppe ist, und
die Arylgruppen wahlweise mit einer oder mehreren Gruppen substituiert sind, die ausgewählt sind aus einem Halogenatom, -O**R₆₆**, -N**R₆₇R₆₈**, -S**R₆₉**, -S(O)**R₇₀**, -SO₂**R₇₁**, - OCO**R₇₂**, -CO₂**R₇₃,** -CON**R₇₄R₇₅**, -CO₂**R₇₆**, Nitro (-NO₂) und Cyano (-CN);
und
▪ **R₆₆** bis **R₇₇** unabhängig voneinander ein Wasserstoffatom oder eine (C₁-C₆)Alkyl-Gruppe darstellen.

2. Pharmazeutische Zusammensetzung nach Anspruch 1, wobei der Inhibitor des regulierten nekrotischen Zelltods eine Verbindung mit der Formel **(I)** ist, wobei:
▪ **R₁** und **R₂** unabhängig voneinander ein Wasserstoffatom, CN, NO₂, O**R₇**, S**R₈,** N**R₉R₁₀**, C(O)**R₁₁**, CO₂**R₁₂**, OC (O) **R₁₃**, N**R₁₄**C(O)**R₁₅**, C (O) N**R₁₆R₁₇**, S(O)**R_{S}**, SO₂**R_{S}**' , eine (C₁-C₆)Alkyl-, eine (C₁-C₆) Halogenalkyl-, eine Aryl-, eine Heterocyclyl-, eine Aryl- (C₁-C₆) alkyl- oder eine Heterocyclyl- (C₁-C₆) alkyl-Gruppe darstellen, wobei die Aryl- oder Heterocyclyl-Gruppe wahlweise durch einen oder mehrere Substituenten substituiert ist, der bzw. die aus der Gruppe ausgewählt ist bzw. sind, die besteht aus einem Halogenatom, CN, NO₂, O**R₁₈**, S**R₁₉**, N**R₂₀R₂₁**, C(O)**R₂₂**, CO₂**R₂₃**, OC(O)**R₂₄**, N**R₂₅**C(O)**R₂₆**, C (O) N**R₂₇R₂₈**, einer (C₁-C₆) Alkyl- und einer (C₁-C₆)Halogenalkyl-Gruppe;
▪ **R₃, R₄**, **R_{4b}** und **R₅** unabhängig voneinander ein Wasserstoffatom, ein Halogenatom, CN, O**R₂₉**, S**R₃₀**, N**R₃₁R₃₂**, C(O)**R₃₃**, CO₂**R₃₄**, OC(O)**R₃₅**, N**R₃₆**C(O)**R₃₇**, C (O) N**R₃₈R₃₉**, eine (C₁-C₆) Alkyl-, eine (C₁-C₆)Halogenalkyl-Gruppe darstellen, wobei die Alkyl- oder Halogenalkyl-Gruppe wahlweise durch einen oder mehrere Substituenten substituiert ist, der bzw. die aus der Gruppe ausgewählt ist bzw. sind, die besteht aus O**R₄₀**, S**R₄₁** und N**R₄₂R₄₃**, einer Aryl-, einer Heterocyclyl-, einer Aryl- (C₁-C₆) alkyl- oder einer Heterocyclyl-(C₁-C₆)Alkyl-Gruppe, wobei die Aryl- oder Heterocyclyl-Gruppe wahlweise durch einen oder mehrere Substituenten substituiert ist, der bzw. die aus der Gruppe ausgewählt ist bzw. sind, die besteht aus einem Halogenatom, CN, NO₂, O**R₄₄**, S**R₄₅**, N**R₄₆R₄₇**, C(O)**R₄₈**, CO₂**R₄₉**, OC(O)**R₅₀**, N**R₅₁**C(O)**R₅₂**, C (O) N**R₅₃R₅₄**, einer (C₁-C₆) Alkyl- und einer (C₁-C₆)Halogenalkyl-Gruppe;
▪ **R₆** ein Wasserstoffatom, eine (C₁-C₆)Alkyl-, eine Aryl- (C₁-C₆) alkyl oder eine -CH₂-CH₂-O-CH₂-CH₂-NH₂-Gruppe oder eine (C₁-C₆)Alkylcarbonyl-Gruppe darstellt, die wahlweise mit einem oder mehreren Substituenten substituiert ist, der bzw. die aus der Gruppe ausgewählt ist bzw. sind, die besteht aus OH, SH, NH₂, einer (C₁-C₆) Alkoxy-, einer (C₁-C₆) Thioalkoxy-, einer (C₁-C₆) Alkylamino- und einer Di((C₁-C₆) alkyl) amino-Gruppe;
▪ **R_{S}** und **R_{S}'** unabhängig voneinander eine (C₁-C₆)Alkyl-, eine Aryl- oder eine Aryl-(C₁-C₆)Alkyl-Gruppe darstellen;
▪ **R₇-R₁₀, R₁₂, R₁₄** und **R₁₆-R₁₇** unabhängig voneinander ein Wasserstoffatom, eine (C₁-C₆)Alkyl-, eine Aryl- oder eine Aryl-(C₁-C₆)Alkyl-Gruppe darstellen;
▪ **R₁₁, R₁₃** und **R₁₅** unabhängig voneinander ein Wasserstoffatom, eine (C₁-C₆)Alkyl-, eine Aryl-, eine Aryl- (C₁-C₆) Alkyl-, eine (C₁-C₆) Alkoxy-, eine (C₁-C₆)Alkylamino- oder eine Di ( (C₁-C₆) alkyl) amino-Gruppe darstellen;
▪ **R₁₈** bis **R₂₈** unabhängig voneinander ein Wasserstoffatom, eine (C₁-C₆) Alkyl- oder eine Arylgruppe darstellen;
▪ **R₂₉** bis **R₃₉** unabhängig voneinander ein Wasserstoffatom, eine (C₁-C₆)Alkyl-, eine Aryl- oder eine Aryl-(C₁-C₆)Alkyl-Gruppe darstellen, wobei die Arylgruppe wahlweise durch einen oder mehrere Substituenten substituiert ist, der bzw. die aus der Gruppe ausgewählt ist bzw. sind, die besteht aus einem Halogenatom, CN, NO₂, O**R₅₅**, S**R₅₆,** N**R₅₇R₅₈**, C(O)**R₅₉,** CO₂**R₆₀**, OC(O)**R₆₁,** N**R₆₂**C(O)**R₆₃,** C(O)N**R₆₄R₆₅,** einer (C₁-C₆) Alkyl- und einer (C₁-C₆) Halogenalkyl-Gruppe;
▪ **R₄₀** bis **R₄₃** unabhängig voneinander ein Wasserstoffatom oder eine (C₁-C₆)Alkyl-Gruppe darstellen;
▪ **R₄₄** bis **R₅₄** unabhängig voneinander ein Wasserstoffatom, eine (C₁-C₆) Alkyl- oder eine Arylgruppe darstellen; und
▪ **R₅₅** bis **R₆₅** unabhängig voneinander ein Wasserstoffatom, eine (C₁-C₆) Alkyl- oder eine Arylgruppe darstellen.

3. Pharmazeutische Zusammensetzung nach Anspruch 1, wobei der Inhibitor des regulierten nekrotischen Zelltods eine Verbindung mit der Formel **(I)** ist, wobei:
▪ **R₁** ein Wasserstoffatom, CN, NO₂, O**R₇**, S**R₈,** N**R₉R₁₀,** C(O)**R₁₁**, CO₂**R₁₂**, OC(O)**R₁₃,** N**R₁₄**C(O)R**₁₅,** C(O)N**R₁₆R₁₇**, **S** (O)**R_{S}, SO₂R_{S}' ,** eine (C₁-C₆) Alkyl-, eine (C₁-C₆)Halogenalkyl-, eine Aryl-, eine Heterocyclyl-, eine Aryl- (C₁-C₆)alkyl oder eine Heterocyclyl- (C₁-C₆)alkyl-Gruppe darstellt, wobei die Aryl- oder die Heterocyclyl-Gruppe wahlweise mit einem Substituenten substituiert ist, der ausgewählt ist aus der Gruppe, die besteht aus einem Halogenatom, CN, NO₂, O**R₁₈**, S**R₁₉**, N**R₂₀R₂₁**, einer (C₁-C₆)Alkyl- und einer (C₁-C₆)Halogenalkyl-Gruppe, wobei **R₁₈** bis **R₂₁** unabhängig voneinander ein Wasserstoffatom oder eine (C₁-C₆) Alkyl-Gruppe darstellen und **R_{S}** und **R_{S}**' unabhängig voneinander eine (C₁-C₆)Alkyl- oder eine Aryl-Gruppe, vorzugsweise eine Aryl-Gruppe, darstellen,
**R₁** vorzugsweise ein Wasserstoffatom, CN, O**R₇**, C(O)**R₁₁**, CO₂**R₁₂**, **OC(O)R₁₃, SO₂R_{S}' ,** eine (C₁-C₆)Alkyl-, eine Heterocyclyl- oder eine Heterocyclyl-(C₁-C₆)Alkyl-Gruppe darstellt, wobei die Heterocyclyl-Gruppe wahlweise durch NO₂ substituiert ist,
noch bevorzugter **R₁** ein Wasserstoffatom oder eine (C₁-C₆)Alkyl-Gruppe, insbesondere ein Wasserstoffatom oder eine (C₁-C₃)Alkyl-Gruppe, vorteilhafterweise ein Wasserstoffatom, darstellt;
▪ **R₂** C(O)**R₁₁,** CO₂**R₁₂**, C(O)N**R₁₆R₁₇,** eine (C₁-C₆) Alkyl-, eine (C₁-C₆) Halogenalkyl-, eine Aryl-, eine Heterocyclyl-, eine Aryl-(C₁-C₆)Alkyl- oder eine Heterocyclyl-(C₁-C₆)alkyl-Gruppe darstellen, wobei die Aryl- oder Heterocyclyl-Gruppe wahlweise durch einen oder mehrere Substituenten substituiert ist, der bzw. die aus der Gruppe ausgewählt ist bzw. sind, die besteht aus einem Halogenatom, O**R₁₈**, S**R₁₉**, N**R₂₀R₂₁**, einer (C₁-C₆) Alkyl- und einer (C₁-C₆) Halogenalkyl-Gruppe, wobei **R₁₈** bis **R₂₁** unabhängig voneinander ein Wasserstoffatom oder eine (C₁-C₆)Alkyl-Gruppe darstellen,
vorzugsweise **R₂** CO₂**R₁₂**, C (O) N**R₁₆R₁₇** oder eine Aryl-(C₁-C₆) Alkyl-Gruppe darstellt, wobei die Arylgruppe wahlweise durch einen oder mehrere Substituenten substituiert ist, der bzw. die aus der Gruppe ausgewählt ist bzw. sind, die besteht aus einem Halogenatom, O**R₁₈**, S**R₁₉** und N**R₂₀R₂₁**, insbesondere O**R₁₈;**
▪ **R₃** ein Wasserstoffatom, ein Halogenatom, eine (C₁-C₆) Alkyl-Gruppe, CN, O**R₂₉**, S**R₃₀**, N**R₃₁R₃₂**, C(O)**R₃₃,** CO₂**R₃₄**, **OC(O)R₃₅,** N**R₃₆**C(O)**R₃₇,** C (O) N**R₃₈R₃₉**, eine Aryl-, eine Heterocyclyl-, eine Aryl- (C₁-C₆) alkyl- oder eine Heterocyclyl- (C₁-C₆) alkyl-Gruppe darstellt, wobei die Aryl- oder Heterocyclyl-Gruppe wahlweise durch einen oder mehrere Sustituenten substituiert ist, der bzw. die ausgewählt ist bzw. sind aus der Gruppe, die besteht aus einem Halogenatom, O**R₄₄**, S**R₄₅**, N**R₄₆R₄₇**, einer (C₁-C₆) Alkyl- und einer (C₁-C₆)Halogenalkyl-Gruppe, wobei **R₂₉** bis **R₃₇** unabhängig voneinander ein Wasserstoffatom oder eine (C₁-C₆) Alkyl-Gruppe darstellen und **R₄₄** bis **R₄₇** unabhängig voneinander ein Wasserstoffatom oder eine (C₁-C₆)Alkyl-Gruppe darstellen,
vorzugsweise **R₃** ein Wasserstoffatom, ein Halogenatom, eine (C₁-C₆) Alkyl-Gruppe, CN, O**R₂₉**, S**R₃₀**, N**R₃₁R₃₂**, OC(O)**R₃₅,** N**R₃₆**C(O)**R₃₇**, eine Heterocyclyl- oder eine Heterocyclyl-(C₁-C₆)Alkyl-Gruppe darstellt, wobei die Heterocyclyl-Gruppe wahlweise durch einen oder mehrere Substituenten substituiert ist, der bzw. die aus der Gruppe ausgewählt ist bzw. sind, die besteht aus einem Halogenatom, O**R₄₄**, S**R₄₅**, N**R₄₆R₄₇** und einer (C₁-C₆) Alkyl-Gruppe,
bevorzugter **R₃** ein Wasserstoffatom, ein Halogenatom, eine (C₁-C₆) Alkyl-Gruppe, CN, N**R₃₁R₃₂**, OC(O)**R₃₅,** eine Heterocyclyl- oder eine Heterocyclyl-(C₁-C₆)Alkyl-Gruppe darstellt, wobei die Heterocyclyl-Gruppe wahlweise durch einen oder mehrere Substituenten substituiert ist, der bzw. die ausgewählt ist bzw. sind aus der Gruppe, die besteht aus einem O**R₄₄**, S**R₄₅** und N**R₄₆R₄₇**, insbesondere O**R₄₄**,
noch bevorzugter **R₃** ein Wasserstoffatom, ein Halogenatom oder eine (C₁-C₃)Alkyl-Gruppe, vorteilhafterweise ein Wasserstoffatom oder ein Halogenatom, darstellt;
▪ **R₄** ein Wasserstoffatom, ein Halogenatom CN, O**R₂₉**, S**R₃₀**, N**R₃₁R₃₂**, eine (C₁-C₆) Alkyl-, eine (C₁-C₆)Halogenalkyl-Gruppe, eine Aryl-, eine Heterocyclyl-, eine Aryl- (C₁-C₆) alkyl- oder eine Heterocyclyl-(C₁-C₆)alkyl-Gruppe darstellt, wobei die Aryl- oder Heterocyclyl-Gruppe wahlweise durch einen oder mehrere Substituenten substituiert ist, der bzw. die ausgewählt ist bzw. sind aus der Gruppe, die besteht aus einem Halogenatom, CN, NO₂, O**R₄₄**, S**R₄₅**, N**R₄₆R₄₇**, C(O)**R₄₈,** CO₂**R₄₉,** OC(O)**R₅₀**, N**R₅₁**C(O)**R₅₂,** C(O)N**R₅₃R₅₄**, einer (C₁-C₆) Alkyl- und einer (C₁-C₆) Halogenalkyl-Gruppe,
**R₄** vorzugsweise ein Wasserstoffatom, ein Halogenatom, O**R₂₉**, S**R₃₀**, N**R₃₁R₃₂**, eine (C₁-C₆) Alkyl-, eine Aryl- oder eine Heterocyclyl-Gruppe darstellt, wobei die Aryl- oder Heterocyclyl-Gruppe wahlweise durch einen oder mehrere Substituenten substituiert ist, der bzw. die aus der Gruppe ausgewählt ist bzw. sind, die besteht aus einem Halogenatom, O**R₄₄**, S**R₄₅**, N**R₄₆R₄₇**, C(O)**R₄₈**, CO₂**R₄₉**, C (O) N**R₅₃R₅₄**, einer (C₁-C₆) Alkyl- und einer (C₁-C₆)Halogenalkyl-Gruppe,
bevorzugter **R₄** ein Wasserstoffatom, ein Halogenatom, N**R₃₁R₃₂**, eine (C₁-C₆)Alkyl-, eine Aryl- oder eine Heterocyclyl-Gruppe darstellt, wobei die Aryl- oder Heterocyclyl-Gruppe wahlweise durch einen oder mehrere Substituenten substituiert ist, der bzw. die aus der Gruppe ausgewählt ist bzw. sind, die besteht aus C(O)**R₄₈** und einer (C₁-C₆)Alkyl-Gruppe,
**R₄** noch bevorzugter ein Wasserstoffatom oder eine (C₁-C₃)Alkyl-Gruppe, vorteilhafterweise ein Wasserstoffatom, darstellt,
**R_{4b}** ein Wasserstoffatom, ein Halogenatom, eine (C₁-C₆) Alkyl-Gruppe, O**R₂₉** oder N**R₃₁R₃₂**, vorzugsweise ein Wasserstoffatom, ein Halogenatom, eine (C₁-C₃)Alkyl-Gruppe oder O**R₂₉**, bevorzugter ein Wasserstoffatom oder eine (C₁-C₃)Alkyl-Gruppe, vorteilhafterweise ein Wasserstoffatom, darstellt,
wobei in den vorhergehenden Definitionen von **R₄** und **R_{4b}, R₂₉** bis **R₃₂** unabhängig voneinander ein Wasserstoffatom, eine (C₁-C₆)Alkyl-, eine Aryl- oder eine Aryl- (C₁-C₆) Alkyl-Gruppe darstellen, wobei die Arylgruppe wahlweise durch einen oder mehrere Substituenten substituiert ist, der bzw. die aus der Gruppe ausgewählt ist bzw. sind, die besteht aus einem Halogenatom, O**R₅₅**, S**R₅₆**, N**R₅₇R₅₈**, C**(O)R₅₉,** CO₂R₆₀, OC (O) **R₆₁,** N**R₆₂**C(O)**R₆₃**, C (O) N**R₆₄R₆₅,** einer (C₁-C₆) Alkyl- und einer (C₁-C₆) Halogenalkyl-Gruppe, insbesondere C(O)**R₅₉**, CO₂**R₆₀**, C (O) N**R₆₄R₆₅,** einer (C₁-C₆) Alkyl- und einer (C₁-C₆)Halogenalkyl-Gruppe, insbesondere C(O)**R₃₉, R₄₈** ein Wasserstoffatom, eine (C₁-C₆)Alkyl- oder eine Arylgruppe, insbesondere eine Arylgruppe, darstellt, und R₅₅ bis **R₆₅** unabhängig voneinander ein Wasserstoffatom, eine (C₁-C₆)Alkyl- oder eine Arylgruppe, insbesondere eine Arylgruppe, darstellen;
▪ **R₅** ein Wasserstoffatom, ein Halogenatom, CN, O**R₂₉**, S**R₃₀**, N**R₃₁R₃₂**, eine (C₁-C₆) Alkyl-, eine (C₁-C₆)Halogenalkyl-Gruppe darstellt, wobei die Alkyl- oder Halogenalkyl-Gruppe wahlweise durch einen oder mehrere Substituenten substituiert ist, der bzw. die ausgewählt ist bzw. sind aus der Gruppe, die besteht aus O**R₄₀**, S**R₄₁** und N**R₄₂R₄₃**, einer Aryl-, einer Heterocyclyl-, einer Aryl- (C₁-C₆) alkyl oder einer Heterocyclyl-(C₁-C₆)alkyl-Gruppe, wobei die Aryl- oder Heterocyclyl-Gruppe wahlweise durch einen oder mehrere Substituenten substituiert ist, der bzw. die ausgewählt ist bzw. sind aus der Gruppe, die besteht aus einem Halogenatom, O**R₄₄**, S**R₄₅**, N**R₄₆R₄₇**, einer (C₁-C₆) Alkyl- und einer (C₁-C₆) Halogenalkyl-Gruppe, wobei **R₂₉** bis **R₃₂** unabhängig voneinander ein Wasserstoffatom oder eine (C₁-C₆)Alkyl-Gruppe darstellen und **R₄₀** bis **R₄₃** unabhängig voneinander ein Wasserstoffatom oder eine (C₁-C₆)Alkyl-Gruppe, insbesondere ein Wasserstoffatom, darstellen,
**R₅** vorzugsweise ein Wasserstoffatom, ein Halogenatom, eine (C₁-C₆) Alkyl-, eine (C₁-C₆)Halogenalkyl-Gruppe darstellt, wobei die Alkyl- oder Halogenalkyl-Gruppe wahlweise durch einen oder mehrere Substituenten substituiert ist, der bzw. die aus der Gruppe ausgewählt ist bzw. sind, die besteht aus O**R₄₀**, S**R₄₁** und N**R₄₂R₄₃**, einer Aryl- (C₁-C₆) alkyl- oder einer Heterocyclyl-(C₁-C₆)Alkyl-Gruppe, wobei die Aryl- oder Heterocyclyl-Gruppe wahlweise durch einen oder mehrere Substituenten substituiert ist, der bzw. die aus der Gruppe ausgewählt ist bzw. sind, die besteht aus einem Halogenatom, einer (C₁-C₆) Alkyl- und einer (C₁-C₆)Halogenalkyl-Gruppe,
bevorzugter **R₅** ein Wasserstoffatom, ein Halogenatom, eine (C₁-C₆) Alkyl-, eine (C₁-C₆)Halogenalkyl-Gruppe oder eine Heterocyclyl-(C₁-C₆)Alkyl-Gruppe darstellt, wobei die Alkyl- oder Halogenalkyl-Gruppe wahlweise durch O**R₄₀** substituiert ist und wobei das Heterocyclyl wahlweise durch eine oder mehrere (C₁-C₆) Alkyl-Gruppen substituiert ist,
**R₅** noch bevorzugter ein Wasserstoffatom oder eine (C₁-C₃)Alkyl-Gruppe, vorteilhafterweise ein Wasserstoffatom, darstellt; und
▪ **R₆** ein Wasserstoffatom, eine (C₁-C₃)Alkyl-, eine Aryl-(C₁-C₃)alkyl oder eine -CH₂-CH₂-O-CH₂-CH₂-NH₂-Gruppe oder eine (C₁-C₆) Alkylcarbonyl-Gruppe darstellt, die wahlweise mit einem oder mehreren Substituenten substituiert ist, der bzw. die aus der Gruppe ausgewählt ist bzw. sind, die besteht aus OH, SH, NH₂, einer (C₁-C₃)Alkoxy-, einer (C₁-C₃)Thioalkoxy-, einer (C₁-C₃)Alkylamino- und einer (C₁-C₆) alkylamino-Gruppe,
vorzugsweise **R₆** ein Wasserstoffatom, eine Methyl-, eine Ethyl-, eine Benzyl-, eine -CH₂-CH₂-O-CH₂-CH₂-NH₂- oder eine (C₁-C₆)Alkylcarbonyl-Gruppe darstellt, die wahlweise mit einem oder mehreren Substituenten substituiert ist, der bzw. die ausgewählt ist bzw. sind aus der Gruppe, die besteht aus OH, NH₂ und einer Thiomethyl-Gruppe, wobei insbesondere **R₆** ein Wasserstoffatom oder eine -CH₂-CH₂-O-CH₂-CH₂-NH₂-Gruppe darstellt.

4. Pharmazeutische Zusammensetzung nach Anspruch 1, wobei der Inhibitor des regulierten nekrotischen Zelltods eine Verbindung mit der Formel **(II)** ist, wobei:
▪ **X₁** eine (C₁-C₆)Alkyl-, eine Aryl-, eine Aryl- (C₁-C₆)Alkyl- oder eine O**R_{X}**-Gruppe darstellt, wobei **R_{X}** eine (C₁-C₆)Alkyl-, eine Aryl- oder eine Aryl-(C₁-C₆)Alkyl-Gruppe ist;
▪ **X₂** bis **X₃** unabhängig voneinander ein Wasserstoffatom oder eine (C₁-C₆)Alkyl-Gruppe darstellen; und
▪ **Y₁, Y₂** und **Y₃** unabhängig voneinander ein Wasserstoffatom, eine (C₁-C₆)Alkyl-, eine Aryl-, eine Aryl-(C₁-C₆)Alkyl-, eine OH- oder eine O**R_{Y}-**Gruppe darstellen, wobei mindestens eines von **Y₁**, **Y₂** und **Y₃** eine (C₁-C₆)Alkyl-, eine Aryl-, eine Aryl-(C₁-C₆)Alkyl- oder eine O**R_{Y}**-Gruppe darstellt, wobei **R_{Y}** ausgewählt ist aus einer (C₁-C₆)Alkyl-, einer Aryl- und einer Aryl-(C₁-C₆)Alkyl-Gruppe.

5. Pharmazeutische Zusammensetzung nach Anspruch 4, wobei:
▪ **X₁** eine O**R_{X}**-Gruppe darstellt, wobei **R_{X}** vorteilhafterweise eine (C₁-C₆)Alkyl-Gruppe ist;
▪ **Y₁** eine O**R_{Y}**-Gruppe darstellt, wobei **R_{Y}** vorteilhafterweise eine Aryl-(C₁-C₆)alkyl-Gruppe ist, und **Y₂** und **Y₃** jeweils ein Wasserstoffatom darstellen.

6. Pharmazeutische Zusammensetzung nach Anspruch 1, wobei der Inhibitor des regulierten nekrotischen Zelltods ist:
**(A)** eine Verbindung mit der folgenden allgemeinen Formel **(I.iii):** 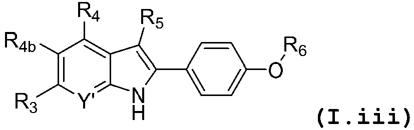 oder ein pharmazeutisch verträgliches Salz und/oder Solvat davon, wobei:
- **R₃, R₄**, **R_{4b}** und **R₅** unabhängig voneinander ein Wasserstoffatom, ein Halogenatom oder eine (C₁-C₆)Alkyl-Gruppe, insbesondere ein Wasserstoffatom, ein Halogenatom oder eine (C₁-C₃)Alkyl-Gruppe, vorzugsweise ein Wasserstoffatom oder ein Halogenatom darstellen, und
- **R₆** ein Wasserstoffatom, eine (C₁-C₃)Alkyl- oder eine -CH₂-CH₂-O-CH₂-CH₂-NH₂-Gruppe, insbesondere ein Wasserstoffatom oder eine -CH₂-CH₂-O-CH₂-CH₂-NH₂-Gruppe darstellt;
oder
**(b)** eine Verbindung mit der folgenden allgemeinen Formel **(II.i):** 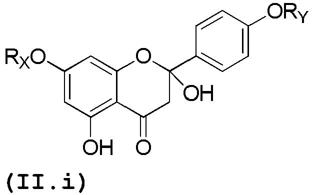 oder ein pharmazeutisch verträgliches Salz und/oder Solvat davon, wobei:
- **R_{X}** eine (C₁-C₆) Alkyl-Gruppe, insbesondere eine (C₁-C₃)Alkyl-Gruppe wie Methyl, Ethyl, n-Propyl, vorteilhafter Methyl, darstellt, und
- **R_{Y}** eine Aryl-(C₁-C₆)Alkyl-Gruppe, wie Benzyl oder -CH₃-Naphthyl, vorzugsweise Benzyl, darstellt.

7. Pharmazeutische Zusammensetzung einem der Ansprüche 1 bis 6, wobei der Inhibitor des regulierten nekrotischen Zelltods aus der Gruppe ausgewählt ist, die besteht aus: 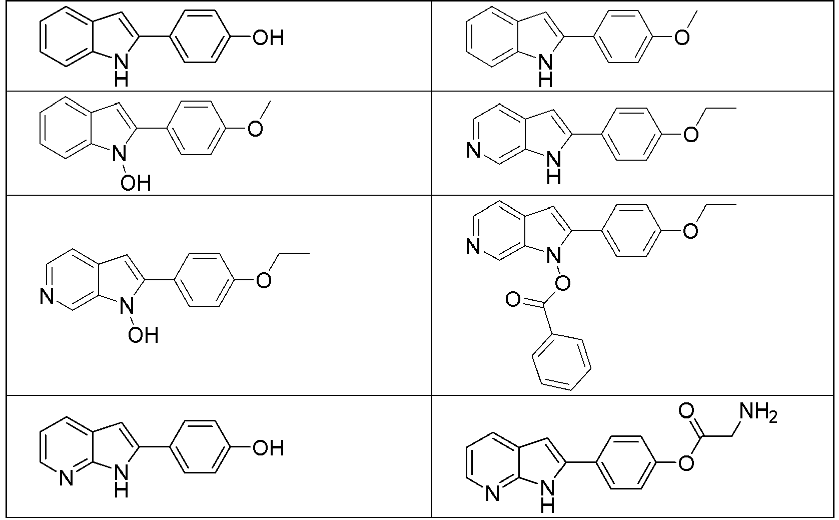 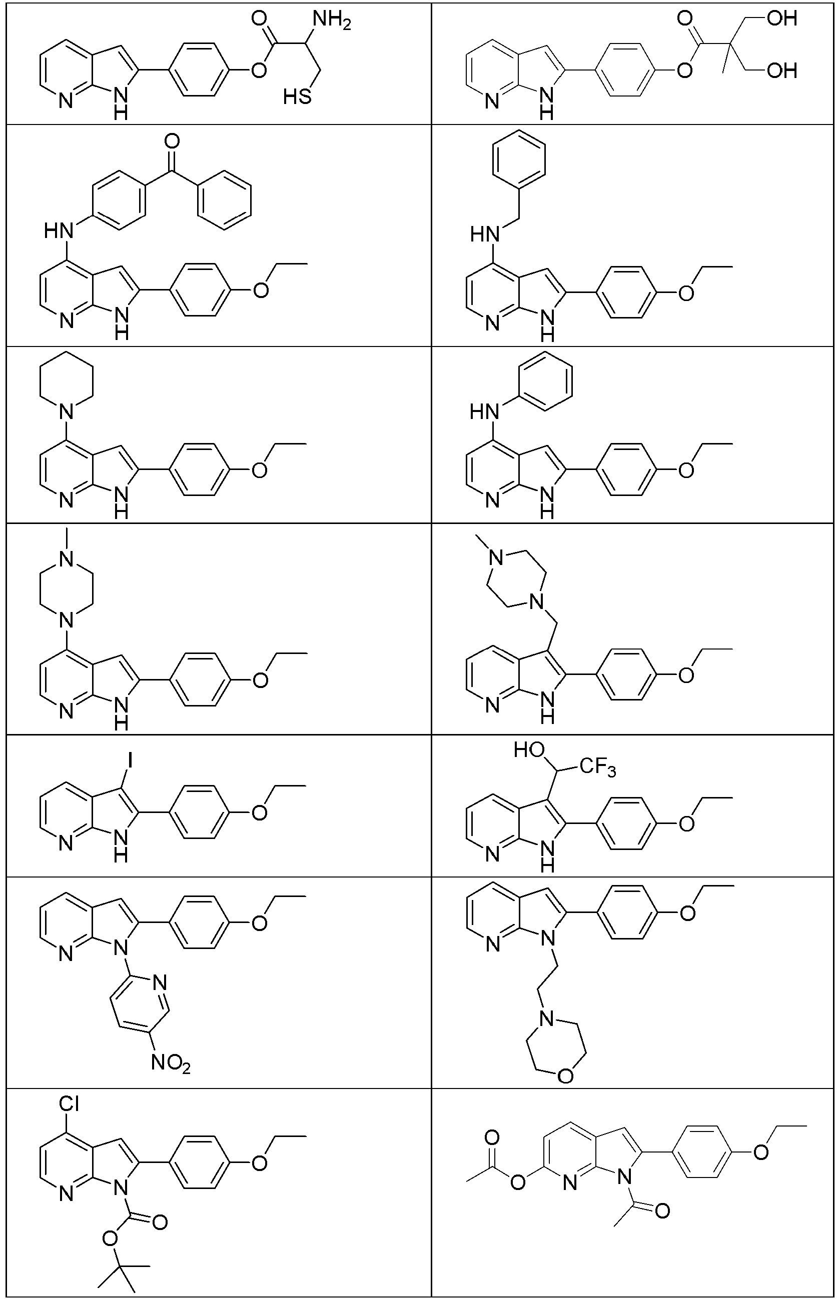 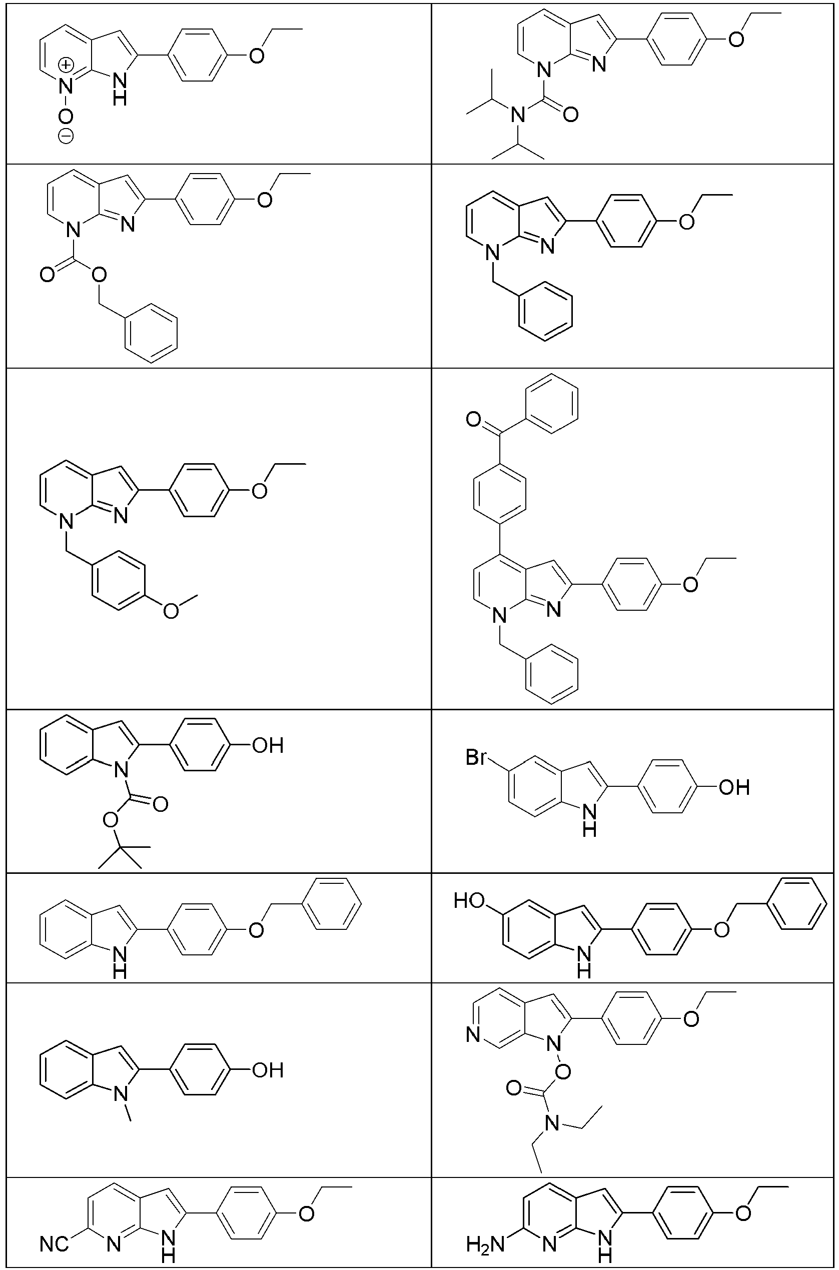 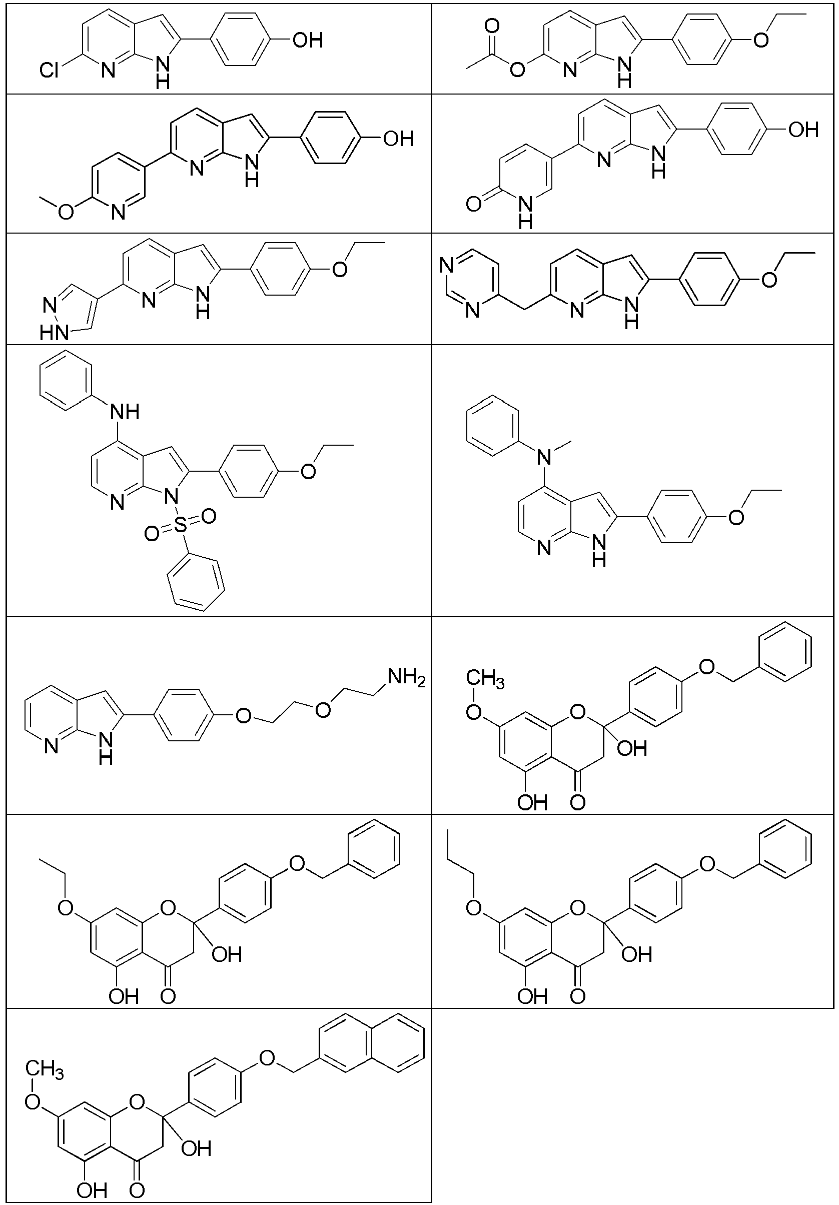 und den pharmazeutisch verträglichen Salzen und/oder Solvaten davon.

8. Pharmazeutische Zusammensetzung einem der Ansprüche 1 bis 7, wobei der Inhibitor des regulierten nekrotischen Zelltods aus der Gruppe ausgewählt ist, die besteht aus: 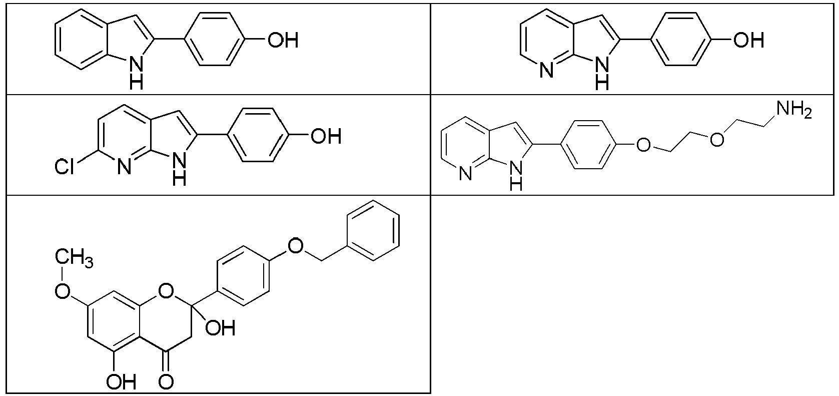 und den pharmazeutisch verträglichen Salzen und/oder Solvaten davon.

9. Pharmazeutische Zusammensetzung nach einem der Ansprüche 1 bis 8, umfassend:
- N-Acetylcystein und/oder ein pharmazeutisches Salz und/oder ein Derivat davon, wobei das Derivat nach Anspruch 1 ist, und
- mindestens einen Inhibitor des regulierten nekrotischen Zelltods
als Kombinationspräparat zur gleichzeitigen, getrennten oder aufeinanderfolgenden Verabreichung.

10. Pharmazeutische Zusammensetzung nach einem der Ansprüche 1 bis 9 zur Verwendung bei der Vorbeugung und/oder Behandlung einer Erkrankung, die mit reguliertem nekrotischem Zelltod in Zusammenhang steht, wobei die Erkrankung ausgewählt ist aus der Gruppe, die besteht aus Hirnerkrankungen oder -störungen, einschließlich neurodegenerativer Erkrankungen oder Störungen, Schlaganfall, traumatischer Hirnverletzung, Epilepsie; Augenerkrankungen oder -störungen, einschließlich Retinopathie, degenerativer Augenerkrankungen oder -störungen; Infektionskrankheiten; Autoimmunerkrankungen; entzündlichen Erkrankungen; Leberschäden, einschließlich akutem Leberversagen und chronischen Lebererkrankungen; Bluthochdruck; Hämochromatose; hämolytischen Störungen; ischämischen Erkrankungen, die Herz, Gehirn oder Nieren betreffen; Nierenschäden, einschließlich akutem Nierenversagen, renaler Ischämie-Reperfusionsschädigung, akuter tubulärer Nekrose, Leberfibrose; Herzschäden; Aortenaneurysma; Pankreatitis; strahleninduzierter Nekrose; chronisch obstruktiver Lungenerkrankung, akutem Atemnotsyndrom; transplantationsbedingten Erkrankungen; Krebserkrankungen, einschließlich Leberkrebs, Augenkrebs, Hirntumoren und Nierenkrebs.

11. Verwendung einer pharmazeutischen Zusammensetzung nach einem der Ansprüche 1 bis 9 zur Invitro-Konservierung und/oder zum Schutz von biologischen Materialien wie Zellen, Geweben, Körperflüssigkeiten und Organen.

## Revendications

1. Composition pharmaceutique comprenant une combinaison de :
la N-acétylcystéine et/ou d'un sel pharmaceutique et/ou d'un dérivé de celle-ci, le dérivé de la N-acétylcystéine étant l'amide de N-acétylcystéine, et/ou l'ester éthylique de N-acétylcystéine et/ou l'ester méthylique de N-acétylcystéine, et/ou un ou des sels de ceux-ci,
avec au moins un inhibiteur de la mort cellulaire nécrotique régulée, dans laquelle l'inhibiteur de la mort cellulaire nécrotique régulée est :
**(A)** un composé de formule générale **(I)** suivante : 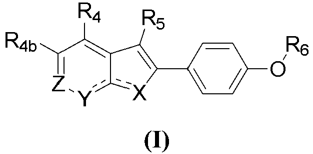
ou un sel et/ou un solvate pharmaceutiquement acceptable de celui-ci,
dans laquelle :
▪ 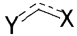 est 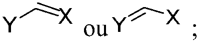
(i) lorsque 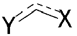 est 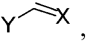 **X** représente N, **Y** représente N(**R₂**) et **Z** représente C(H) ;
(ii) lorsque 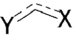 est 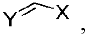
- **X** est N(**R₁**), et
- **Y** est N ou N⁺ (O⁻ ) et **Z** est C(**R₃** ), ou
**Y** est CH et **Z** est N, ou
**Y** et **Z** sont CH ;
et dans laquelle :
▪ **R₁** et **R₂** représentent, indépendamment l'un de l'autre, un atome d'hydrogène, CN, NO₂ , O**R₇**, **SR₈** , **NR₉ R₁₀** , C(O)**R₁₁** , CO₂ **R₁₂** , OC(O)**R₁₃** , **NR₁₄**C(O)**R₁₅** , C(O)**NR₁₆ R₁₇** , S(O)**R_{S}** , SO₂ **R_{S}** ', un groupe alkyle en C₁ à C₆ , un groupe halogénoalkyle en C₁ à C₆ , un groupe -(alkyle en C₁ à C₆ )-[O-(alkyle en C₁ -C₆ )alkyle]ₘ -**NR_{N1} R_{N2}** , où m varie de 1 à 6, un groupe aryle, un groupe aryl-alkyle en (C₁ -C₆ ), un groupe hétérocyclyle ou un groupe hétérocyclyle-alkyle en (C₁ -C₆ ), dans lequel ledit groupe aryle ou hétérocyclyle est optionnellement substitué par un ou plusieurs substituants choisis parmi le groupe constitué d'un atome d'halogène, de CN, de NO₂ , **de** O**R₁₈**, **SR₁₉** , **de NR₂₀ R₂₁** , de C(O)**R₂₂** ,de CO₂ **R₂₃** , OC(O)**R₂₄** , N**R₂₅** C(O)**R₂₆** , C(O)N**R₂₇ R₂₈** , un groupe alkyle en (C₁ -C₆ ) et un groupe halogénoalkyle en (C₁ -C₆ ) ;
▪ **R₃** , **R₄**, **R_{4b}** et **R₅** représentent, indépendamment les uns des autres, un atome d'hydrogène, un atome d'halogène, CN, **OR₂₉** , S**R₃₀** , **NR₃₁ R₃₂** , C(O)**R₃₃** , CO₂ **R₃₄** , OC(O)**R₃₅** , **NR₃₆** C(O)**R₃₇** , C(O)**NR₃₈ R₃₉** , un groupe alkyle en (C₁ - C₆) , un groupe halogénoalkyle en (C₁ - C₆) , ledit groupe alkyle ou halogénoalkyle étant optionnellement substitué par un ou plusieurs substituants choisis parmi le groupe constitué de **OR₄₀**, **SR₄₁** et **NR₄₂ R₄₃** , un groupe aryle, hétérocyclyle, aryle- alkyle en (C₁ -C₆ )ou hétérocyclyle- alkyle en (C₁ -C₆ ), dans lequel ledit groupe aryle ou hétérocyclyle est optionnellement substitué par un ou plusieurs substituants choisis parmi le groupe constitué d'un atome d'halogène, CN, NO₂ , **OR₄₄**, **SR₄₅** , **NR₄₆ R₄₇** , C(O)**R₄₈** , CO₂ **R₄₉** , OC(O)**R₅₀** , **NR₅₁** C(O)**R₅₂** , C(O)**NR₅₃ R₅₄** , un groupe alkyle en (C₁ -C₆ ) et un groupe halogénoalkyle en (C₁ -C₆ ) ;
▪ **R₆** représente un atome d'hydrogène, un groupe alkyle en C₁ -C₆ , un groupe aryl- alkyle en (C₁ -C₆ ), un groupe hétérocyclyle- alkyle en (C₁ -C₆ ), un groupe alkyle en -(C₁ -C₆ )-[O-alkyle en (C₁ -C₆ )]_{m'} **-NR_{N'1} R_{N'2}** , m' étant compris entre 1 et 6, ou un groupe alkylcarbonyle en (C1-C6), lesdits groupes alkyle en (C₁ -C₆ ), aryle-alkyle en (C₁ -C₆ ) et alkylcarbonyle en (C₁-C₆) étant optionnellement substitués par un ou plusieurs substituants choisis parmi le groupe constitué de OH, SH, NH₂ , un groupe alcoxy en (C1-C6), un groupe thioalcoxy en (C₁ -C₆) , un groupe alkylamino en (C₁ -C₆ ) et un groupe di(alkyle en (C₁ -C₆ ))amino ;
▪ **R_{S}** et **R_{S}** ' représentent, indépendamment l'un de l'autre, un groupe alkyle en (C₁ -C₆) , un groupe aryle ou un groupe aryle- =alkyle en (C₁ -C₆ );
▪ **R₇ -R₁₀**, **R₁₂** , **R₁₄** et **R₁₆ -R₁₇** représentent, indépendamment les uns des autres, un atome d'hydrogène, un groupe alkyle en (C₁ -C₆) , un groupe aryle ou un groupe aryl- alkyle en (C₁ -C₆ );
▪ **R₁₁**, **R₁₃** et **R₁₅** représentent, indépendamment les uns des autres, un atome d'hydrogène, un groupe alkyle en (C₁-C₆), un groupe aryle, un groupe aryle-alkyle en (C₁-C₆)-, un groupe alcoxy en (C₁ -C₆), un groupe alkylamino en (C₁C6) ou un groupe di((alkyle en C₁-C₆)amino ;
▪ **R₁₈** à **R₂₈** représentent, indépendamment les uns des autres, un atome d'hydrogène, un groupe alkyle en (C₁ -C₆) ou un groupe aryle ;
▪ **R₂₉** à **R₃₉** représentent, indépendamment les uns des autres, un atome d'hydrogène, un groupe alkyle en (C₁ -C₆) , hétérocyclyle-alkyle en (C₁ -C₆), aryle ou aryle-alkyle en (C₁ -C₆ ), ledit groupe aryle étant optionnellement substitué par un ou plusieurs substituants choisis parmi le groupe constitué d'un atome d'halogène, CN, NO₂ , **OR₅₅**, **SR₅₆**, **NR₅₇ R₅₈** , C(O)**R₅₉** , CO₂ **R₆₀**, OC(O)**R₆₁** , **NR₆₂** C(O)**R₆₃** , C(O)**NR₆₄ R₆₅** , un groupe alkyle en (C₁₋C₆ ) et un groupe halogénoalkyle en (C₁-C₆);
▪ **R₄₀** à **R₄₃** représentent, indépendamment les uns des autres, un atome d'hydrogène ou un groupe alkyle en (C₁ -C₆) ;
▪ **R₄₄** à **R₅₄** représentent, indépendamment les uns des autres, un atome d'hydrogène, un groupe alkyle en (C₁-C₆) ou un groupe aryle ;
▪ **R₅₅ à R₆₅** représentent, indépendamment les uns des autres, un atome d'hydrogène, un groupe alkyle en (C₁-C₆), un groupe aryle- alkyle en (C₁-C₆) ou un groupe aryle ; et
**R_{N1}, R_{N'1}** , **R_{N2}** et **R_{N'2}** représentent, indépendamment les uns des autres, un atome d'hydrogène,
un groupe alkyle en (C₁-C₆) , un groupe aryle- alkyle en (C₁ à C₆ ) ou un groupe aryle ; ou
**(B)** un composé de formule générale **(II)** suivante : 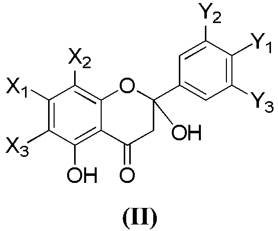 ou un sel et/ou un solvate pharmaceutiquement acceptable de celui-ci, dans laquelle :
▪ **X₁, X₂** et **X₃** représentent, indépendamment les uns des autres, un atome d'hydrogène, un groupe alkyle en (C₁ -C₆), un groupe aryle, un groupe aryl-alkyle en (C₁-C₆, ou un groupe OH, ou un groupe choisi parmi **OR_{X}, SR_{X}** , SO₂ **Rx** et N**R_{X} R_{Z} ,**
dans lesquels au moins un parmi **X₁, X₂** et **X₃** représente un groupe alkyle en (C₁-C₆), un groupe aryle ou un groupe aryl-alkyle en (C₁-C₆), ou un groupe choisi parmi **OR_{X},**S**R_{X},** SO₂**R_{X}** et **NR_{X}R_{Z},** dans lequel
**R_{X}** représente un groupe alkyle en (C₁-C₆), un groupe aryle ou un groupe aryl-alkyle en (C₁-C₆),
**R_{Z}** est un atome d'hydrogène ou un groupe alkyle en (C₁-C₆), et
les groupes aryle sont optionnellement substitués par un ou plusieurs groupes choisis parmi un atome d'halogène, **-OR₆₆** , **-NR₆₇ R₆₈ , -SR₆₉** , -S(O)**R₇₀** , -SO₂ **R₇₁** , -OCO**R₇₂** , -CO₂**R₇₃** , -CON**R₇₄R₇₅** , -CO₂**R₇₆** , nitro (-NO₂ ) et cyano (-CN) ;
▪ **Y₁, Y₂** et **Y₃** représentent, indépendamment les uns des autres, un atome d'hydrogène, un groupe alkyle en (C₁-C₆), un groupe aryle, un groupe aryl-alkyle en (C₁-C₆) ou un groupe OH, ou un groupe choisi parmi **OR_{Y}, SR_{Y}** , SO₂**R_{Y}** et **NR_{Y}R'_{Z}** ,
dans laquelle au moins l'un des groupes **Y₁, Y₂** et **Y₃** représente un groupe alkyle en (C₁-C₆), un groupe aryle ou un groupe aryl-alkyle en (C₁-C₆), ou un groupe choisi parmi **OR_{Y}, SR_{Y},** SO**₂R_{Y}** et **NR_{Y}R'_{Z},** dans lequel
**R_{Y}** est un groupe alkyle en (C₁-C₆), un groupe aryle ou un groupe aryl-alkyle en (C₁- C₆), **R'_{Z}** est un atome d'hydrogène ou un groupe alkyle en (C₁-C₆), et
les groupes aryle sont optionnellement substitués par un ou plusieurs groupes choisis parmi un atome d'halogène, -O**R₆₆** , -N**R₆₇R₆₈** , **-SR₆₉** , -S(O)**R₇₀** , -SO₂**R₇₁** , -O**COR₇₂** , -CO₂**R₇₃** , -CON**R₇₄ R₇₅** , -CO₂**R₇₆** , nitro (-NO₂) et cyano (-CN) ;
et
▪ **R₆₆** à **R₇₇** représentent, indépendamment les uns des autres, un atome d'hydrogène ou un groupe alkyle en (C₁ -C₆).

2. Composition pharmaceutique selon la revendication 1, dans laquelle l'inhibiteur de la mort cellulaire nécrotique régulée est un composé de formule **(I),** dans laquelle :
▪ **R₁** et **R₂** représentent, indépendamment l'un de l'autre, un atome d'hydrogène, CN, NO₂ , O**R₇** , S**R₈** , N**R₉R₁₀** , C(O)**R₁₁** , CO₂**R₁₂** , OC(O)**R₁₃** , N**R₁₄**C(O)**R₁₅** , C(O)**NR₁₆R₁₇** , S(O)**R_{S}** , SO₂**R_{S}**'un groupe alkyle en (C₁ -C₆ ), un groupe halogénoalkyle en (C₁ -C₆ ), un groupe aryle, un groupe hétérocyclyle, un groupe aryle- alkyle en (C₁ -C₆ ) ou un groupe hétérocyclyle- alkyle en (C₁ -C₆ ), dans lequel ledit groupe aryle ou hétérocyclyle est optionnellement substitué par un ou plusieurs substituants choisis parmi le groupe constitué d'un atome d'halogène, CN, NO₂ , **OR₁₈, SR₁₉ , NR₂₀R₂₁** , C(O)**R₂₂** , CO₂**R₂₃** , OC(O)**R₂₄** , **NR₂₅**C(O)**R₂₆** , C(O)**NR₂₇R₂₈** , un groupe alkyle en (C₁ -C₆ ) et un groupe halogénoalkyle en (C₁ -C₆ ) ;
▪ **R₃** , **R₄ , R_{4b}** et **R₅** représentent, indépendamment les uns des autres, un atome d'hydrogène, un atome d'halogène, CN, O**R₂₉** , S**R₃₀** , N**R₃₁ R₃₂** , C(O)**R₃₃** , CO₂**R₃₄** , OC(O)**R₃₅** , N**R₃₆** C(O)**R₃₇** , C(O)**NR₃₈R₃₉** , un groupe alkyle en (C₁-C₆), un groupe halogénoalkyle en (C₁-C₆), ledit groupe alkyle ou halogénoalkyle étant optionnellement substitué par un ou plusieurs substituants choisis parmi le groupe constitué de O**R₄₀**, S**R₄₁** et N**R₄₂R₄₃** , un groupe aryle, hétérocyclyle, aryle- alkyle en (C₁ -C₆ ) ou hétérocyclyle- alkyle en (C₁ -C₆ ), dans lequel ledit groupe aryle ou hétérocyclyle est optionnellement substitué par un ou plusieurs substituants choisis parmi le groupe constitué d'un atome d'halogène, CN, NO₂ , O**R₄₄**, S**R₄₅** , N**R₄₆ R₄₇** , C(O)**R₄₈** , CO₂ **R₄₉** , OC(O)**R₅₀** , N**R₅₁** C(O)**R₅₂** , C(O)N**R₅₃R₅₄** , un groupe alkyle en (C₁ -C₆ ) et un groupe halogénoalkyle en (C₁ -C₆ ) ;
▪ **R₆** représente un atome d'hydrogène, un groupe alkyle en (C₁ -C₆), un groupe aryle-alkyle en (C₁ -C₆) ou un groupe -CH₂-CH₂-O-CH₂-CH₂-NH₂ , ou un groupe alkyl carbonyle en (C₁-C₆ ) optionnellement substitué par un ou plusieurs substituants choisis parmi le groupe constitué de OH, SH, NH₂ , un groupe alcoxy en (C₁ -C₆ ), un groupe thioalcoxy en (C₁ -C₆ ), un groupe alkylamino en (C₁ -C₆) et un groupe di(alkyle en (C₁ -C₆ ))amino ;
▪ **R_{S}** et **R_{S}'** représentent, indépendamment l'un de l'autre, un groupe alkyle en (C₁ -C₆), un groupe aryle ou un groupe aryle- alkyle en (C₁ -C₆ );
▪ **R₇-R₁₀** , **R₁₂** , **R₁₄** et **R₁₆ -R₁₇** représentent, indépendamment les uns des autres, un atome d'hydrogène, un groupe alkyle en (C₁-C₆), un groupe aryle ou un groupe aryle-alkyle en (C₁ -C₆) ;
▪ **R₁₁, R₁₃** et **R₁₅** représentent, indépendamment les uns des autres, un atome d'hydrogène, un groupe alkyle en (C₁-C₆), un groupe aryle, un groupe aryle- alkyle en (C₁ -C₆ ), un groupe alcoxy en (C₁ -C₆) , un groupe alkylamino en (C₁ -C₆) ou un groupe di(alkyle en (C₁ - C₆))amino ;
▪ **R₁₈ à R₂₈** représentent, indépendamment les uns des autres, un atome d'hydrogène, un groupe alkyle en (C₁-C₆) ou un groupe aryle ;
▪ **R₂₉** à **R₃₉** représentent, indépendamment les uns des autres, un atome d'hydrogène, un groupe alkyle en (C₁ à C₆), un groupe aryle ou un groupe aryle- alkyle en (C₁-C₆) ; ledit groupe aryle étant optionnellement substitué par un ou plusieurs substituants choisis parmi le groupe constitué d'un atome d'halogène, CN, NO₂, **OR₅₅SR₅₆, e NR₅₇R₅₈,** C(O)**R₅₉**, CO₂**R₆₀** , OC(O)**R₆₁** , **NR₆₂**C(O)**R₆₃** , C(O)**NR₆₄R₆₅** , un groupe alkyle en (C₁-C₆)et un groupe halogénoalkyle en (C₁-C₆);
▪ **R₄₀** à R₄₃ représentent, indépendamment les uns des autres, un atome d'hydrogène ou un groupe alkyle en (C₁-C₆) ;
▪ **R₄₄** à **R₅₄** représentent, indépendamment les uns des autres, un atome d'hydrogène, un groupe alkyle en (C₁ -C₆) ou un groupe aryle ; et
▪ **R₅₅ à R₆₅** représentent, indépendamment les uns des autres, un atome d'hydrogène, un groupe alkyle en (C₁-C₆) ou un groupe aryle.

3. Composition pharmaceutique selon la revendication 1, dans laquelle l'inhibiteur de la mort cellulaire nécrotique régulée est un composé de formule **(I),** dans laquelle :
▪ **R₁** représente un atome d'hydrogène, CN, NO₂ , O**R₇** , S**R₈** , N**R₉R₁₀** , C(O)**R₁₁** , CO₂**R₁₂** , OC(O)**R₁₃** , N**R₁₄**C(O)**R₁₅** , C(O)**NR₁₆R₁₇** , S(O)**R_{S}** , SO₂**R_{S}**', un groupe alkyle en (C₁ -C₆), un groupe halogénoalkyle en (C₁-C₆ ), un groupe aryle, un groupe hétérocyclyle, un groupe aryle- alkyle en (C₁ -C₆ ) ou un groupe hétérocyclyle- alkyle en (C₁ -C₆ ), dans lequel ledit groupe aryle ou hétérocyclyle est optionnellement substitué par un substituant choisi parmi le groupe constitué d'un atome d'halogène, CN, NO₂ , O**R₁₈**, S**R₁₉** , N**R₂₀R₂₁** , un groupe alkyle en (C₁ -C₆ ) et un groupe halogénoalkyle en (C₁-C₆), dans lequel **R₁₈** à **R₂₁** représentent, indépendamment les uns des autres, un atome d'hydrogène ou un groupe alkyle en (C₁-C₆) et **R_{S}** et **R_{S}'** représentent, indépendamment l'un de l'autre, un groupe alkyle en (C₁-C₆) ou un groupe aryle, de préférence un groupe aryle,
de préférence, **R₁** représente un atome d'hydrogène, CN, O**R₇**, C(O)**R₁₁**, CO₂**R₁₂**, OC(O)**R₁₃**, SO₂**R_{S}**', un groupe alkyle en (C₁-C₆), un groupe hétérocyclyle ou un groupe hétérocyclyle-alkyle en (C₁- C₆), ledit groupe hétérocyclyle étant optionnellement substitué par NO₂, plus préférentiellement, **R₁** représente un atome d'hydrogène ou un groupe alkyle en (C₁-C₆ ), en particulier un atome d'hydrogène ou un groupe alkyle en (C₁-C₃ ), avantageusement un atome d'hydrogène ;
▪ **R₂** représente C(O)**R₁₁** , CO₂**R₁₂** , C(O)**NR₁₆R₁₇** , un groupe alkyle en (C₁-C₆), un groupe halogénoalkyle en C₁-C₆), un groupe aryle, un groupe hétérocyclyle, un groupe aryle-alkyle en C₁-C6) ou un groupe hétérocyclyle-alkyle en (C₁ -C₆ ), dans lequel ledit groupe aryle ou hétérocyclyle est optionnellement substitué par un ou plusieurs substituants choisis parmi le groupe constitué d'un atome d'halogène, O**R₁₈**, S**R₁₉** N**R₂₀R₂₁** , un groupe alkyle en (C₁-C₆) et un groupe halogénoalkyle en (C₁-C₆), dans lequel **R₁₈** à **R₂₁** représentent, indépendamment les uns des autres, un atome d'hydrogène ou un groupe alkyle en (C₁-C₆),
de préférence, **R₂** représente CO₂**R₁₂,** C(O)N**R₁₆R₁₇** ou un groupe aryle- alkyle en (C₁ -C₆ ), dans lequel ledit groupe aryle est optionnellement substitué par un ou plusieurs substituants choisis parmi le groupe constitué d'un atome d'halogène, O**R₁₈,** S**R₁₉** et N**R₂₀ R₂₁** , notamment O**R₁₈** ;
▪ **R₃** représente un atome d'hydrogène, un atome d'halogène, un groupe alkyle en (C₁ -C₆) , CN, O**R₂₉** , S**R₃₀** , N**R₃₁ R₃₂** , C(O)**R₃₃** , CO₂**R₃₄** , OC(O)**R₃₅** , N**R₃₆**C(O)**R₃₇** , C(O)**NR₃₈ R₃₉,** un groupe aryle, un groupe hétérocyclyle, un groupe aryle- alkyle en (C₁ -C₆ ) ou un groupe hétérocyclyle- alkyle en(C₁ -C₆ ), dans lequel ledit groupe aryle ou hétérocyclyle est optionnellement substitué par un ou plusieurs substituants choisis parmi le groupe constitué d'un atome d'halogène, O**R₄₄**, S**R₄₅** , N**R₄₆ R₄₇ ,** un groupe alkyle en (C₁ -C₆ ) et un groupe halogénoalkyle en (C₁ -C₆ ), dans lequel **R₂₉** à **R₃₇** représentent, indépendamment les uns des autres, un atome d'hydrogène ou un groupe alkyle en (C₁ -C₆ ), et **R₄₄** à **R₄₇** représentent, indépendamment les uns des autres, un atome d'hydrogène ou un groupe alkyle en (C₁ -C₆ ), de préférence, **R₃** représente un atome d'hydrogène, un atome d'halogène, un groupe alkyle en (C₁-C₆), CN, O**R₂₉**, S**R₃₀**, N**R₃₁R₃₂**, OC(O)**R₃₅**, N**R₃₆**C(O)**R₃₇** , un groupe hétérocyclyle ou un groupe hétérocyclyle- alkyle en (C₁-C₆ ), dans lequel ledit groupe hétérocyclyle est optionnellement substitué par un ou plusieurs substituants choisis parmi le groupe constitué d'un atome d'halogène, O**R₄₄**, S**R₄₅** , N**R₄₆ R₄₇** et un groupe alkyle en (C₁ -C₆),
plus préférentiellement, **R₃** représente un atome d'hydrogène, un atome d'halogène, un groupe alkyle en (C₁ -C₆ ), CN, **NR₃₁R₃₂** , OC(O)**R₃₅** , un groupe hétérocyclyle ou un groupe hétérocyclyle- alkyle en(C₁ -C₆ ), ledit groupe hétérocyclyle étant optionnellement substitué par un ou plusieurs substituants choisis parmi le groupe constitué de O**R₄₄**, S**R₄₅** et N**R₄₆ R₄₇ ,** notamment **OR₄₄** ,
et de manière encore plus préférentielle, **R₃** représente un atome d'hydrogène, un atome d'halogène ou un groupe alkyle en (C₁ -C₃), avantageusementun atome d'hydrogène ou un atome d'halogène ;
▪ **R₄** représente un atome d'hydrogène, un atome d'halogène, un groupe CN, O**R₂₉** , S**R₃₀** , N**R₃₁ R₃₂** , un groupe alkyle en (C₁ à C₆), un groupe halogénoalkyle en (C₁-C₆), un groupe aryle, un groupe hétérocyclyle, un groupe aryle-alkyle en (C₁-C₆) ou un groupe hétérocyclyle-alkyle en (C₁ -C₆ ), dans lequel ledit groupe aryle ou hétérocyclyle est optionnellement substitué par un ou plusieurs substituants choisis parmi le groupe constitué d'un atome d'halogène, CN, NO₂ , O**R₄₄**, S**R₄₅** , N**R₄₆R₄₇** , C(O)**R₄₈** ,de CO₂**R₄₉** , OC(O)**R₅₀** , N**R₅₁**C(O)**R₅₂** , C(O)N**R₅₃R₅₄** , un groupe alkyle en (C₁ -C₆ ) et un groupe halogénoalkyle en (C₁ -C₆ ),
de préférence, **R₄** représente un atome d'hydrogène, un atome d'halogène, O**R₂₉** , S**R₃₀** , N**R₃₁R₃₂** , un groupe alkyle en (C₁-C₆ ), un groupe aryle ou un groupe hétérocyclyle, ledit groupe aryle ou hétérocyclyle étant optionnellement substitué par un ou plusieurs substituants choisis parmi le groupe constitué d'un atome d'halogène, O**R₄₄**, S**R₄₅** , N**R₄₆R₄₇** , C(O)**R₄₈** , CO₂**R₄₉** , C(O)**NR₅₃R₅₄** , un groupe alkyle en (C₁-C₆) et un groupe halogénoalkyle en (C₁- C₆)
plus préférentiellement, **R₄** représente un atome d'hydrogène, un atome d'halogène, N**R₃₁R₃₂** , un groupe alkyle en (C₁-C₆), un groupe aryle ou un groupe hétérocyclyle, ledit groupe aryle ou hétérocyclyle étant optionnellement substitué par un ou plusieurs substituants choisis parmi le groupe constitué de C(O)**R₄₈** et d'un groupe alkyle en (C₁ -C₆ ),
plus préférentiellement encore, R₄ représente un atome d'hydrogène ou un groupe alkyle en (C₁ -C₃), avantageusement un atome d'hydrogène,
**R_{4b}** représente un atome d'hydrogène, un atome d'halogène, un groupe alkyle en (C₁ -C₆) , OR₂₉ ou NR₃₁R₃₂ , de préférence un atome d'hydrogène, un atome d'halogène, un groupe alkyle en (C₁ -C₃) ou OR₂₉, plus préférentiellement un atome d'hydrogène ou un groupe alkyle en (C₁ -C₃), avantageusement un atome d'hydrogène,
dans laquelle, dans les définitions ci-dessus de R₄ et **R_{4b}** , **R₂₉** à **R₃₂** représentent, indépendamment les uns des autres, un atome d'hydrogène, un groupe alkyle en (C₁ -C₆ ), un groupe aryle ou un groupe aryle- alkyle en (C₁-C₆), ledit groupe aryle étant optionnellement substitué par un ou plusieurs substituants choisis parmi le groupe constitué d'un atome d'halogène, O**R₅₅**, S**R₅₆**, N**R₅₇R₅₈**, C(O)**R₅₉**, CO₂**R₆₀** , OC(O)**R₆₁** , N**R₆₂**C(O)**R₆₃** , C(O)**NR₆₄R₆₅** , un groupe alkyle en (C₁ -C₆ ) et un groupe halogénoalkyle en (C₁ -C₆ ), notamment C(O)**R₅₉** , CO₂ **R₆₀** , C(O)**NR₆₄ R₆₅** , un groupe alkyle en (C₁-C₆) et un groupe halogénoalkyle en (C₁-C₆), en particulier C(O)**R₅₉, R₄₈** représente un atome d'hydrogène, un groupe alkyle en (C₁-C₆) ou un groupe aryle, notamment un groupe aryle, et **R₅₅** à **R₆₅** représentent, indépendamment les uns des autres, un atome d'hydrogène, un groupe alkyle en (C₁-C₆) ou un groupe aryle, notamment un groupe aryle ;
▪ **R₅** représente un atome d'hydrogène, un atome d'halogène, un groupe CN, O**R₂₉** , S**R₃₀** , N**R₃₁R₃₂** , un groupe alkyle en (C₁- C₆) , un groupe halogénoalkyle en (C₁-C₆), ledit groupe alkyle ou halogénoalkyle étant optionnellement substitué par un ou plusieurs substituants choisis parmi le groupe constitué de **OR₄₀**, **SR₄₁** et **NR₄₂ R₄₃** , un groupe aryle, hétérocyclyle, aryle- alkyle en (C₁ -C₆ ) ou hétérocyclyle- alkyle en(C₁ -C₆ ), dans lequel ledit groupe aryle ou hétérocyclyle est optionnellement substitué par un ou plusieurs substituants choisis parmi le groupe constitué d'un atome d'halogène, **OR₄₄, SR₄₅, de NR₄₆ R₄₇** , un groupe alkyle en (C₁ -C₆) et un groupe halogénoalkyle en (C₁ -C₆ ), dans lequel **R₂₉** à R₃₂ représentent, indépendamment les uns des autres, un atome d'hydrogène ou un groupe alkyle en (C₁ -C₆ ), et **R₄₀** à **R₄₃** représentent, indépendamment les uns des autres, un atome d'hydrogène ou un groupe alkyle en (C₁ -C₆) , notamment un atome d'hydrogène,
de préférence **R₅** représente un atome d'hydrogène, un atome d'halogène, un groupe alkyle en (C₁-C₆), un groupe halogénoalkyle en (C₁-C₆), ledit groupe alkyle ou halogénoalkyle étant optionnellement substitué par un ou plusieurs substituants choisis parmi le groupe constitué de **OR₄₀**, **SR₄₁** et **NR₄₂ R₄₃** , un groupe aryle- alkyle en (C₁ -C₆ ) ou un groupe hétérocyclyle-alkyle en (C₁ -C₆ ), dans lequel ledit groupe aryle ou hétérocyclyle est optionnellement substitué par un ou plusieurs substituants choisis parmi le groupe constitué d'un atome d'halogène, d'un groupe alkyle en (C₁ -C₆ ) et d'un groupe halogénoalkyle en (C₁ -C₆ ),
plus préférentiellement, **R₅** représente un atome d'hydrogène, un atome d'halogène, un groupe alkyle en (C₁-C₆), un groupe halogénoalkyle en (C₁-C₆) ou un groupe hétérocyclyl-(C₁à C₆)alkyle, ledit groupe alkyle ou halogénoalkyle étant optionnellement substitué par **OR₄₀**,et ledit hétérocyclyle étant optionnellement substitué par un ou plusieurs groupes alkyle en (C₁-C₆),
encore plus préférentiellement, **R₅** représente un atome d'hydrogène ou un groupe alkyle en (C₁ -C₃), de préférence un atome d'hydrogène ; et
▪ **R₆** représente un atome d'hydrogène, un groupe alkyle en (C₁ -C₃), un groupe aryle- alkyle en (C₁ -C₃ ) ou un groupe -CH₂-CH₂-O-CH₂-CH₂-NH₂ , ou un groupe alkylcarbonyle en (C₁-C₆ ) optionnellement substitué par un ou plusieurs substituants choisis parmi le groupe constitué de OH, SH, NH₂ , un groupe alcoxy en (C₁ -C₃ ), un groupe thioalcoxy en (C₁ -C₃ ) et un groupe alkylamino en (C₁ -C₃) ,
de préférence, **R₆** représente un atome d'hydrogène, un groupe méthyle, un groupe éthyle, un groupe benzyle, un groupe -CH₂-CH₂-O-CH₂-CH₂-NH₂ ou un groupe alkylcarbonyle en (C₁ -C₍₆) optionnellement substitué par un ou plusieurs substituants choisis parmi le groupe constitué de OH, NH₂ et d'un groupe thiométhyle ; en particulier, **R₆** représente un atome d'hydrogène ou un groupe -CH₂-CH₂-O-CH₂-CH₂-NH₂ .

4. Composition pharmaceutique selon la revendication 1, dans laquelle l'inhibiteur de la mort cellulaire nécrotique régulée est un composé de formule (**II)**, dans laquelle :
▪ **X₁** représente un groupe alkyle en (C₁ -C₆), un groupe aryle, un groupe aryle-alkyle en (C₁ - C₆ ) ou un groupe **OR_{X}**, dans lequel **R_{X}** est un groupe alkyle en (C₁ -C₆), un groupe aryle ou un groupe aryle- alkyle en (C₁-C₆) ;
▪ **X₂** et **X₃** représentent, indépendamment l'un de l'autre, un atome d'hydrogène ou un groupe alkyle en C₁-C₆; et
▪ **Y₁**, **Y₂** et **Y₃** représentent, indépendamment les uns des autres, un atome d'hydrogène, un groupe alkyle en (C₁-C₆), un groupe aryle, un groupe aryle- alkyle en (C₁à C₆, un groupe OH ou un groupe **OR_{Y},** dans lequel au moins un parmi **Y₁**, **Y₂** et **Y₃** représente un groupe alkyle en (C₁-C₆), un groupe aryle, un groupe aryle- alkyle en (C₁ -C₆ ) ou un groupe **OR_{Y}** , dans lequel **R_{Y}** est choisi parmi un groupe alkyle en (C₁ -C₆), un groupe aryle et un groupe aryle-alkyle en (C₁ -C₆ ).

5. Composition pharmaceutique selon la revendication 4, dans laquelle :
▪ **X₁** représente un groupe **OR_{X}**, **R_{X}** étant avantageusement un groupe alkyle en (C₁- C₆);
▪ **Y₁** représente un groupe **OR_{Y}, R_{Y}** étant de préférence un groupe aryle- alkyle en (C₁ -C₆ ), et **Y₂** et **Y₃** représentent chacun un atome d'hydrogène.

6. Composition pharmaceutique selon la revendication 1, dans laquelle l'inhibiteur de la mort cellulaire nécrotique régulée est :
**(a)** un composé de formule générale **(I.iii**) suivante : 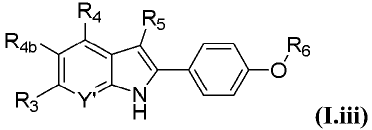 ou un sel et/ou un solvate pharmaceutiquement acceptable de celui-ci, dans laquelle :
- **R₃**, **R₄**, **R_{4b}**et **R₅** représentent, indépendamment les uns des autres, un atome d'hydrogène, un atome d'halogène ou un groupe alkyle en (C₁-C₆), en particulier un atome d'hydrogène, un atome d'halogène ou un groupe alkyle en (C₁-C₃), de préférence un atome d'hydrogène ou un atome d'halogène, et
- R₆ représente un atome d'hydrogène, un groupe alkyle en (C₁ -C₃) ou un groupe -CH₂-CH₂-O-CH₂-CH₂-NH₂, en particulier un atome d'hydrogène ou un groupe -CH₂-CH₂-O-CH₂-CH₂-NH₂;
ou
**(b)** un composé de formule générale (**II.i**) suivante : 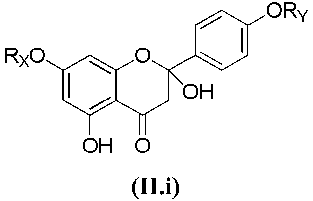 ou un sel et/ou un solvate pharmaceutiquement acceptable de celui-ci, dans laquelle :
- **R_{X}** représente un groupe alkyle en (C₁-C₆), notamment un groupe alkyle en (C₁ -C₃) tel que le méthyle, l'éthyle, le *n*-propyle, de préférence le méthyle, et
- **R_{Y}** représente un groupe aryl-alkyle en (C₁ -C₆ ), tel que le benzyle ou le -CH₃ -naphtyle, de préférence le benzyle.

7. Composition pharmaceutique selon l'une quelconque des revendications 1 à 6, dans laquelle l'inhibiteur de la mort cellulaire nécrotique régulée est choisi parmi le groupe constitué de : 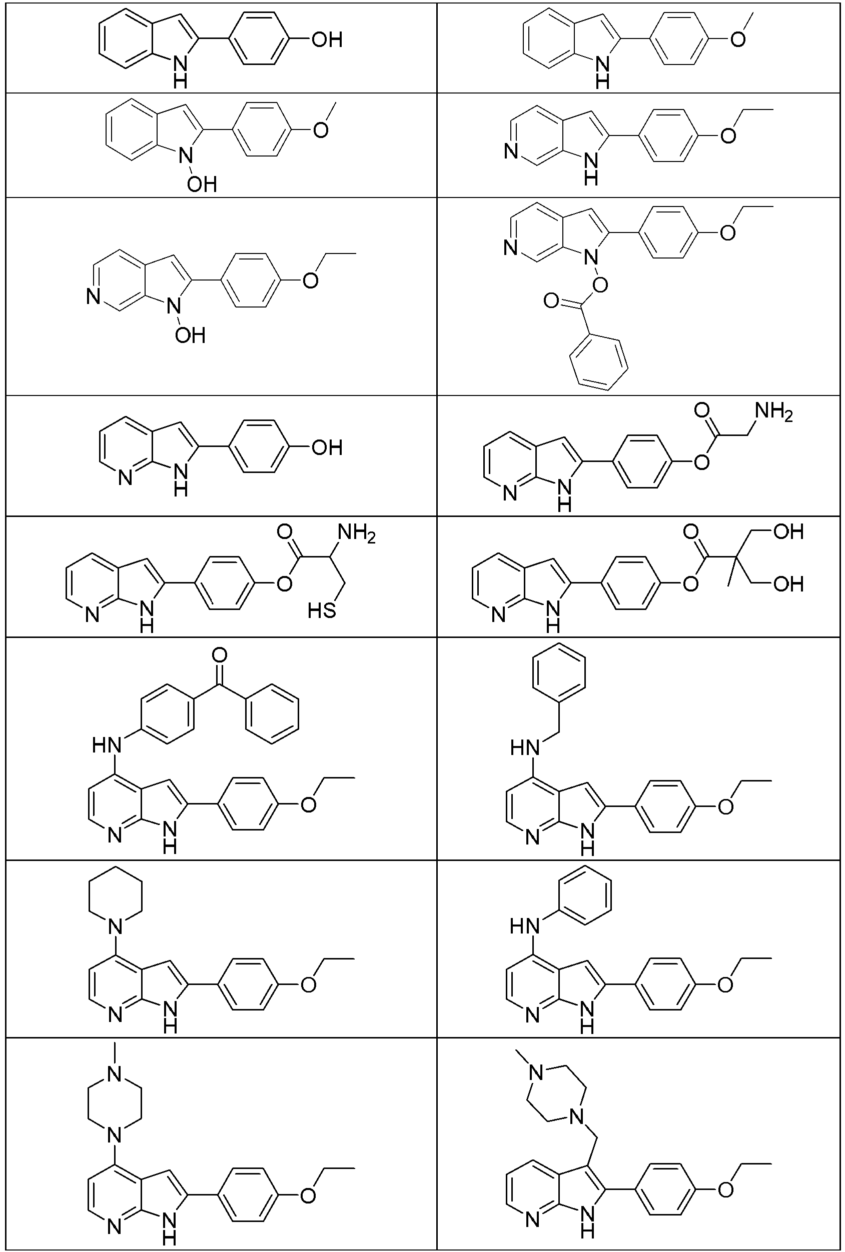 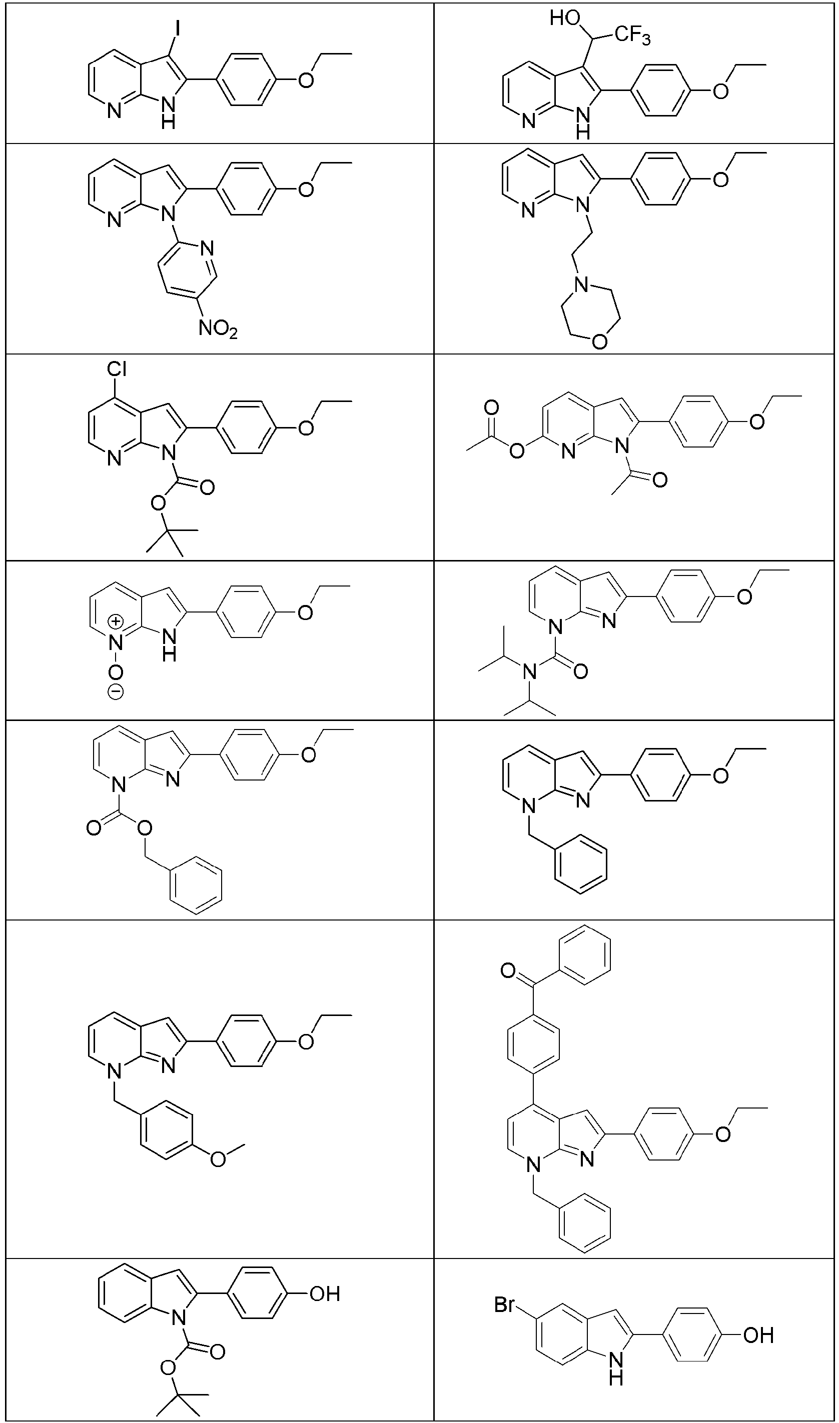 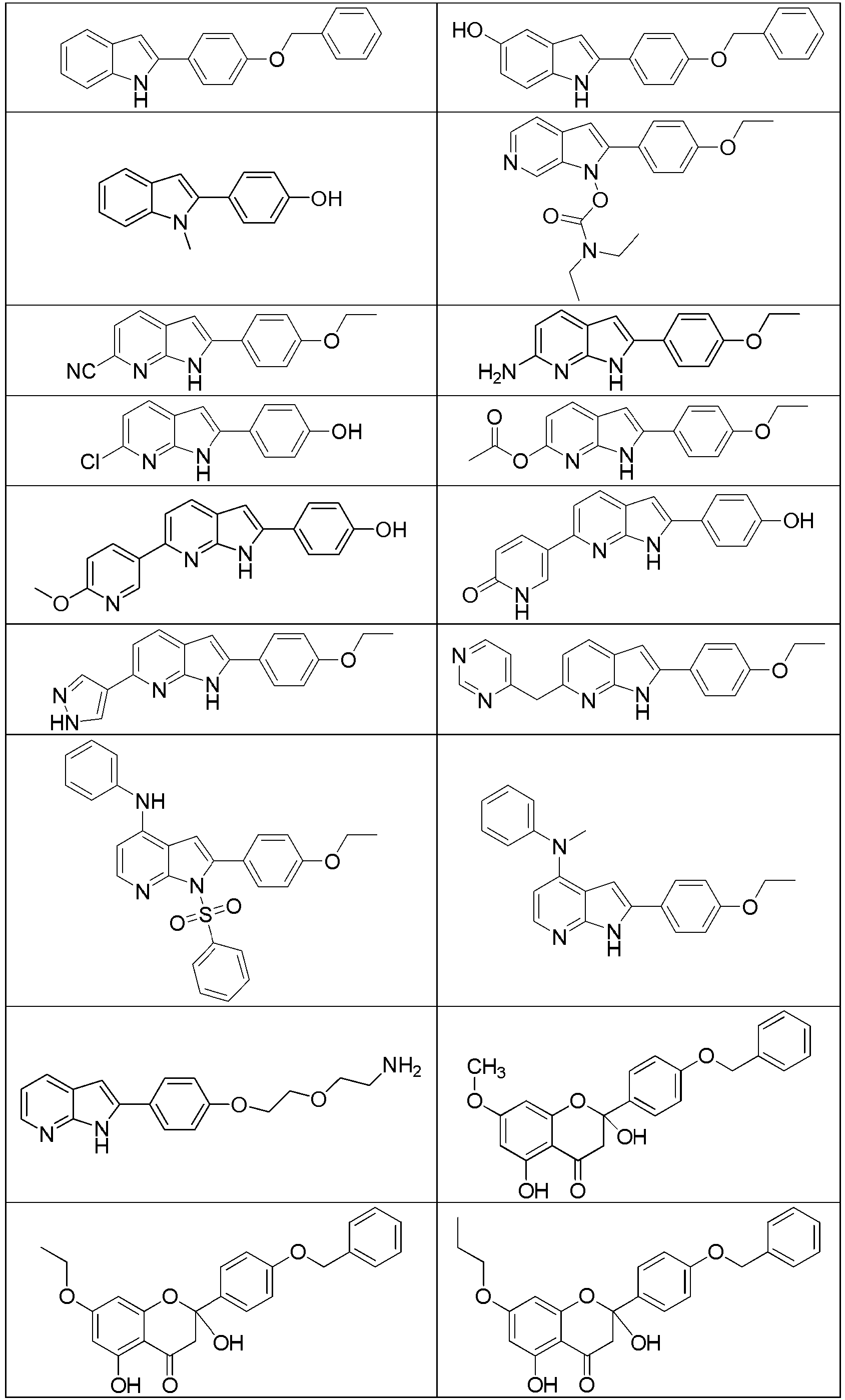 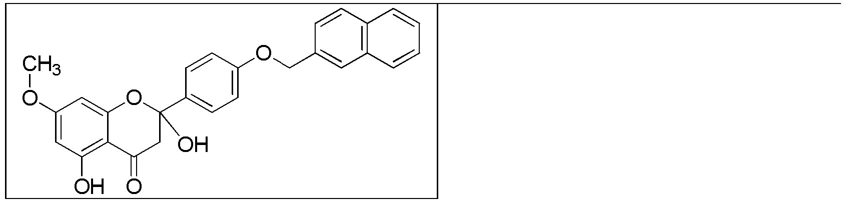 et de leurs sels et/ou solvates pharmaceutiquement acceptables.

8. Composition pharmaceutique selon l'une quelconque des revendications 1 à 7, dans laquelle l'inhibiteur de la mort cellulaire nécrotique régulée est choisi parmi le groupe constitué de : 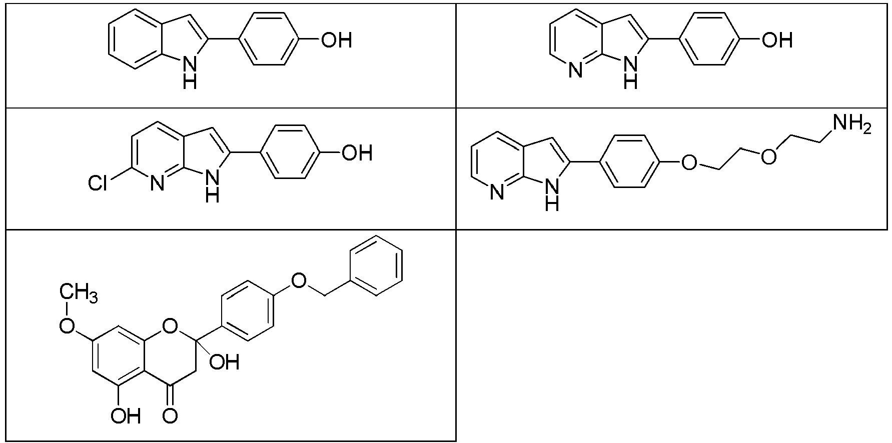 et de leurs sels et/ou solvates pharmaceutiquement acceptables.

9. Composition pharmaceutique selon l'une quelconque des revendications 1 à 8, comprenant :
- la N-acétylcystéine et/ou un sel pharmaceutique et/ou un dérivé de celle-ci, le dérivé étant tel que défini dans la revendication 1, et
- au moins un inhibiteur de la mort cellulaire nécrotique régulée sous forme de produit combiné destiné à une administration simultanée, séparée ou séquentielle.

10. Composition pharmaceutique selon l'une quelconque des revendications 1 à 9, pour son utilisation dans la prévention et/ou le traitement d'un trouble associé à la mort cellulaire nécrotique régulée, dans laquelle le trouble est choisi parmi le groupe comprenant les maladies ou troubles cérébraux, y compris les maladies ou troubles neurodégénératifs, les accidents vasculaires cérébraux, les lésions cérébrales traumatiques, l'épilepsie ; les maladies ou troubles oculaires, y compris la rétinopathie, les maladies ou troubles oculaires dégénératifs ; les maladies infectieuses ; les maladies auto-immunes ; les maladies inflammatoires ; les lésions hépatiques, y compris l'insuffisance hépatique aiguë et les maladies hépatiques chroniques ; l'hypertension ; l'hémochromatose ; les troubles hémolytiques ; les troubles ischémiques affectant le cœur, le cerveau ou les reins ; les lésions rénales, y compris les lésions rénales aiguës, les lésions rénales d'ischémie-reperfusion, la nécrose tubulaire aiguë, la fibrose hépatique ; les lésions cardiaques ; l'anévrisme aortique ; la pancréatite ; la nécrose induite par les rayonnements ; maladie pulmonaire obstructive chronique, syndrome de détresse respiratoire aiguë ; maladies liées à la transplantation ; cancers, y compris les cancers du foie, de l'œil, du cerveau et du rein.

11. Utilisation d'une composition pharmaceutique selon l'une quelconque des revendications 1 à 9, pour la conservation et/ou la protection *in vitro* de matériaux biologiques tels que des cellules, des tissus, des fluides biologiques et des organes.
